# EUROPEAN PATENT SPECIFICATION

(11) **EP 2 185 721 B1**
(45) Date of publication and mention of the grant of the patent: **02.04.2014**
(21) Application number: 08786197.7
(22) Date of filing: 17.07.2008
(51) Int. Cl.: C12Q 1/37, G01N 33/542

(54) **CASPASE IMAGING PROBES**
CASPASE-BILDGEBUNGSSONDEN
SONDES D'IMAGERIE DE CASPASE

(30) Priority: 03.08.2007 EP 07015256
(43) Date of publication of application: 19.05.2010
(73) Proprietor: SANOFI, 75008 Paris (FR)
(72) Inventor: KINDERMANN, Maik, 4053 Basel (CH); MINIEJEW, Catherine, 81369 München (DE); WENDT, Karl-Ulrich, 65926 Frankfurt Am Main (DE)
(74) Representative: Sieber, Frank
(86) International application number: PCT/EP2008/059358
(87) International publication number: WO 2009/019115

(56) References cited:
- US-A1- 2004 002 128
- POUPART SÉVERINE ET AL: "Aminopropargyl derivative of terpyridine-bis(methyl-enamine) tetraacetic acid chelate of europium (Eu (TMT)-AP3): a new reagent for fluorescent labelling of proteins and peptides." ORGANIC & BIOMOLECULAR CHEMISTRY 21 NOV 2006, vol. 4, no. 22, 21 November 2006 (2006-11-21), pages 4165-4177, XP002461151 ISSN: 1477-0520
- TUNG C-H ET AL: "PREPARATION OF A CATHEPSIN D SENSITIVE NEAR-INFRARED FLUORESCENCE PROBE FOR IMAGING" BIOCONJUGATE CHEMISTRY, ACS, WASHINGTON, DC, US, vol. 10, September 1999 (1999-09), pages 892-896, XP001222045 ISSN: 1043-1802
- TAWA PAUL ET AL: "Correlating the fractional inhibition of caspase-3 in NT2 cells with apoptotic markers using an active-caspase-3 enzyme-linked immunosorbent assay." ANALYTICAL BIOCHEMISTRY 1 MAR 2006, vol. 350, no. 1, 1 March 2006 (2006-03-01), pages 32-40, XP002461152 ISSN: 0003-2697

## Description

The present invention relates to molecular probes (substrates) that allow the observation of the catalytic activity of individual proteolytic enzymes or groups of proteolytic enzymes in *in vitro* assays, in cells or in multicellular organisms. The invention furthermore relates to methods for the synthesis and the design of such probes (substrates).

### Background of the Invention

Proteolytic enzymes (proteases) cleave or degrade other enzymes or peptides in-and outside of the living cell. Proteases are involved in a multitude of vital processes, many of which are critical in cellular signalling and tissue homeostasis. Aberrant or enhanced activity of proteases is associated with a variety of diseases including cancer, osteoarthritis, arthereosclerosis, inflammation and many others. Since proteolytic activity has to remain under stringent control in living systems many proteases are expressed as inactive precursor proteins (zymogens) which are activated by controlled proteolytic cleavage. Additional control of proteolytic activity results from endogenous inhibitors that bind to and thereby inactivate catalytically active form of the enzyme. In view of this stringent regulation the investigation of protease function in cellular or physiological events requires the monitoring of protease activity rather than the monitoring of protease expression alone. Consequently, a variety of activity based chemical probes have been proposed in the literature. Commonly applied protease probes generate a detectable signal either (i) through enzymatic cleavage of a peptide bond leading to a change of the spectroscopic properties of a reporter system t or (ii) by covalent attachment of a mechanism based inhibitor to the protease of interest. The localization and quantitative investigation of the activity and inhibition of a specific protease or a group of proteases (e.g. in cell-based assays or whole-animal imaging experiments) require the development of imaging probes that (i) reach the physiologically relevant *locus* of protease action (e.g. the cytosol of a cell or a specific organ in whole animal imaging) and (ii) are selective for the desired protease or a group of proteases. The generation of protease selective probes has imposed a considerable challenge for the field. The present invention relates (i) to novel selective probes for cysteine proteases preferably from the caspases subfamily, (ii) to the application of these probes for in-vitro assays, in cells or in multicellular organisms (e.g. by the means of molecular imaging) and (iii) to methods for the synthesis and the design of such probes.

Within recent years several molecular imaging technologies (optical and non-optical) have become more and more important for the non-invasive visualisation of specific molecular targets and pathways *in vivo.* Since the information content of any image signal is primarily a function of internal contrast, the development of internally quenched imaging probes that are activable upon enzymatic reaction (e.g. cleavage of a peptide bond) has been commonly applied to image and localize catalytically active proteases. The generation of probes that are selective for individual proteases and exhibit the ability to reach the *locus* of protease action *in vivo* has rarely been achieved with conventional approaches. Medicinal chemists in the pharmaceutical industry face related challenges in the development of drugs with appropriate pharmacokinetic properties and appropriate specificity for a given target. In our invention we have devised a new route towards selective activity based probes for cysteine proteases and have applied this approach to proteases from the caspases subfamily.

Cysteine proteases are characterized by a cysteine residue in the active site which serves as a nucleophile during catalysis. The catalytic cysteine is commonly hydrogen bonded with appropriate neighboring residues, so that a thiolate ion can be formed. When a substrate is recognized by the protease, the scissile peptide bond is placed in proximity to the catalytic cysteine, which attacks the carbonyl carbon forming an oxoanion intermediate. The amide bond is then cleaved liberating the C-terminal peptide as an amine. The N-terminal portion of the scissile peptide remains in the covalent acyl-enzyme intermediate, which is subsequently cleaved by water, resulting in regeneration of the enzyme. The N-terminal cleavage product of the substrate is liberated as a carboxylic acid.

Caspases are a family of cysteinyl aspartate-specific proteases. The human genome encodes 11 caspases. Eight of them (caspase-2,3,6,7,8,9,10 and 14) function in apoptosis or programmed cell death. They process through a highly regulated signalling cascade. In a hierarchical order, some initiator caspases (caspase-2,8,9 and 10) cleave and activate effector caspases (caspase-3,6 and 7). These caspases are involved in cancers, autoimmune diseases, degenerative disorders and strokes. Three other Caspases (caspase-1, 4 and 5) serve a distinct function: inflammation mediated by activation of a subset of inflammatory cytokines.

Caspase-1 or interleukin-1β-converting enzyme (ICE) is primarily found in monocytic cells. This protease is responsible for the production of the pro-inflammatory cytokines interleukin-1-beta and interleukine-18. Inhibition of caspase-1 has been shown to be beneficial in models of human inflammation disease, including rheumatoid arthritis, osteoarthritis, inflammatory bowel disease and asthma.

Caspase-3 is responsible for proteolitic cleavage of a variety of fundamental proteins including cytoskeletal proteins, kinases and DNA-repair enzymes. It is a critical mediator of apoptotsis in neurons. Inhibition of caspase-3 have shown efficacy in models such as stroke, traumatic brain spinal cord injury, hypoxic brain damage, cardiac ischemia and reperfusion injury.

Caspase-8 is an apoptosis initiator caspase, downstream of TNF super-family death receptors. Its substrates include apoptosis-related effector caspases and pro-apoptotic Bcl-2 family members. Resistance to apoptosis in cancer has been linked to low expression levels of caspase-8 and inhibition of caspase-8 increases resistance to apoptosis-inducing stressors such as chemotherapy and radiation. Thus caspase-8 is an attractive target for therapy of tumours and metastatic lesions. Knockout studies reveal as well several other potential roles for caspases-8 which are independent of apoptosis. For example, caspase-8 knockouts exhibit deficiencies in leukocyte differentiation, proliferation and immune response.

For proteolytic enzymes, it is their activity, rather than mere expression level, that dictates their functional role in cell physiology and pathology. Accordingly, molecules that inhibit the activity of caspases are useful as therapeutic agents in the treatment of diseases and in the development of specific imaging biomarkers that visualize the proteolytic activity as well as their inhibition through drug candidates may accelerate target validation, drug development and even clinical trials (H. Pien et al. Drug Discovery Today, 2005, 10, 259-266). Using activity based imaging reagents, a specific protein or protein family can be readily monitored in complex protein mixtures, intact cells, and even *in vivo.* Furthermore, enzyme class specific probes can be used to develop screens for small molecule inhibitors that can be used for functional studies (D.A. Jeffery, M. Bogyo Curr. Opp. Biotech. 2003, 14, 87-95).

So far, activity based imaging probes incorporating a peptide substrate have been developed to monitor and label in cell based assays caspase-1 (W.Nishii et al., FEBS Letters 2002, 518, 149-153) or caspase-3 (S. Mizukami et al., FEBS Letters 1999, 453, 356-360). Furthermore a near-infrared fluorescent probe has been reported to detect caspase-1 activity in living animals (S. Messerli et al., Neoplasia 2004, 6, 95-105).

US 2004/002128 disclose the construction of FRET probes containing the caspase-3 recognition motif GGDEVDGG. A terpyridine-based europium (III) chelate probe is described using Asp-Glu-Val-Asp-Lys as recognition motif, which is suitable for detecting caspase-3 activity (S. Poupart et al., Organic & Biomolecular Chemistry 2006, Vol. 4, No. 22, 4165-417). A fluorescence probe for detecting cathepsin D activity is described by Tung et al. (Bioconjugate Chemistry, 1999, 10, 892-896). A probe with a caspase-3 recognition site, linker and biotin conjugation suitable for a coupled bioluminescent assay is also described (P. Tawa et. al., Analytical Biochemistry 2006, Vol. 350, no. 1, 32-40).

The enzymatic mechanism used by the caspases has been well studied and is highly conserved. From the investigation and screening data of cleavable peptides, electrophilic substrate analogs have been developed that only react in the context of this conserved active site. The electrophilic center in such probes is usually part of a so called "warhead", a molecular entity that is optimized in its electrophilic character and its geometric placement to fit perfectly into the active site of caspases, where it reacts with the catalytic cysteine residue. A wide variety of such electrophilic substrates have been described as mechanism based cysteine protease inhibitors including for example but not exclusively: diazomethyl ketones, fluoromethyl ketones, acyloxymethyl ketones, O-acylhydroxylamines, vinyl sulfones and epoxysuccinic derivatives (S. Verhelst, M. Bogyo QSAR Comb. Sci. 2005, 24, 261-269).

Another tool to monitor protease activity consists in bioluminescent assay. This method make use of amino-modified beetles pro-luciferine (caged luciferine) or carboxy-terminal derivatives thereof linked to a protease substrate. A first proteolytic cleavage releases luciferine which is subsequently converted by luciferase, detectable as a luminescent signal. This secondary assay has a similar application spectra than fluorescent probes and present the additionally advantage of a high signal to noise ratio.

To be effective as biological tools, protease inhibitors must be not only very potent but also highly selective in binding to a particular protease. The development of small molecule inhibitors for specific proteases has often started from peptide substrates. Although peptides display a diverse range of biological properties, their use as drugs can be compromised by their instability and their low oral bioavailability. To be effective drugs, protease inhibitors with reduced peptide-like character, high stability against non selective proteolytic degradation, high selectivity for a given protease, and good bioavailability to the *locus* of protease action are desirable. These requirements led to the development of caspases inhibitors A-B where A is a chemical scaffolds covalently linked to an electrophilic warhead B. In presence of caspase, B reacts covalently with the catalytic cysteine (mechanism based inhibitor). In many cases, the selectivity and pharmacokinetic properties of such inhibitors were successfully optimized in the context of biomedical research. To enable the effective nucleophilic attack of the catalytic cysteine, the electrophilic center of such inhibitors must be oriented precisely within the active site of the enzyme. The special arrangement of catalytic cysteine to the electrophilic carbon atom of the warhead corresponds well to the spatial arrangement of the catalytic cysteine and the peptide carbonyl of a scissile peptide substrate. This comparison guided us to the idea that a "redesign" of optimized covalent inhibitors (with a chemical scaffold A and an electrophilic warhead B) into a cleavable substrate should be possible. Since the chemical scaffold A can be considered as the determinant of inhibitor selectivity, our approach would allow for the transfer of the selectivity or parts of the selectivity of an optimized inhibitor into an activity based chemical probe. We refer to this process as "reversed design" of selective activity based probes from selective caspase inhibitors.

The invention relates to molecular probes for cysteine proteases of the formula (I)

{L1-R1-L}ₙ-A-CO-NH-R2-L2 (I)

wherein
A is a group recognizable by a caspase;
wherein A is recognizable by caspase-1 and is selected from a group 1. - 28. of the table below:

| | |
|---|---|
| 1. | 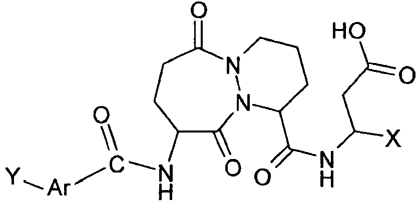 |
| | wherein Ar is phenyl if n is 1 and Ar is isoquinoline if n is 0,preferably in the configuration: |
| | 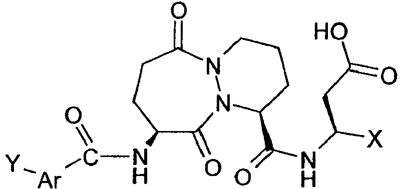 |
| 2. | 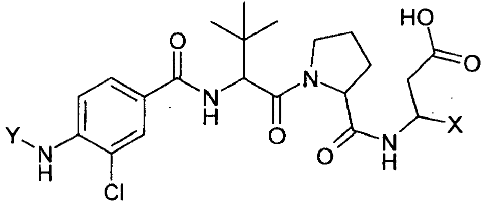 |
| | preferably in the configuration: |
| | 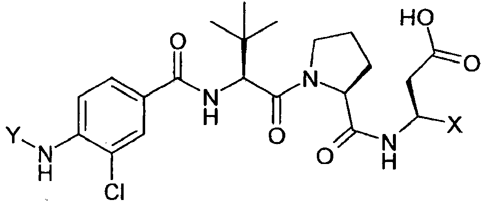 |
| 3. | 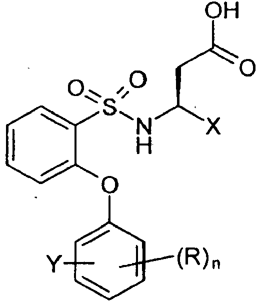 |
| | wherein R is (C₁-C₅)alkyl, phenyl, (C₅-C₆)cycloalkyl; and n is 1-3; |
| 4. | 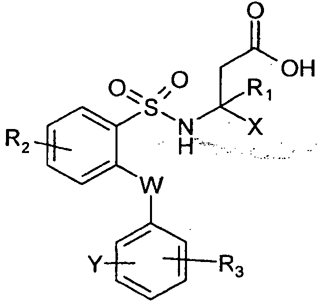 |
| | preferably in the configuration: |
| | 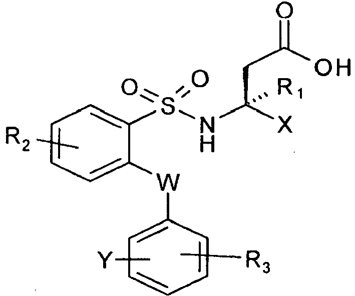 |
| | wherein R₁ is hydrogen, (C₁-C₆)alkyl, aryl or -CH₂-aryl, R₂ and R₃ are independently of each other hydrogen, or an aryl, substituted aryl, heteroaryl, substituted heteroaryl, cycloalkyl, substituted cycloalkyl, heterocycle, or substituted heterocycle group that is fused to the phenyl group that contains a group R₂ as a substituent; |
| | W is a bond, NR₅, CO, S, O, SO₂,O(CHR₅)ₙ-, CHR₅, NR₅CO,CONR₅, OCHR₅, CHR₅O, SCHR₅, CHR₅S, SO₂NR₅, (C₁-C₆)alkyl, NR₅SO₂, CH₂CHR₅, CHR₅CH₂, COCH₂ or CH₂CO; wherein R₅ is independently hydrogen, (C₁-C₆)alkyl, aryl, (CH₂)ₙ-aryl, or (CH₂)ₙ-cycloalkyl; and each n is independently 0 to 5; |
| 6. | 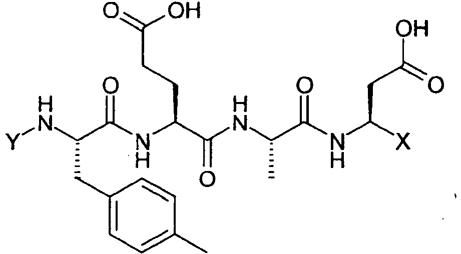 |
| 7. | 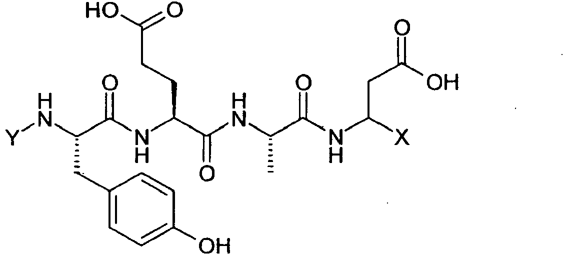 |
| 8. | 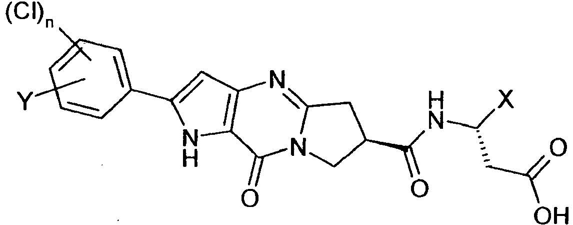 |
| | wherein n is 0 or 1; |
| 9. | 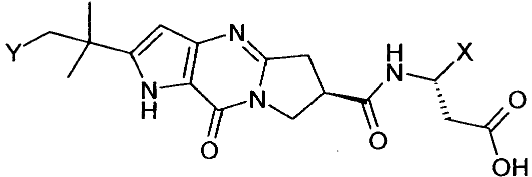 |
| 10. | 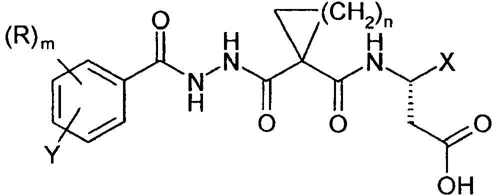 |
| | wherein n is 1-4 |
| | m is 1 or 2, and |
| | R is methyl or methoxy; |
| 11. | 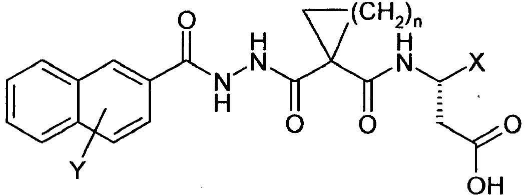 |
| | wherein n is 1-4; |
| 12. | 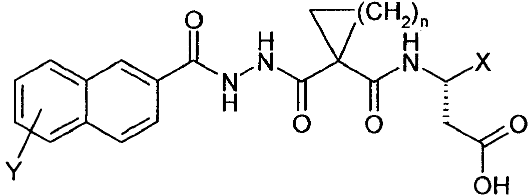 |
| | wherein n is 1-4; |
| 13. | 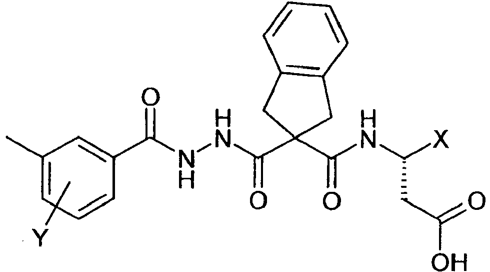 |
| 14. | 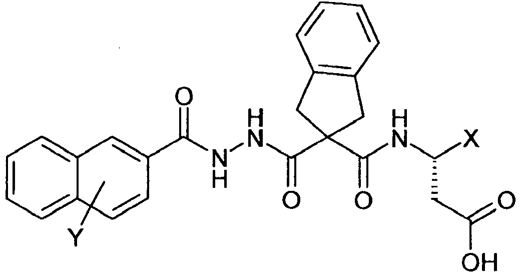 |
| 15. | 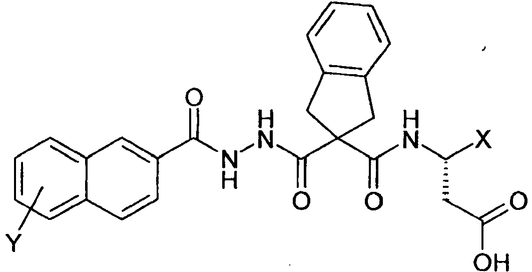 |
| 16. | 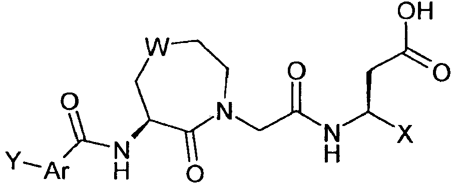 |
| | wherein W is S or S(O)₂, and Ar is aryl or heteroaryl, preferably phenyl, naphthyl, benzothiophene or isoquinolyl; |
| 17. | 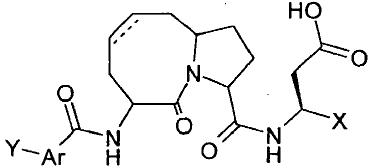 |
| | wherein Ar is an aryl or heteroaryl group selected from phenyl, benzothiophene, isoquinolyl, cinnamyl, naphthyl, which is optionally once or independently twice substituted by methoxy, chloro, methyl, CF₃, and wherein |
| | 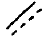 |
| | means either a single or a double bond; |
| 18. | 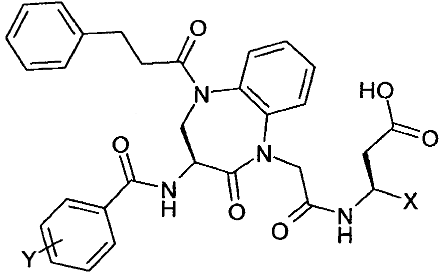 |
| 19. | 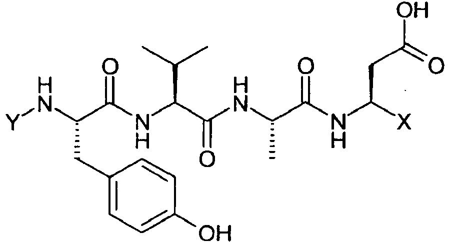 |
| 20. | 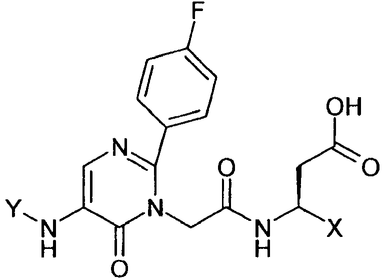 |
| 21. | 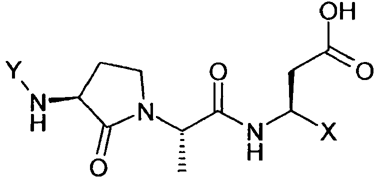 |
| 22. | 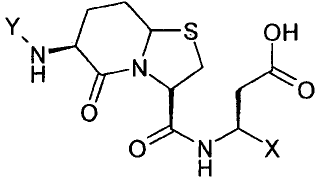 |
| 23. | 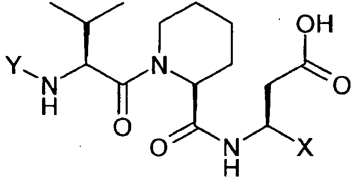 |
| 24. | 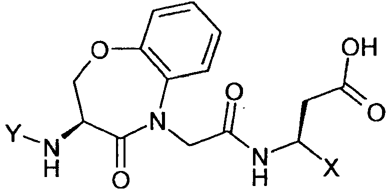 |
| 25. | 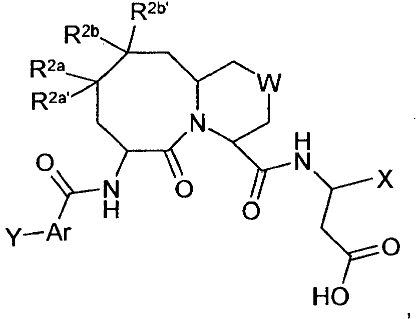 |
| | preferably in the all-(S) configuration, |
| | wherein Ar is aryl or heteroaryl; |
| | W is CH₂, O or NR9 wherein R9 is hydrogen or (C₁-C₆)alkyl, aryl, heteroaryl, heterocyclyl; |
| | R^{2a}, R^{2a'}, R^{2b} and R^{2b}' are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond; |
| 26. | 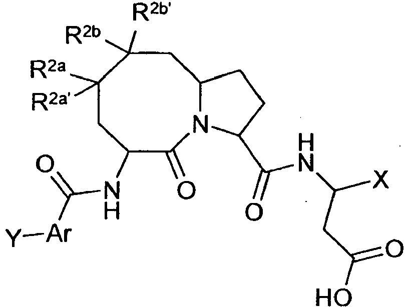 |
| | preferably in the all-(S) configuration, |
| | wherein Ar is aryl or heteroaryl; |
| | R^{2a}, R^{2a'}, R^{2b} and R^{2b}' are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond; |
| 27. | 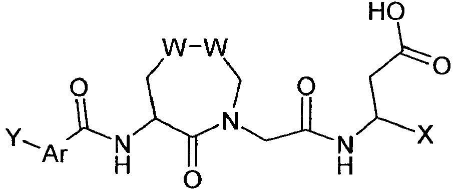 |
| | preferred in the all-(S) configuration |
| | wherein Ar is aryl or heteroaryl; and |
| | W is independently selected from: C(R¹)₂; C(O); NR²; S; S(O); S(O)₂; wherein R¹ and R² are independently hydrogen, [C(R³)₂]ₚ(CH=CH)_{q}R³, C(=Z)R³, C(=Z)[C(R³)²]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂. SO₂R³, P(O)(OR³)R³, P(O)(OR³)₂; wherein p is 0 to 12; wherein q is 0 to 12; |
| | wherein Z is O, S, NR³; wherein R³ is independently hydrogen, alkyl, cycloalkyl, aryl, heteroaryl, heterocylyl. |
| 28. | 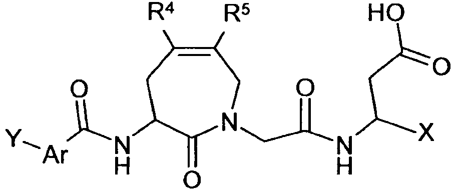 |
| | preferred in the all-(S) configuration |
| | wherein Ar is aryl or heteroaryl; and R⁴ and R⁵ are independently selected from: C(R¹)₂; C(O); NR²; S; S(O); S(O)₂; wherein R¹ and R² are independently hydrogen, [C(R³)₂]ₚ(CH=CH)_{q}R³, C(=Z)R³, C(=Z)[C(R³)₂]ₚ(CH=CH)_{q}R³, C(=Z)N(R³)₂, C(=Z)NR³N(R³)₂, CN, CF, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂, SO₂R³, P(O)(OR³)R³, P(O)(OR³)₂; wherein p is 0 to 12; wherein q is 0 to 12; wherein Z is O, S, NR³; wherein R³ is independently hydrogen, alkyl, cycloalkyl, aryl, heteroaryl, heterocylyl; |

or A is recognisable by caspase-3 and is selected from a group 29. - 42. of the table below :

| | |
|---|---|
| 29. | 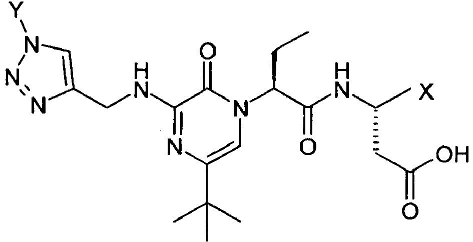 |
| 30. | 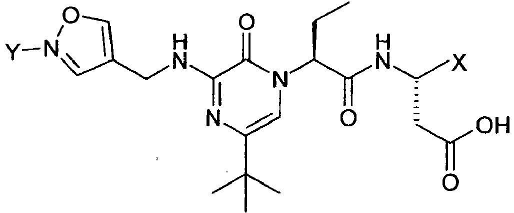 |
| 31. | 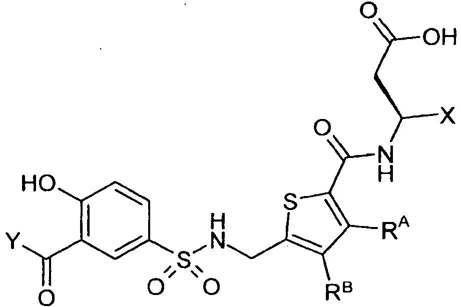 |
| | wherein R^{A} and R^{B} are independently hydrogen; (C₁-C₆)alkyl, hydroxyl, (C₁-C₆)alkoxy or halogen. |
| 32. | 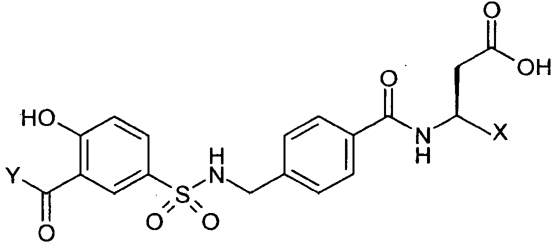 |
| 33. | 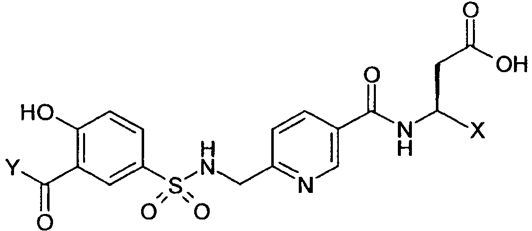 |
| 34. | 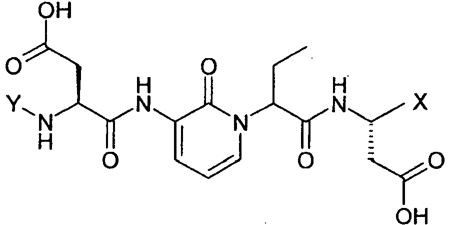 |
| 35. | 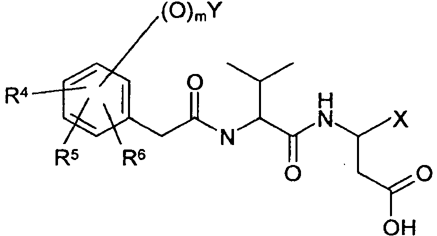 |
| | preferably in the all-(S) configuration, |
| | wherein m is 0 or 1; and |
| | R⁴, R⁵ and R⁶ are independently selected from the group consisting of: |
| | 1) H, |
| | 2) halogen, |
| | 3) (C₁-C₄)alkoxy optionally substituted with 1-3 halogen atoms, |
| | 4) NO₂, |
| | 5) OH, |
| | 6) benzyloxy, the benzyl portion of which is optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 7) NH(C₁-C₄)acyl, |
| | 8) (C₁-C₄)acyl, |
| | 9) O-(C₁-C₄)alkyl-CO₂H, optionally esterified with a (C₁-C₆)alkyl or a (C₅-C₇)cycloalkyl group, |
| | 10) CH=CH-CO₂H, |
| | 11) CO₂H, |
| | 12) (C₁-C₅)alkyl-CO₂H, |
| | 13) C(O)NH₂, optionally substituted on the nitrogen atom by 1-2 (C₁-C₄)alkyl groups; |
| | 14) (C₁-C₅)alkyl-C(O)NH₂, optionally substituted on the nitrogen atom by 1-2 (C₁-C₄)alkyl groups; |
| | 15) S(O)₀₋₂-(C₁-C₄)alkyl; |
| | 16) (C₁-C₂)alkyl-S(O)₀-₂-(C₁-C₄)alkyl; |
| | 17) S(O)₀₋₂-(C₁-C₆)alkyl or S(O)₀₋₂-phenyl, said alkyl and phenyl portions thereof being optionally substituted with 1-3 members selected from the group consisting of: halogen, CN, (C₁-C₄)alkyl and (C₁-C₄)alkoxy, said alkyl and alkoxy being optionally substituted by 1-3 halogen groups, |
| | 18) benzoyl optionally substituted by 1-2 members selected from the group consisting of: halogen, CN, (C₁-C₄)alkyl and (C₁-C₄)alkoxy, said alkyl and alkoxy groups being optionally substituted by 1-3 halogen groups, |
| | 19) phenyl or naphthyl, optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 20) CN, |
| | 21) (C₁-C₄)alkylene-HET2, wherein HET2 represents a 5-7 membered aromatic or non-aromatic ring containing 1-4 heteroatoms selected from O, S, NH and N(C₁-C₄) and optionally containing 1-2 oxo groups, and optionally substituted with 1-3 (C₁-C₄)alkyl, OH, halogen or (C₁-C₄)acyl groups; |
| | 22) O-(C₁-C₄)alkyl-HET3, wherein HET3 is a 5 or 6 membered aromatic or non-aromatic ring containing from 1 to 3 heteroatoms selected from O, S and N, and optionally substituted with one or two groups selected from halogen and (C₁-C₄)alkyl, and optionally containing 1-2 oxo groups, and |
| | 23) HET4, wherein HET4 is a 5 or 6 membered aromatic or non-aromatic ring, and the benzofused analogs thereof, containing from 1 to 4 heteroatoms selected from O, S and N, and is optionally substituted by one or two groups selected from halogen, (C₁-C₄)alkyl and (C₁-C₄)acyl; and wherein halogen includes F, Cl, Br and I. |
| 36. | 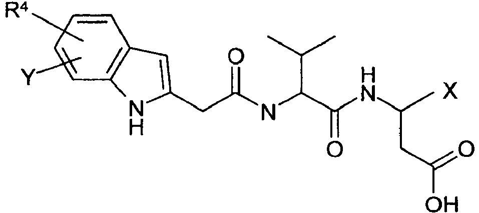 |
| | preferably in the all-(S) configuration, |
| | wherein R⁴ is selected from the group consisting of: |
| | 1) H, |
| | 2) halogen, |
| | 3) (C₁-C₄)alkoxy optionally substituted with 1-3 halogen atoms, |
| | 4) NO2, |
| | 5) OH, |
| | 6) benzyloxy, the benzyl portion of which is optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 7) NH(C₁-C₄)acyl, |
| | 8) (C₁-C₄)acyl, |
| | 9) O-(C₁-C₄)alkyl-CO₂H, optionally esterified with a (C₁-C₆)alkyl or a (C₅-C₇)cycloalkyl group, |
| | 10) CH=CH-CO₂H, |
| | 11) CO₂H, |
| | 12) (C₁-C₅)alkyl-CO₂H, |
| | 13) C(O)NH₂, optionally substituted on the nitrogen atom by 1-2 (C₁-C₄)alkyl groups; |
| | 14) (C₁-C₅)alkyl-C(O)NH₂, optionally substituted on the nitrogen atom by 1-2 (C₁-C₄)alkyl groups; |
| | 15) S(O)₀₋₂-(C₁-C₄)alkyl; |
| | 16) (C₁-C₂)alkyl-S(O)₀₋₂-(C₁-C₄)alkyl; |
| | 17) S(O)₀₋₂-(C₁-C₆)alkyl or S(O)₀₋₂-phenyl, said alkyl and phenyl portions thereof being optionally substituted with 1-3 members selected from the group consisting of: halogen, CN, (C₁-C₄)alkyl and (C₁-C₄)alkoxy, said alkyl and alkoxy being optionally substituted by 1-3 halogen groups, |
| | 18) benzoyl optionally substituted by 1-2 members selected from the group consisting of: halogen, CN, (C₁-C₄)alkyl and (C₁-C₄)alkoxy, said alkyl and alkoxy groups being optionally substituted by 1-3 halogen groups, |
| | 19) phenyl or naphthyl, optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 20) CN, |
| | 21) (C₁-C₄)alkylene-HET2, wherein HET2 represents a 5-7 membered aromatic or non-aromatic ring containing 1-4 heteroatoms selected from O, S, NH and N(C₁-C₄) and optionally containing 1-2 oxo groups, and optionally substituted with 1-3 (C₁-C₄)alkyl, OH, halogen or (C₁-C₄)acyl groups; |
| | 22) O-(C₁-C₄)alkyl-HET3, wherein HET3 is a 5 or 6 membered aromatic or non-aromatic ring containing from 1 to 3 heteroatoms selected from O, S and N, and optionally substituted with one or two groups selected from halogen and (C₁-C₄)alkyl, and optionally containing 1-2 oxo groups, and |
| | 23) HET4, wherein HET4 is a 5 or 6 membered aromatic or non-aromatic ring, and the benzofused analogs thereof, containing from 1 to 4 heteroatoms selected from O, S and N, and is optionally substituted by one or two groups selected from halogen, (C₁-C₄)alkyl and (C₁-C₄)acyl; and wherein halogen includes F, Cl, Br and I. |
| 37. | 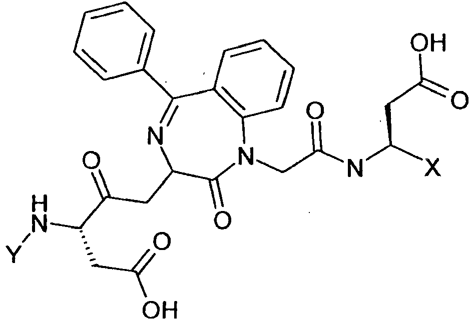 |
| 38. | 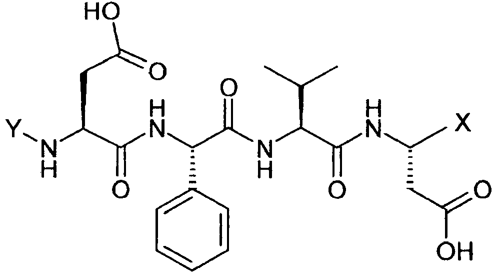 |
| 39. | 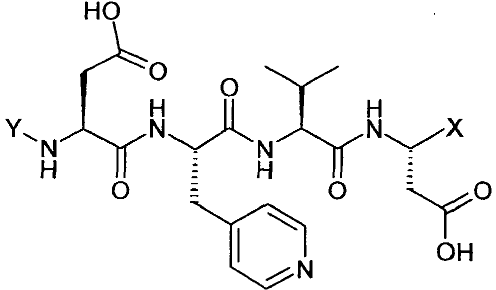 |
| 40. | 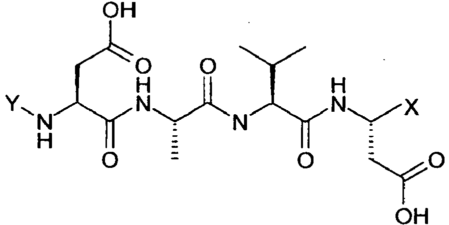 |
| 41. | 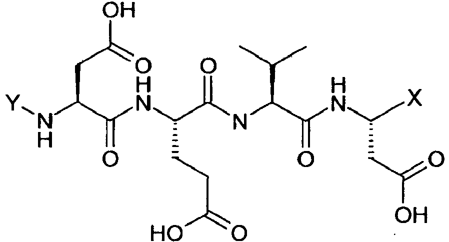 |
| 42. | 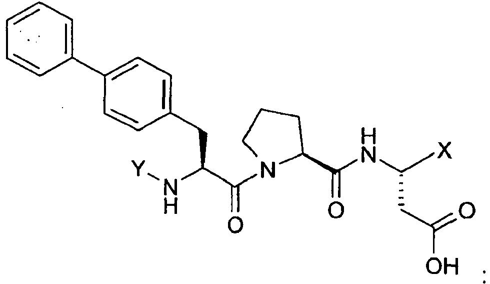 |

or A is recognizable by caspase-8 and is selected from a group 43. - 44. of the table below:

| | |
|---|---|
| 43. | 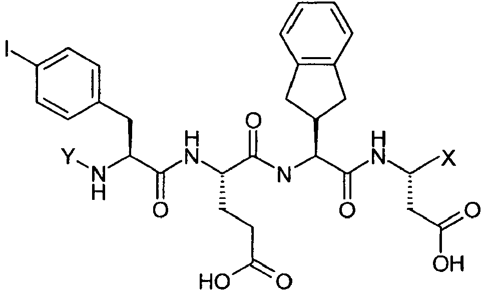 |
| 44. | 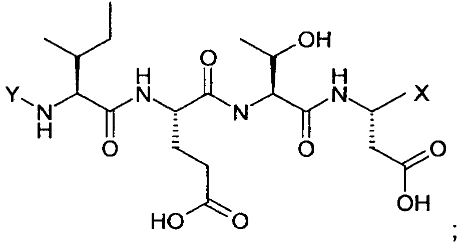 |

X is -CONH-R2-L2;
Y is-L-R1-L1;
R1 is a linker;
R2 is a bond or a linker
wherein the linker is a straight or branched chain alkylene group with 1 to 300 carbon atoms, wherein optionally
(a) one or more carbon atoms are replaced by oxygen, in particular wherein every third carbon atom is replaced by oxygen, e.g. a poylethyleneoxy group with 1 to 100 ethyleneoxy units; and/or
(b) one or more carbon atoms are replaced by nitrogen carrying a hydrogen atom, and the adjacent carbon atoms are substituted by oxo, representing an amide function -NH-CO-; and/or
(c) one or more carbon atoms are replaced by an ester function -O-CO-;
(d) the bond between two adjacent carbon atoms is a double or a triple bond; and/or
(e) two adjacent carbon atoms are replaced by a disulfide linkage;
L is a bond or a group selected from 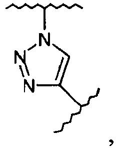 -(NRx)-, -O-, -C=N-, -C(=O)-, -C(=O)-NH-, -NH-C(=O)-, -C(=O)H, -CRx=CRy-, -C=C- and phenyl, wherein Rx and Ry are independently H or (C₁-C₆)alkyl;
L1 and L2 are, independent of each other, at least one label optionally bound to a solid support; and
n is 1;
or
R2 is a bond;
L2 is a substrate, suitable for a coupled bioluminescent assay;
Y is H; and
n is 0.

In their most basic form, the chemical probe consists of four functional elements, a) an amide group -CO-NH- as a reactive group, that can be cleaved by the action of a protease, b) a scaffold A which defines the selectivity for a given protease target, c) linker moieties R1 and R2 to connect subunits to each other and d) set of label L1 and L2 for detection.

Group A is preferably the main determinant for specificity towards a given caspase or a group of caspases, preferably for caspase-1,3 and 8 as shown in compounds 1- 44 above. Activity-based probes of the present invention show selectivity for a given caspase of the factor 1000 to 1, preferably a factor 10 to 1, wherein selectivity is defined by the relative turnover number (turnover number (turnover number with enzyme 1 versus turnover number with enzyme 2) at a preferred substrate concentration. The relative turnover number is determined for each enzyme pair by dividing the turnover number of the enzyme of interest (enzyme 1) by the turnover number of another enzyme against which selectivity is desired (enzyme 2). For *in vivo* applications high selectivity is desired at low (e.g. micromolar or submicromolar) substrate concentrations.

Scheme 1 shows the reaction of a protease P with a substrate wherein A represents the specificity determinant, and P represents the protease with its reactive cysteine comprising the thiol group S⁻:

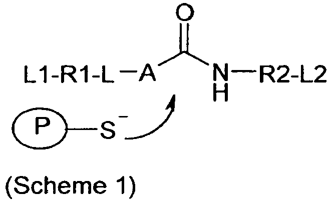

The reaction rate is dependent on the structure of the substrate.

The linker group R1 or R2 is preferably a flexible linker connected to a label L1 or L2, respectively, or a plurality of same or different label L2 or L1. The linker group is chosen in the context of the envisioned application, i.e. in context of an activity based imaging probe for a specific protease. The linker may also increase the solubility of the substrate in the appropriate solvent. The linkers used are chemically stable under the conditions of the actual application. The linker does not interfere with the reaction of a selected protease target nor with the detection of the label L1 and/or L2, but may be constructed such as to be cleaved at some point in time.

The label L1 and L2 of the substrate can be chosen by those skilled in the art dependent on the application for which the probe is intended.

The label L1 and L2 is independently of each other a spectroscopic probe such as a fluorophore; a quencher or a chromophore; a magnetic probe; a contrast reagent; a molecule which is one part of a specific binding pair which is capable of specifically binding to a partner; a molecule which is a substrate for an enzyme, a molecule covalently attached to a polymeric support, a dendrimer, a glass slide, a microtiter plate known to those proficient in the art; or a molecule possessing a combination of any of the properties listed above.

A preferred embodiment of the present invention is the use of a modified aminoluciferin or a carboxy-terminal protected derivative thereof as a reporter group, which upon cleavage from the central scaffold A can generate a luminescent signal through its conversion by a luciferase. Therefore, label L2 may alternatively be a substrate, suitable for a coupled bioluminescent assay, characterized in a modified aminoluciferin or a carboxy-terminal protected derivative thereof as a reporter group.

US7148030 discloses examples of bioluminescent protease assays comprising peptides as caspase substrates which are linked to modified aminoluciferines.

Preferred is a probe which consists of intramolecularly quenched fluorescent probes comprising a polymeric backbone and a plurality of fluorochromes covalently linked *via* scaffold A to the backbone at a density which leads to fluorescent quenching.

Another preferred embodiment of the present invention is the use of a dendritic macromolecule onto which two or more fluorophores are covalently linked *via* scaffold A at a density which leads to fluorescent quenching. The use of a polymeric probe has the advantage of localized probe delivery (targeting) and a prolonged circulation time in the blood stream of an animal or humans. Polymer conjugation alters the biodistribution of low-molecular-weight substances, enabling tumour-specific targeting (by the enhanced permeability and retention effect (EPR effect)) with reduced access to sites of toxicity and the combination of polymer conjugates with low-molecular-weight imaging probes is a most preferred embodiment of the present invention for imaging of multicellular organisms including mammals such as mice, rats etc.. The polymeric backbone can consist of any biocompatible polymer and may comprise a polypeptide, a polysaccharide, a nucleic acid or a synthetic polymer. A comprehensive summary of polymers useful in the context of the present invention can be found in M.J. Vincent et al. Trends Biotech. 2006, 24, 39-47 and R. Duncan, Nature Reviews Cancer, 2006, 688-701. A further description of polymers useful in the context of the present invention is disclosed in WO99/58161. The polymeric or dendrimeric probe can comprise protective chains covalently linked to the backbone or the dendritic molecule. Protective chains include polyethylene glycol, methoxypolyethyleneglycol and further copolymers of ethyleneglycol.

The probe of the present invention can additionally comprise a targeting moiety such as an antibody, an antibody fragment, a receptor-binding ligand, a peptide fragment or a synthetic protein inhibitor.

Label L1 and L2 can further be positively charged linear or branched polymers. Said polymers are known to those skilled in the art to facilitate the transfer of attached molecules over the plasma membrane of living cells. This is especially preferred for substances which otherwise have a low cell membrane permeability or are in effect impermeable for the cell membrane of living cells. A non cell permeable chemical probe will become cell membrane permeable upon conjugation to such a group L1 or L2. Such cell membrane transport enhancer groups L1 and L2 comprise, for example, a linear poly(arginine) of D- and/or L-arginine with 6 - 15 arginine residues, linear polymers of 6 - 15 subunits each of which carry a guanidinium group, an oligomer or a short-length polymer of from 6 to up to 50 subunits, a portion of which have attached guanidinium groups, and/or parts of the sequence of the HIV-tat protein, for example the subunit Tat49-Tat57 (RKKRRQRRR in the one letter amino acid code). A linear poly(arginine) of D-and/or L-arginine with 6 - 15 arginine residues is preferably utilized as polymeric label in case L1 is one member and L2 is the other member of two interacting spectroscopic probes L1 / L2, such as in a FRET pair.

Most preferred as label L1 and/or L2 are spectroscopic probes. Most preferred as label L2 are molecules representing one part of a spectroscopic interaction pair with L1, furthermore a label which is capable of specifically binding to a partner and molecules covalently attached to a solid support.

Particularly preferred are label such that L1 is one member and L2 is the other member of two interacting spectroscopic probes L1 / L2, wherein energy can be transferred non-radiatively between the donor and acceptor (quencher) through either dynamic or static quenching. Such said pair of label L1 / L2 changes its spectroscopic properties upon reaction/cleavage through the corresponding caspase protease. An example of such a pair of label L1 / L2 is a FRET (Förster resonance energy transfer) pair, e.g. a pro-fluorescent probe covalently labelled at one end (e.g. L1) with a donor (reporter), and the another position (L2) with an acceptor (quencher), or *vice versa.*

In particular, L1 is a donor (reporter) and L2 is an acceptor (quencher), or L1 is a quencher and L2 is a reporter. In using this probe, the reaction of the cystein protease with the probe will lead to a change in fluorescence. The reporter-quencher distance within the double labelled substrate is changed upon reaction with the protease leading to a spatial separation of reporter and quencher which causes the appearance of fluorescence or change of the emission wavelength. A broad selection of reporter groups may be used as label L1 or L2, respectively, including e.g. near infra-red emitting fluorophores. The substrate containing reporter and quencher remains dark until it reacts with the protease, whereupon the reaction mixture is "lit up" switching on the fluorophore emission, since the reporter label and the quencher label are now spatially separated. Fluorescence quenching and energy transfer can be measured by the emission of only one of the two labels, the quenched or energy donor label. When energy transfer occurs and the energy accepting label is also fluorescent, the acceptor label fluorescence can also be measured. A donor label of these two interacting label can be chosen from chemoluminescent donor probes which eliminates the need of an excitation lamp and reduces acceptor background fluorescence. The mentioned particular method using such double-labelled substrates is useful to determine reaction kinetics based on fluorescence time measurements, and may be applied *in vivo* as well as *in vitro.*

Alternatively, the label L2 may be a solid support or be additionally attached to solid support or attached or attachable to a polymer/ solid support. Linear poly(arginine) of D- and/or L-arginine with 6 - 15 arginine residues is preferably utilized as polymeric label for a L1 / L2 FRET pair.

Particular preferred combinations are two different affinity label, especially a pair of spectroscopic interacting label L1 / L2, e.g. a FRET pair. An affinity label is defined as a molecule which is one part of a specific binding pair which is capable of specifically binding to a partner. A specific binding pair considered is e.g. biotin and avidin or streptavidin furthermore methotrexate, which is a tight-binding inhibitor of the enzyme dihydrofolate reductase (DHFR).

Appropriate pairs of reporters and quenchers can bee chosen by those skilled in the art. Typically reporter and quencher are fluorescent dyes with large spectral overlap as, for example, fluorescein as a reporter and rhodamine as a quencher. Other quenchers are gold clusters, and metal cryptates.

A second class of quenchers used in this invention are "dark quenchers", i.e. dyes without native fluorescence having absorption spectra that overlap with the emission spectra of common reporter dyes leading to maximal FRET quenching. Furthermore pairs of dyes can be chosen such that their absorption bands overlap in order to promote a resonance dipole-dipole interaction mechanism within a ground state complex (static quenching).

Particular fluorophores and quenchers considered are: Alexa dyes, including Alexa 350, Alexa 488, Alexa 532, Alexa 546, Alexa 555, Alexa 635 and Alexa 647 (US5696157, US6130101, US6716979); dimethylaminocoumarin (e.g. 7-dimethylaminocoumarin-4-acetic acid succinimidyl ester supplied as product D374 by Invitrogen, CA 92008, USA); quenchers QSY 35, QSY 9 and QSY 21 (Invitrogen, CA 92008, USA); Cyanine-3 (Cy 3), Cyanine 5 (Cy 5) and Cyanine 5.5 (Cy 5.5) (Amersham - GE Healthcare, Solingen, Germany); BHQ-1, BHQ-2 and BHQ-3 (Black Hole QuencherTM of Biosearch Technologies, Inc., Novato, CA 94949, USA); fluorophores ATTO 488, ATTO 532, ATTO 600 and ATTO 655 and quenchers ATTO 540Q and ATTO 612Q (Atto-Tec, D57076 Siegen, Germany); fluorophores DY-505, DY-547, DY-632 and DY-647 (Dyomics, Jena, Germany); 5/6-carboxyfluorescein, tetramethylrhodamine, 4-dimethylaminoazobenzene-4'-sulfonyl derivatives (Dabsyl) and 4-dimethylaminoazobenzene-4'-carbonyl derivatives (Dabcyl). These can be advantageously combined in the following combinations:

| Fluorophore | Quencher |
|---|---|
| • Alexa 350, dimethylaminocoumarin, 5/6- carboxyfluorescein, Alexa 488, ATTO 488, DY-505 | • Dabsyl, Dabcyl, BHQ 1, QSY 35 |
| • 5/6-carboxyfluorescein, Alexa 488, Alexa 532, Alexa 546, Alexa 555, ATTO 488, ATTO 532, tetramethylrhodamine, Cy 3, DY-505, DY-547, | • BHQ 2, QSY 9, ATTO 540Q |
| • Alexa 635, Alexa 647, ATTO 600, ATTO 655, DY-632, Cy 5, DY-647 Cy 5.5 | • BHQ 3, ATTO 612Q, QSY 21 |

Bioluminescent assays that are linked to an enzymatic event yield light coupled to the instantaneous rate of catalysis. The method comprises an amino-modified beetle amino-luciferin or a carboxy-terminal protected derivative thereof were the amino-group of aminoluciferin is linked via an amide bond to the central scaffold A, resulting in a substrate that is recognized and subsequently cleaved by a caspase.

The enzymatic activity of a caspase leads to the cleavage of the peptide bond which links the aminoluciferin to the scaffold A liberating the aminoluciferin a substrate for a luciferase. The following reaction of the luciferase with its substrate yields a detectable signal (luminescence). The method thus relates caspase activity with a second enzymatic reaction, generating luminescence as a read-out signal. This type of assay requires the development of a "pro-luciferin" ("caged luciferin"), which is recognized by a luciferase as a substrate only when converted to luciferine by a precedent enzymatic event e.g. proteolytic cleavage. In this way, the luminescent signal is directly dependent on the previous enzymatic event. It is therefore a further embodiment of the present invention to provide a probe for detecting proteolytic activity of caspases by means of luminescence.

In a particular embodiment, the method involves a substrate wherein L2 is a solid support or attached to a solid support further carrying one member of the reporter / quencher pair, or wherein L2 is a combination of a solid support and one member of the reporter / quencher pair, and L1 is the other member of this pair. In this way, the dark solid support becomes fluorescent upon reaction with the appropriate protease.

A solid support, may be a glass slide, a microtiter plate or any polymer known to those proficient in the art, e.g. a functionalized polymers (preferably in the form of beads), chemically modified oxidic surfaces, e.g. silicon dioxide, tantalum pentoxide or titanium dioxide, or also chemically modified metal surfaces, e.g. noble metal surfaces such as gold or silver surfaces. A solid support may also be a suitable sensor element.

The compound of the formula (I) comprises a group A being an inhibitor of caspase-1. The preparation of scaffolds A having caspase-1 inhibitory activity is for example described in US5670494; W09526958; W09722619; W09816504; WO0190063; W003106460; W003104231; W003103677; W. G. Harter, Bioorg. Med. Chem. Lett. 2004, 14, 809-812; Shahripour et al., Bioorg. Med. Chem. Lett. 2001, 11, 2779-2782; Shahripour et al., Bioorg. Med. Chem. 2002, 10, 31-40; M. C. Laufersweiler et al., Bioorg. Med. Chem. Lett. 2005, 15, 4322-4326; K. T. Chapman, Bioorg. Med. Chem. Lett. 1992, 2, 613-618; Dolle et al., J. Med. Chem. 1997, 40, 1941-1946; D. L. Soper et al., Bioorg. Med. Chem. Lett. 2006, 16, 4233-4236; D. L. Soper et al., Bioorg. Med. Chem. 2006, 14, 7880-7892; D. J. Lauffer et al., Bioorg. Med. Chem. Lett. 2002, 12,1225-1227; and C. D. Ellis et al., Bioorg. Med. Chem. Lett. 2006, 16, 4728-4732.

The following definitions apply if not otherwise stated:
Alkyl means a straight or branched chain hydrocarbon having 1 to 6 carbon atoms. Examples of (C₁-C₆)alkyl groups are methyl, ethyl, propyl, isopropyl, isobutyl, butyl, tert-butyl, sec-butyl, pentyl, and hexyl.

Acyl is defined as a group -C(=O)alkyl.

Aryl is defined as an aromatic hydrocarbon having 6 to 10 carbon atoms. Examples of aryl groups include phenyl and naphthyl.

Heteroaryl is defined as an aryl group wherein one or more carbon atom of the aromatic hydrocarbon has been replaced with a heteroatom wherein the term "heteroatom" includes oxygen, nitrogen, sulfur, and phosphorus. Examples of heteroaryl groups include furan, thiophene, benzothiophene, pyrrole, thiazole, pyridine, pyrimidine, pyrazine, benzofuran, indole, coumarin, quinoline, isoquinoline, and naphthyridine.

Cycloalkyl means a cyclic alkyl group having 3 to 10 carbon atoms. Examples of cycloalkyl groups include cyclopropane, cyclobutane, cyclopentane, and cyclohexane.

Heterocycle or heterocylyl means a cycloalkyl group on which one or more carbon atom has been replaced with a heteroatom. Examples of heterocycles include piperazine, morpholine, and piperidine.

The aryl, heteroaryl, or cycloalkyl groups may be substituted with one or more substituents, which can be the same or different. Examples of suitable substituents include alkyl, alkoxy, thioalkoxy, hydroxy, halogen, trifluoromethyl, amino, alkylamino, dialkylamino, NO₂, CN, CO₂H, CO₂alkyl, SO₃H, CHO, C(=O)alkyl, CONH₂, CONH-alkyl, CONHR_{q}, C(=O)N(alkyl)₂, (CH₂)ₙNH₂, OH, CF₃, O(C₁-C₆)alkyl, (CH₂)ₙNH-alkyl, NHR_{q}, NHCOR_{q}, phenyl, where n is 1 to 5 and Rq is hydrogen or (C₁-C₆)alkyl.

The activity based probes of the present invention may be synthesized by using appropriate protecting group chemistry known in the art to build up the central scaffold A and to attach either linker and label L1 or L2 to this unit via a group L and a group -C(O)-NH-. Appropriate building blocks as well as FRET-pairs such as the cyanine-dyes (e.g. Cy3 B, Cy 5.5, Cy 7) are commercially available (e.g. Sigma-Aldrich, GE-Healthcare). For a subset of probe, descried in this invention, the solid-phase synthesis method is particularly useful (B. J. Merrifield, Methods in Enzymology 1997, 289, 3-13). Depending on the synthetic requirements, attachment linker, quencher or fluorophore may be done on the solid support or by solution phase chemistry.

In general, reactive side chain residues on the central scaffold A and optionally the group L will be protected and liberated sequentially for further modification with the subunits L1 R1 and L2R2 respectively. Conjugation of these subunits can be accomplished by known methods of chemical synthesis. Particular useful is the reaction between a dye active ester and a primary amine group of the scaffold A to connect both units *via* an amide bond. Intermediates as well as final probe molecules may be purified by high performance liquid chromatography (HPLC) and characterized by mass spectrometry and analytical HPLC before they are used in labelling and imaging experiments.

The present invention is illustrated in the following paragraph by several but nonlimiting examples:
In a preferred embodiment, the probe of the formula (I) comprises a scaffold A which is derived from a tetrapeptide caspase-1 inhibitor (A is compound 2) bearing a chromophore at the C-terminal side and at the N-terminal side. Appropriate chromophores are chosen in a way that their spectral properties are suitable for fluorescence resonance energy transfer (FRET). Chromophores can be fluorescent or non fluorescent. In principle, a broad variety of chromophores may be used in the present invention, as long as the central requirement that is a spectral change after proteolytic cleavage of a peptide bond is met. The attachment of such interacting chromophores and the central scaffold is made optionally *via* linker units.

Preferably, the fluorophore are chosen from the group of xanthene- or cyanine dyes. More preferred are cyanine dyes from the group of carbacyanines, thiacyanines, oxacyanines and azacyanines. Cyanine dyes suitable to be used in the context of the present invention are disclosed in US5268468 and US5627027. They include the dyes with the trademark (Amersham, GE Healthcare) Cy 3, Cy 3B, Cy 3.5, Cy 5, Cy 5.5, Cy 7 and Cy 7.5.

Preferably, the quencher unit is a non-fluorescent chromophore which include 2,4-dinitrophenyl, 4-(4-dimethylaminophenyl)azobenzoic acid (DABCYL), 7-methoxy-coumarin-4-yl)acetyl and non fluorescent cyanine-dyes as described in WO9964519.

In a preferred embodiment, the quencher does not show a significant emission and more preferably is a non-fluorescent chromophore. In this embodiment, the imaging reagent comprises a fluorophore and a non-fluorescent (dark) acceptor chromophore.

More preferred is a probe of the formula (I) based on a tetrapeptide scaffold (A is compound 2) bearing a QSY 21-Quencher at the N-terminal side and a CY 5.5 fluorophore at the C-terminal side (Scheme 2):

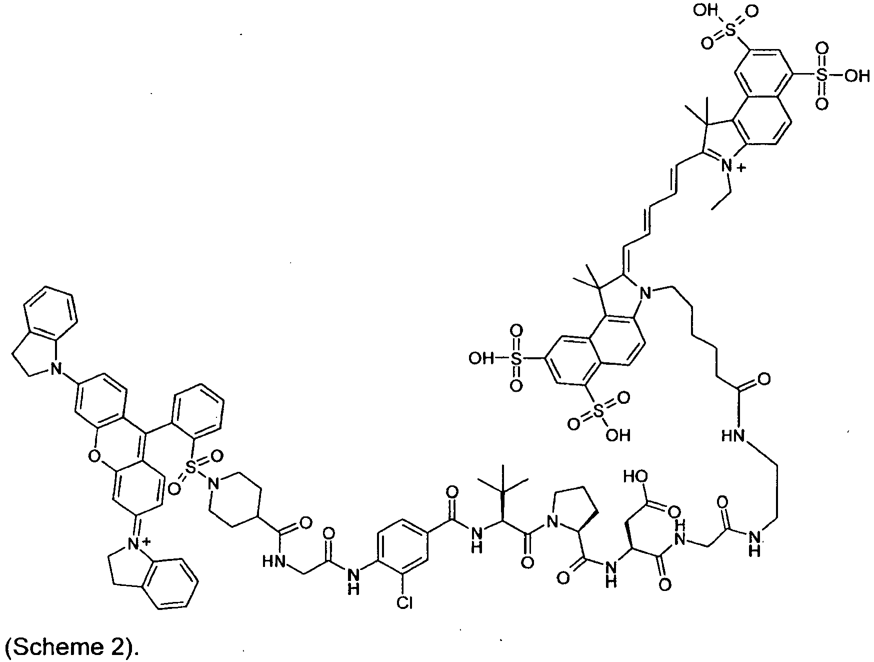

A further preferred embodiment includes the same scaffold bearing the dark quencher BHQ 3 at the N-terminal side and a Cy 7 fluorophore at the C-terminal side (Scheme 3):

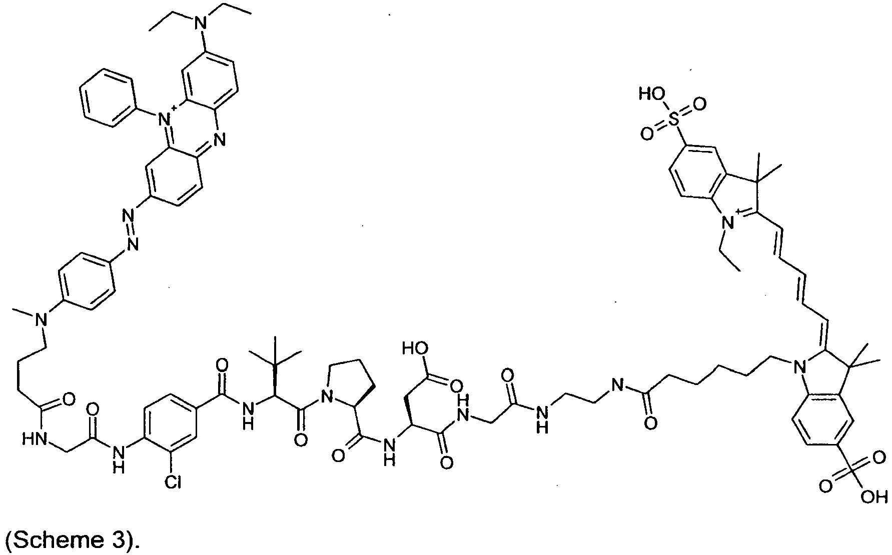

In a preferred embodiment, fluorescein and tetramethylrhodamine are chosen as an interacting FRET pair and the tetramethylrhodamine is placed at the N-terminal side of the scaffold whereas the fluorescein is linked at the C-terminal side as shown in (Scheme 4):

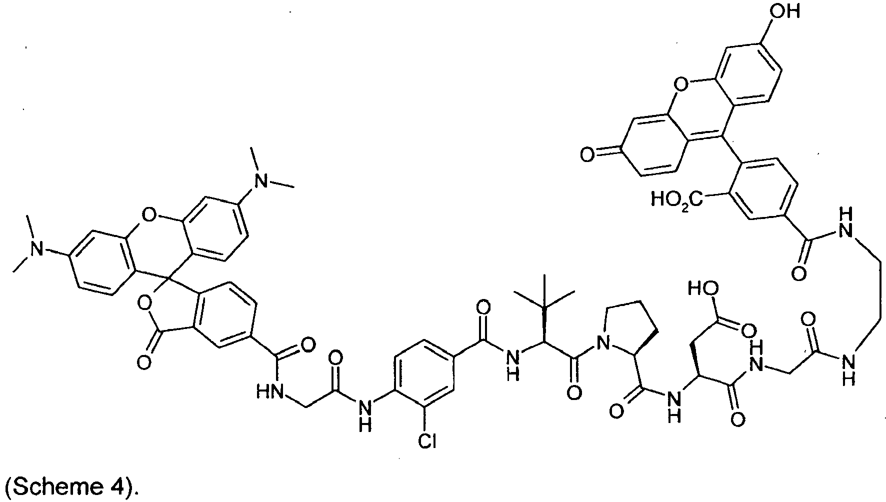

In a further preferred embodiment, one interaction partner of the FRET pair comprises a nanoparticle. More preferred in the context of the present inventions are CdSe nanoparticles (e.g. Quantum-dots), lanthanide-ion doped oxide nanoparticles (e.g. Y₀.₆Eu_{0.4}VO₄) and iron-oxide nanoparticles (e.g. AminoSPARK 680 and AminoSPARK 750 supplied by VisEn Medical, Inc., MA 01801, USA). If such nanoparticles are used as a donor in a FRET pair, they can be excited at a wavelength much shorter than the acceptor absorption thus minimizing direct acceptor excitation. In addition, the narrow donor emission does not overlap with acceptor emission. Furthermore, such nanoparticles proved to be much more photostable than organic dyes which undergo fast photobleaching. Activated quantum dots for chemical conjugation are commercially available (Invitrogen, CA 92008, USA) and their emission wavelength can be chosen from a variety of products.

Schemes 5 and 6 show quantum dot based probes of the formula (I) that are specific for caspase-1. Thus, in a further preferred probe of the formula (I) the quantum dot (e.g. QD605 supplied by Invitrogen, CA 92008, USA) might be positioned *via* an appropriate linker either at the N-terminal side of the caspase-1 probe (Scheme 5) or at the C-terminal side of the caspase-1 probe (Scheme 6).

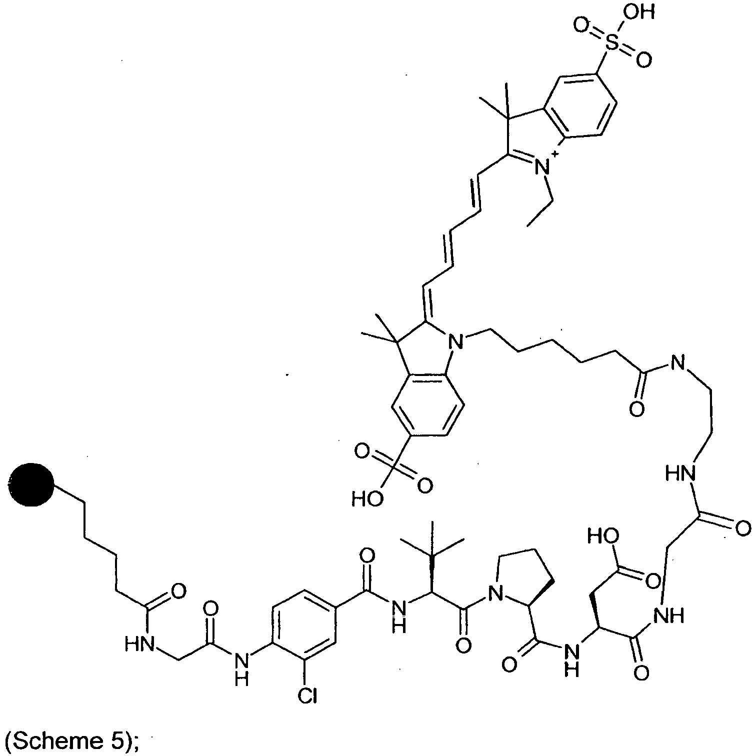

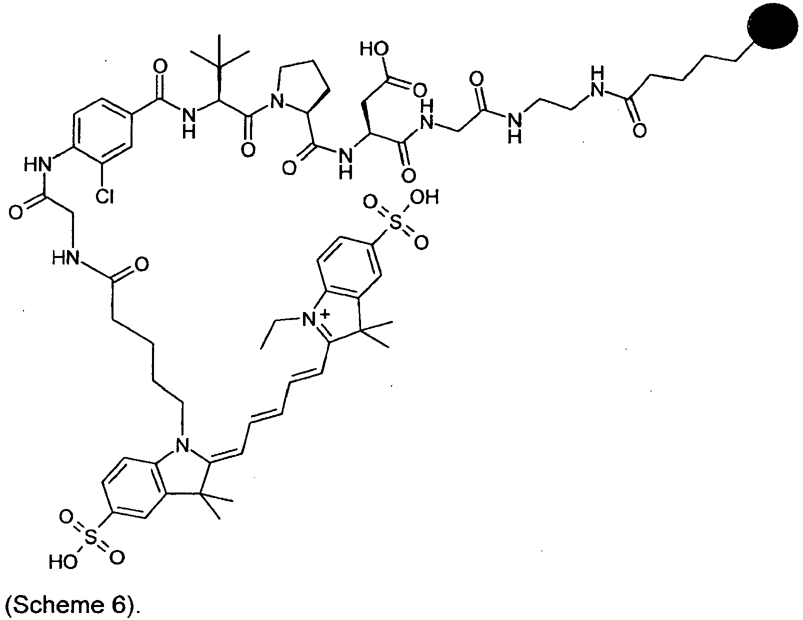

The quantum dot is represented as a black circle and an appropriate acceptor molecule is represented by the cyanine-dye CY 7.

In a further preferred embodiment, the quantum dots in the probe of the formula (I) are connected to gold-nanoparticles *via* a proteolytic cleavable subunit (Scheme 7):

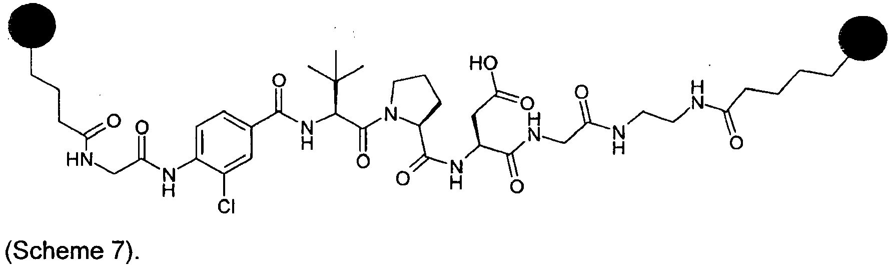

The quantum dot and the gold-nanoparticle are represented as a black circle.

Gold nanoparticles (AuNPs) have been shown as effective quenchers for organic fluorescent dyes as well as for quantum dots. The application of quantum dots in combination with AuNPs is e.g. disclosed in W02006126570.

In a further preferred embodiment, the probes of the formula (I) are designed to have a long circulation time, have high tumoral accumulation and contain quenched fluorescent markers which become fluorescent in the near-infrared spectrum after enzyme activation. These probes are based on synthetic graft copolymer [partially methoxy poly(ethylene glycol) modified poly-L-lysine] onto which multiple NIR fluorochromes were attached to free poly-lysine residues. The fluorescence of these macromolecules is highly reduced, due to internal quenching by the high density and close proximity of the NIR-chromophores.

In a further preferred embodiment, the probes of the formula (I) are designed to be used in an homogeneous enzyme linked luminescence assay. The following scheme shows the above-mentioned mechanism of action generically. The luciferine is a substrate for luciferase and a luminescent signal will be generated by a second enzymatic reaction:

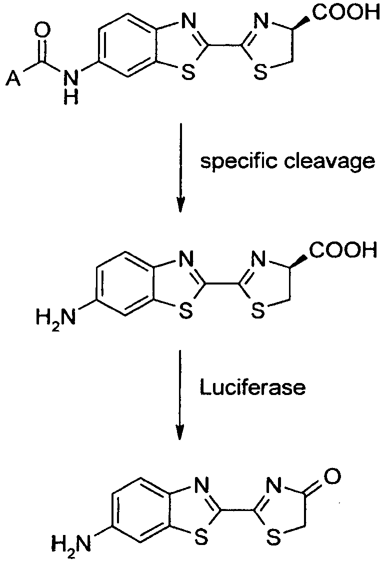

The following scheme shows the above-mentioned mechanism of action, were luciferine is masked with a pyridazinodiazepine-derivative and liberated through the proteolytic activity of said caspase-1:

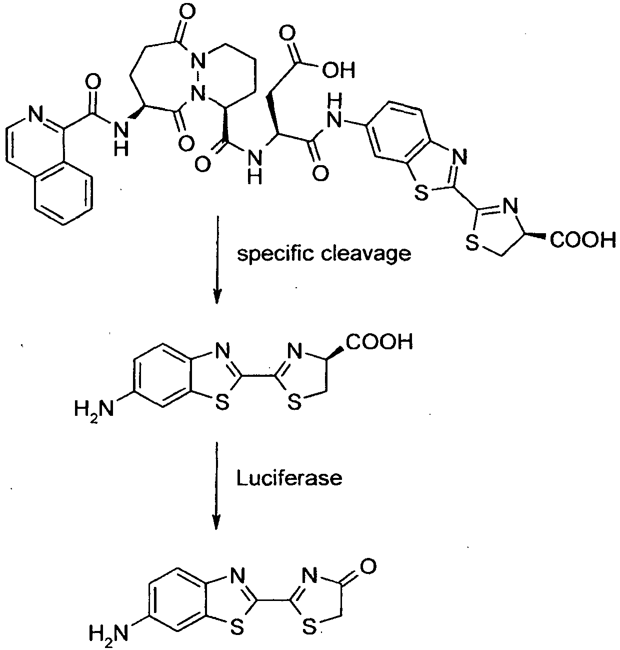

The invention further relates to a method for the design of a molecular probe for the observation of the catalytic activity of one individual proteolytic enzyme or groups of proteolytic enzymes, such as e.g. one caspase or several caspases, in *in vitro* assays, in cells or in multicellular organisms, characterized in transforming an inhibitor for an individual proteolytic enzyme or a group of proteolytic enzymes into a selective imaging probe for these individual proteolytic enzyme or group of proteolytic enzymes, preferably caspase enzymes. To achieve this we replace the electrophilic groups of certain known caspase inhibitors with a scissile amide bond. Preferred compounds are synthesized in a way that a detectable signal is generated by the enzymatic (e.g. proteolytic) activity of a specific target. Particularly, preferred probes comprise internally quenched fluorophores (e.g. appropriate FRET-pairs) linked to (i) the specificity determinant A at the N-terminal portion of the scissile bond and (ii) at the C-terminal portion of the scissile bond. The invention allows for the transfer elements of desirable and previously optimized properties of known inhibitors into novel activity based probes.

Caspase inhibitors described in the prior art utilize an electrophilic warhead in P1 position. The activity based probes of the present invention make use of said known scaffolds and introduce two modifications, firstly the conversion of the electrophilic warhead into a scissile amide group and secondly the positioning of interacting labelling pairs or property modulators on both sides of the scissile amide bound.

*In vitro,* the reaction of the protease with the substrate of the invention can generally be either performed in cell extracts or with purified or enriched forms of the protease. For *in vivo* application, the reporters are preferably emitters in the near infra red (NIR) region because that region is absent of interfering biofluorescence. Known cyanine NIR dyes matching these requirements are preferably incorporated in the substrates of the present invention.

The molecular architecture of compounds of the formula (I) consists of a central scaffold A bearing an amide functional group and two subunits L1 R1 and L2R2 respectively. L2R2 is, as shown in formula (I), always connected to scaffold A *via* an amide group since the amide group can be cleaved by the caspase enzyme. Appropriate functional groups for the attachment of subunits L1R1 to scaffold A can be chosen by those skilled in the art, and examples are given below. The specific functional groups L' in the precursor compound can be placed on the scaffold A for the attachment of suitable L1R1 subunits to yield the group L within the compound of the formula (I) are limited only by the requirement of the synthesis strategy and the final use of such substrate as an activity based imaging reagent. Thus their selection will depend upon the specific reagents chosen to build the desired substrates. Examples of functional groups L' which can be provided on scaffold A to connect A with the subunit L1R1 include fluoro, chloro, bromo, cyano, nitro, amino, azido, alkylcarbonylamino, carboxy, carbamoyl, alkoxycarbonyl, aryloxycarbonyl, carbaldehyde, hydroxy, alkoxy, aryloxy, alkylcarbonyloxy, arylcarbonyloxy, a carbon-carbon double bond, a carbon-carbon triple bond, and the like. Most preferable examples include amino, azido, hydroxy, cyano, carboxy, carbamoyl, carbaldehyde, or a carbon-carbon double or a carbon-carbon triple bond. Thus, L is a direct bond or a group selected from 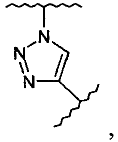 -(NRx)-, -O-, -C=N-, -C(=O)-, -C(=O)-NH-, -NH-C(=O)-, -C(=O)H, - CRx=CRy-, -C≡C- and phenyl, wherein Rx and Ry are independently H or (C₁-C₆)alkyl.

In particular, the preferred synthesis of a compound of formula (I) makes use of orthogonally protected functional groups. Such a choice of protective groups allows for a separate deprotection so that each released functionality in turn can be further chemically manipulated towards the attachment of the corresponding subunits to scaffold A. Appropriate protecting groups for the envisioned functionalities can be chosen by those skilled in the art, and are e.g. summarized in T.W. Greene and P.G.M. Wuts in "Protective Groups in Organic Synthesis", John Wiley & Sons, New York 1991.

Compounds of the formula L'-A-CO-OH (scaffolds) can be prepared by standard methods known in the art, e.g. as described in international patent applications US5670494; W09526958; W09722619; WO9816504; WO0032620; W00055127; W00105772; W00190063; W003024955; W003106460; W003104231; W003103677; W. G. Harter, Bioorg. Med. Chem. Lett. 2004, 14, 809-812; Shahripour et al., Bioorg. Med. Chem. Lett. 2001, 11, 2779-2782; Shahripour et al., Bioorg. Med. Chem. 2002, 10, 31-40; M. C. Laufersweiler et al., Bioorg. Med. Chem. Lett. 2005, 15, 4322-4326; K. T. Chapman, Bioorg. Med. Chem. Lett. 1992, 2, 613-618; Dolle et al., J. Med. Chem. 1997, 40, 1941-1946; D. L. Soper et al., Bioorg. Med. Chem. Lett. 2006, 16, 4233-4236; D. L. Soper et al., Bioorg. Med. Chem. 2006, 14, 7880-7892; D. J. Lauffer et al., Bioorg. Med. Chem. Lett. 2002, 12,1225-1227; C. D. Ellis et al., Bioorg. Med. Chem. Lett. 2006, 16, 4728-4732; P. Tawa et al., Cell Death and Differentiation 2004, 11, 439-447; Micale et al., J. Med. Chem. 2004, 47, 6455-6458; Berger et al., Molecular Cell, 2006, 23, 509-521; and Garcia-Calvo, J. Biol. Chem. 1998, 273 (49), 32608-32613.

The present invention also relates to a method for the preparation of a compound of the formula (I) characterized in, if n is 1:
(a) a compound of the formula (II)

   L'-A-CO-OH (II)

   wherein A is as defined above in its generic and preferred meanings and L' is fluoro, chloro, bromo, cyano, nitro, amino, azido, alkylcarbonylamino, carboxy, carbamoyl, alkoxycarbonyl, aryloxycarbonyl, carbaldehyde, hydroxy, alkoxy, aryloxy, alkylcarbonyloxy, arylcarbonyloxy, a carbon-carbon double bond, a carbon-carbon triple bond, preferably amino, azido, hydroxy, cyano, carboxy, carbamoyl, carbaldehyde, or a carbon-carbon double or a carbon-carbon triple bond, more preferred amino,
   is reacted under conditions known to a skilled person with a compound of the formula L1-R1-H wherein L1 is as defined above in its generic and preferred meanings to a compound of the formula (III)

   L1-R1-L-A-CO-OH (III)
(b) the compound (III) is reacted with a compound H₂N-R2-L2 to a compound of the formula (I).

Optionally, the synthesis of the compound of the formula (I) makes use of orthogonally protected functional groups. Such a choice of protective groups allows for a separate deprotection so that each released functionality in turn can be further manipulated chemically either to attach a label to it or for the introduction of further extension of the linker R1 and/or R2. Appropriate protecting groups for the envisioned functionalities can be chosen by those skilled in the art, and are e.g. summarized in T.W. Greene and P.G.M. Wuts in "Protective Groups in Organic Synthesis", John Wiley & Sons, New York 1991.

A further method for the preparation of the probe of the formula (I) wherein n is 1 comprises
(a1) the reaction of a compound of the formula (II) with a compound of the formula (IV)

   H₂N-L2-PG2 (IV)

   to a compound of the formula (V)

   L'-A-CO-NH-R2-PG2 (V)

   under conditions known to the skilled person,
(b) subsequently reacting the compound (V) with a compound (VI)

   PG1 -R1-L" (VI)

   to a compound

   PG1-R1-L-A-CO-NH-R2-PG2 (VI)

   under conditions known to the skilled person for the respective groups,
   wherein PG1 and PG2 are independent of each other protecting groups, preferably orthogonally protecting groups,
   L" is the respective connecting group for L' to be selected by the person skilled in the art, or bond,
(c1) the group PG2 of the compound (VI) is cleaved and the resulting compound is reacted with a label L2, and subsequently the protecting group PG1 is cleaved and the resulting compound is reacted with a label L1 to a compound of the formula (I), or
(c2) the group PG1 of the compound (VI) is cleaved and the resulting compound is reacted with a label L1, and subsequently the protecting group PG2 is cleaved and the resulting compound is reacted with a label L2 to a compound of the formula (I).

In step (b), preferred combinations of L' and L" and reaction types (in brackets) are as follows:
When L' is fluoro, chloro, bromo, iodo, L" is amino (R-NH₂), hydroxy (R-OH), triple-bond (Sonogashira Reaction), a double bond (Heck reaction), an alkyl borane (Suzuki-reaction);
when L' is cyano, L" is amino (R-NH₂), hydroxy (R-OH), thiol (R-SH);
when L' is amino, L" is an activated carboxylic acid (NHS-ester,...), an carbaldehyde, fluoro, chloro, bromo, iodo;
when L' is azido, L" is a triple bond, a phosphine moiety (Staudinger ligation);
when L' is carboxy, L" is amino, hydroxyl, hydrazide;
when L' is alkoxycarbonyl, L" is amino, hydroxyl, hydrazide;
when L' is aryloxycarbonyl, L" is amino, hydroxyl, hydrazide;
when L' is hydroxy, L" is fluoro, chloro, bromo, iodo, hydroxy (Mitsunobu-reaction), carboxy;
when L' is carbaldehyde, L" is amino, hydrazine;
when L' is carbon-carbon double bond, bromo, chloro, iodo (Heck reaction), an alkyl borane (Suzuki-reaction);
when L' is a carbon-carbon triple bond,, L" is bromo, chloro, iodo (Sonogashira Reaction), azido.

Compounds of the formula (I) wherein n is 0 can be prepared by reacting a compound of the formula A-CO-OH (IV) with a compound H₂N-R2-L2 to the probe of the formula (I).

Preferably cysteine protease substrates functionalized with different label are synthesized on the solid support.

For the synthesis of caspase probes of the formula (I) with a peptidomimetic structure non-peptidic building blocks may be utilized for the solid-phase synthesis. Building block syntheses are further described in Examples 8.

Building block (VII) is preferably used for the synthesis of caspase-1 probes, e.g. the compounds of Examples 1 and 2,

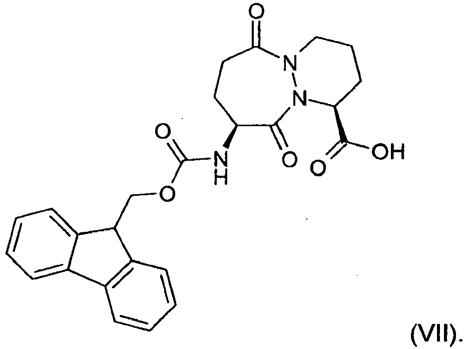

The probes of the present invention are used for molecular imaging in vitro, in cell-culture experiments, or ex-vivo experiments., including screening and whole animal imaging. Mostly preferred are imaging modalities such as optical imaging and magnetic resonance imaging (MRI).

The probes of the present invention are intended to be used for diagnostic imaging of protease activity. Most preferred are applications which provide methods of monitoring the effect of a drug or drug-like substance towards the targeted proteases. Administration of such a drug or drug like substance should have a measurable effect to the signal from the probe of the present invention.

A further aspect of the present invention is a probe of formula (I) for use in a method for molecular imaging in a living organism.

A "living organism" may be any live cell or whole organism comprising the cysteine protease to-be-detected, preferably the living organism is a mammal, e.g. a mouse or a rat.

The probes of the present invention are highly selective, whereby a risk of false positives can be avoided.

### Abbreviations:

- DMF: = dimethylformamide
- DMSO: = dimethylsulfoxide
- DCM: = dichloromethane
- equiv.: = equivalents
- sat.: = saturated
- THF: = tetrahydrofuran
- DIPEA: = diisopropylethyl amine
- HOAt: = 1-Hydroxy-7-azabenzotriazole
- HATU: = *O*-(7-Azabenzotriazol-1-yl)-*N,N,N;N*'-tetramethyluronium hexafluorophosphate,
- NHS: = N-hydroxysuccinimidyl ester

### General Procedure for Solid Phase Peptide Synthesis:

The following probes were synthesized using standard solid phase peptide synthesis. The 2-chlorotrityl-resin was used as solid support. For the loading of the resin, 2 equiv. of Fmoc-protected amino acid and 3 equiv. of DIPEA were solved in DCM and the reaction mixture was added to the resin (loading: 1.4 mmol/g). The reaction mixture was shaken at room temperature over night. The resin was washed with DCM and DMF. For Fmoc-deprotection the resin was treated two times for 15 minutes with 30% piperidine/DMF solution. For solid phase peptide synthesis a standard protocol was used: 4 equiv. of Fmoc-protected amino acid, 4 equiv. of HATU, 4 equiv. of HOAt and 8 equiv. of DIPEA were solved in a mixture of DCM/DMF (1/1). The reaction mixture was stirred at room temperature for 20 minutes and then added to the resin. The reaction mixture was shaken for 2 hours or longer if the Fmoc-protected amino acid were sterically hindered. For cleavage from the solid phase, the resin was treated with 5% TFA in DCM two times for 15 minutes. The solvent was coevaporated with toluene under reduced pressure and the final product was purified by preparative HPLC (Gradient: H₂O+0.05% TFA; 5 to 95% CH₃CN).

### Example 1: Caspase-1 probe

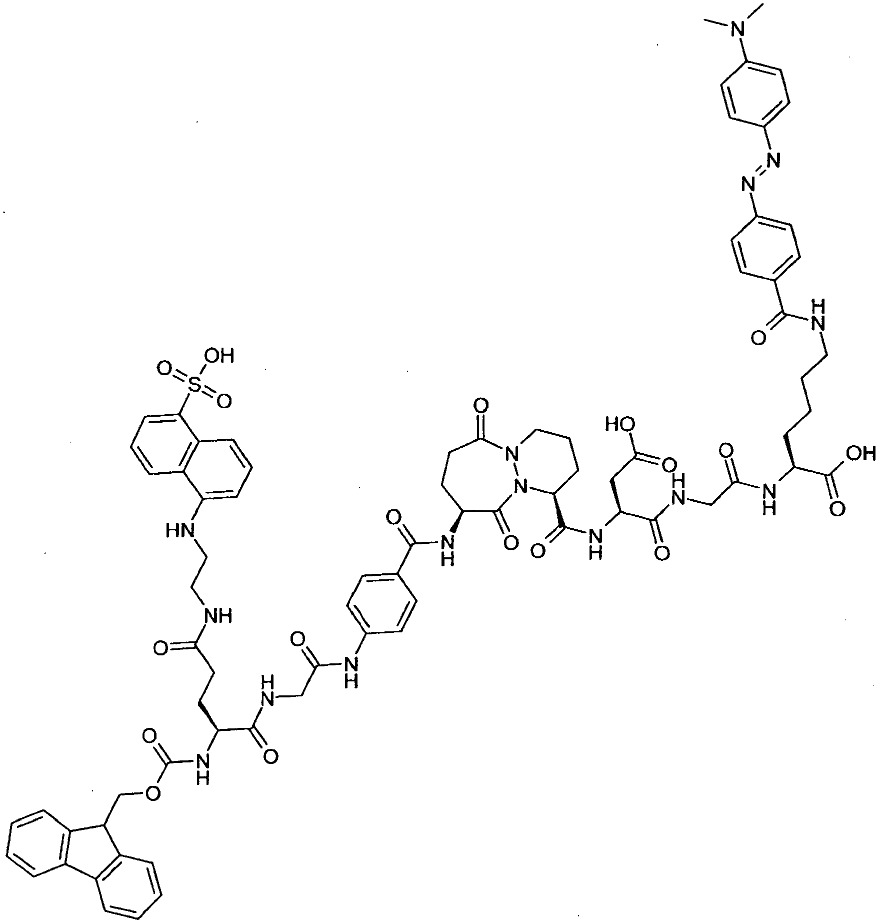

The compound was prepared on solid-support according to the general procedure and purified by HPLC (H₂O+0.05% TFA; 4-95% CH₃CN). Calculated: [M+H]⁺ = 1569,70, found: [M+H]⁺ = 1569,45. Yield: 54%.

### Example 2: Caspase-1 probe

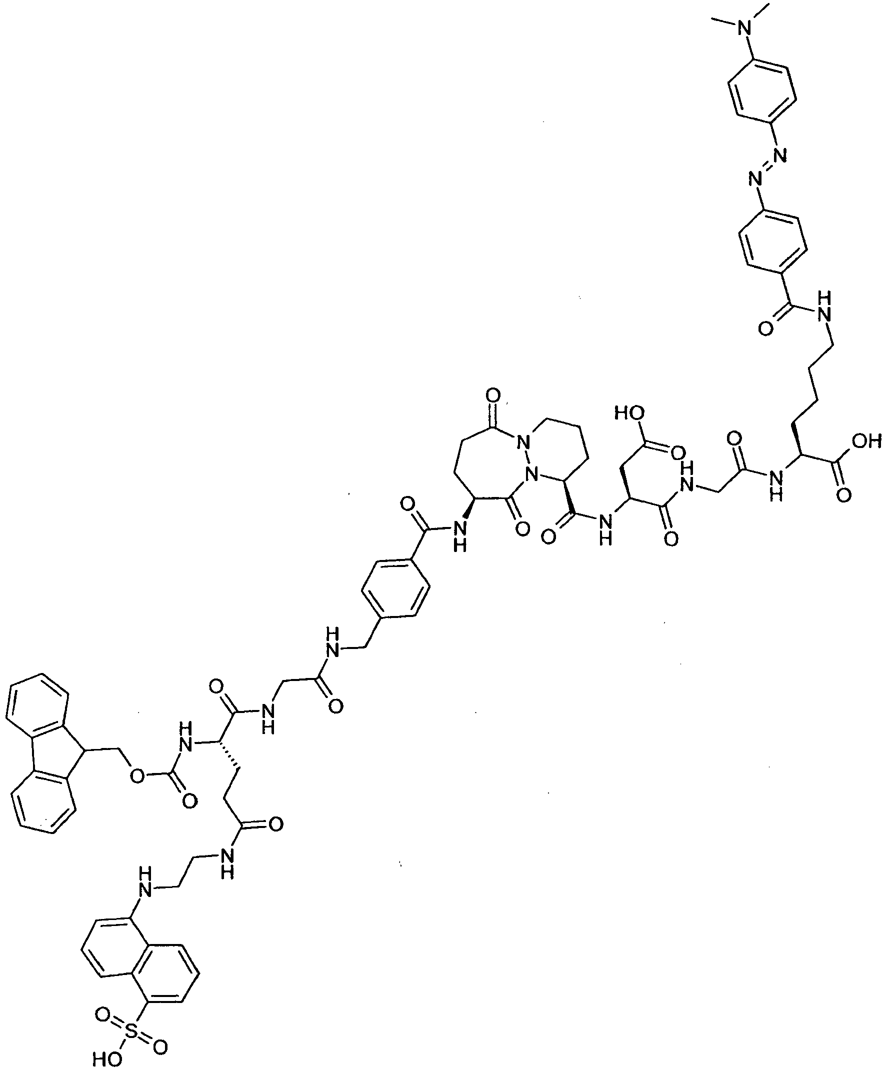

The compound was prepared on solid-support according to the general procedure and purified by HPLC (H₂O+0.05% TFA; 4-95% CH₃CN). Calculated: [M+H]⁺= 1583,73 found: [M+H]⁺= 1583,2. Yield: 72%.

### Example 3: Caspase-1 probe

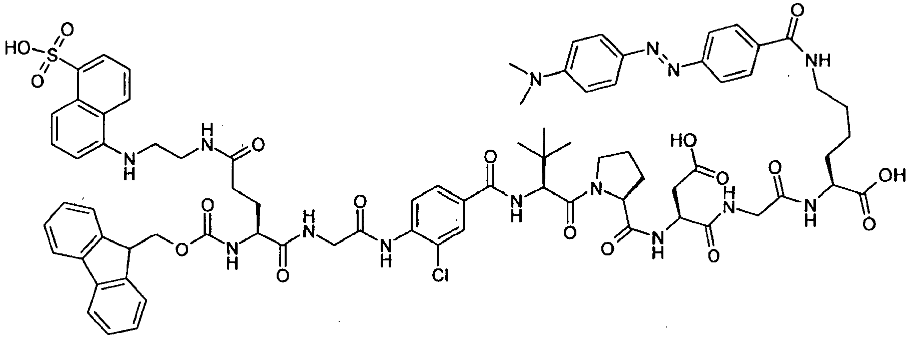

The compound was prepared on solid-support according to the general procedure and purified by HPLC (H₂O+0.05% TFA; 4-95% CH₃CN). Calculated: [M+H]⁺ = 1591,19 found: [M+H]⁺= 1591,50. Yield: 66%.

### Example 4: Caspase-3 probe

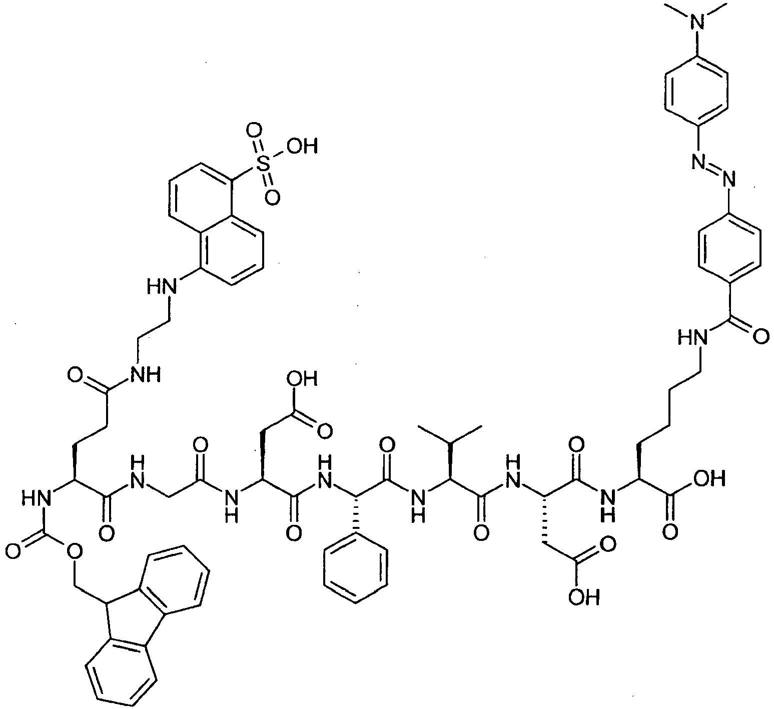

The compound was prepared on solid-support according to the general procedure and purified by HPLC (H₂O+0.05% TFA; 4-95% CH₃CN). Calculated: [M+H]⁺ = 1517,66 found: [M+H]⁺= 1517,55. Yield: 59%.

### Example 5: Caspase-3 probe

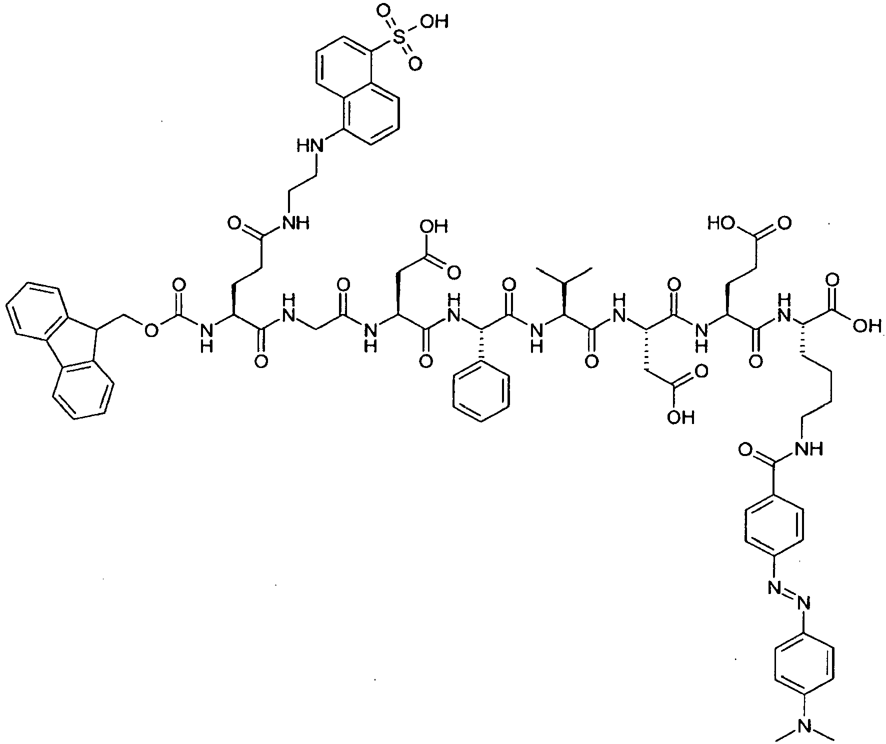

The compound was prepared on solid-support according to the general procedure and purified by HPLC (H₂O+0.05% TFA; 4-95% CH₃CN). Calculated: [M+H]⁺= 1546,79 found: [M+H]⁺= 1546,35. Yield: 61%.

### Example 6: Caspase-8 probe

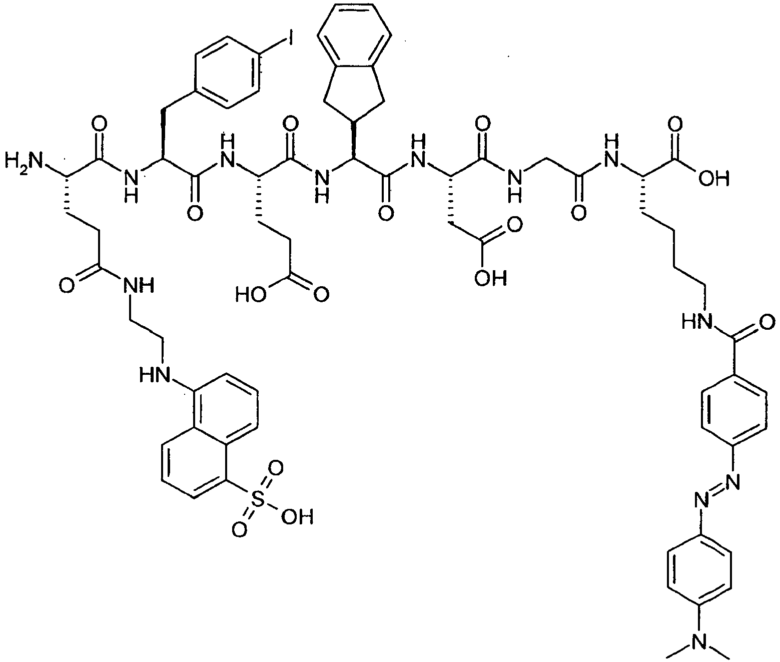

The compound was prepared on solid-support according to the general procedure and purified by HPLC (H₂O+0.05% TFA; 4-95% CH₃CN). Calculated: [M+H]⁺ = 1523,45 found: [M+H]⁺ = 152325. Yield: 55%.

### Example 7

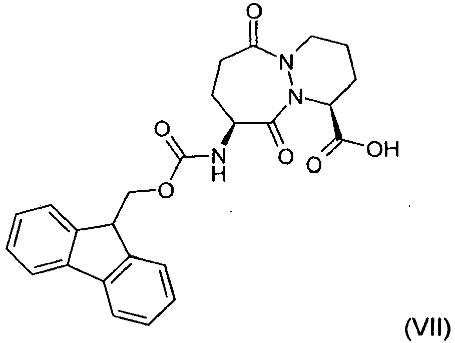

Building block (VII) has been prepared according to the procedure described in WO9722619.

### Example 8: Caspase-1 bioluminescent probe

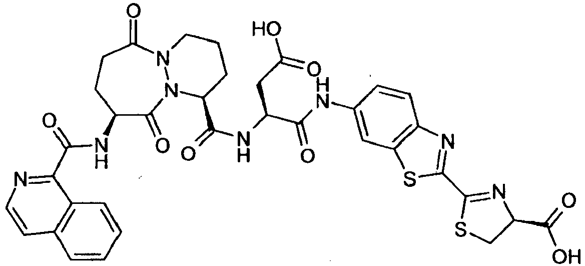

The compound was prepared on solid-support, starting from 6-Fmoc-Amino-D-Luciferin, according to the general procedure and purified by HPLC (H₂O+0.05% TFA; 4-95% CH₃CN). Calculated: [M+H]⁺ = 772.82, found: [M+H]⁺ =773.15. Yield: 13%.

## Claims

1. Molecular probe for cysteine proteases of the formula (I)
{L1-R1-L}ₙ-A-CO-NH-R2-L2 (I)
wherein
A is a group recognizable by a caspase;
wherein A is recognizable by caspase-1 and is selected from a group 1. - 28. of the table below:
| | |
|---|---|
| 1. | 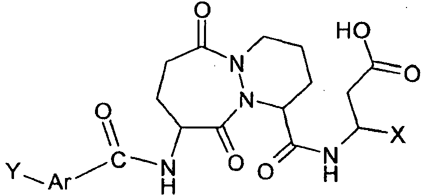 |
| | wherein Ar is phenyl if n is 1 and Ar is isoquinoline if n is 0, preferably in the configuration: |
| | 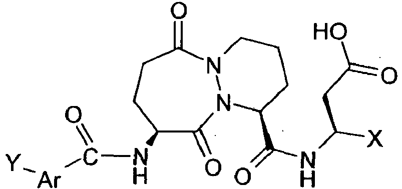 |
| 2. | 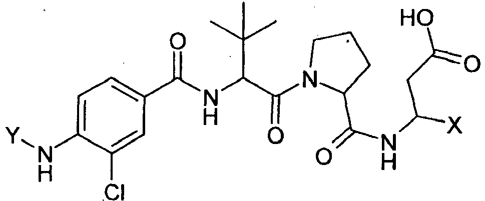 |
| | preferably in the configuration: |
| | 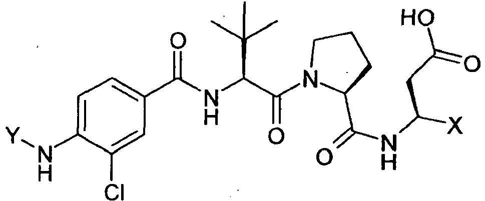 |
| 3. | 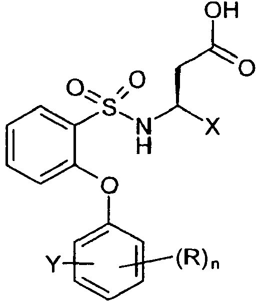 |
| | wherein R is (C₁-C₅)alkyl, phenyl, (C₅-C₆)cycloalkyl; and n is 1-3; |
| 4. | 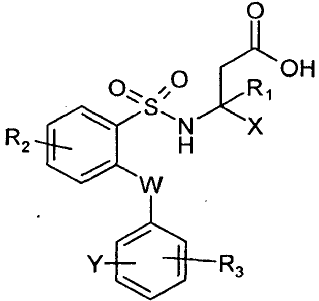 |
| | preferably in the configuration: |
| | 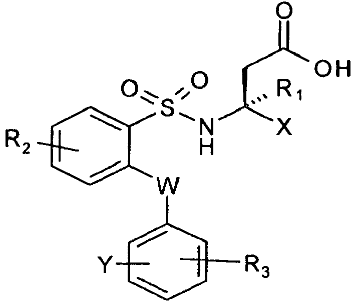 |
| | wherein R₁ is hydrogen, (C₁-C₆)alkyl, aryl or -CH₂-aryl, |
| | R₂ and R₃ are independently of each other hydrogen, or an aryl, substituted aryl, heteroaryl, substituted heteroaryl, cycloalkyl, substituted cycloalkyl, heterocycle, or substituted heterocycle group that is fused to the phenyl group that contains a group R₂ as a substituent; |
| | W is a bond, NR₅, CO, S, O, SO₂, O(CHR₅)ₙ-, CHR₅, NR₅CO,CONR₅, OCHR₅, CHR₅O, SCHR₅, CHR₅S, SO₂NR₅, (C₁-C₆)alkyl, NR₅SO₂, CH₂CHR₅, CHR₅CH₂, COCH₂ or CH₂CO; |
| | wherein R₅ is independently hydrogen, (C₁-C₆)alkyl, aryl, (CH₂)ₙ-aryl, or (CH₂)ₙ-cycloalkyl; and each n is independently 0 to 5; |
| 6. | 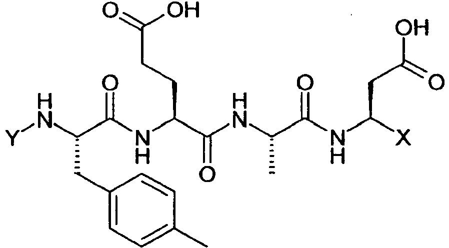 |
| 7. | 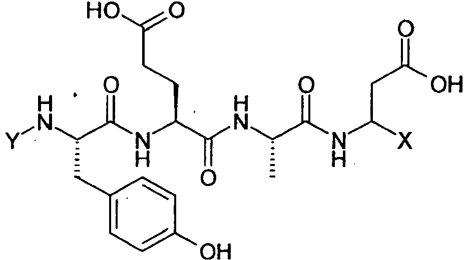 |
| 8. | 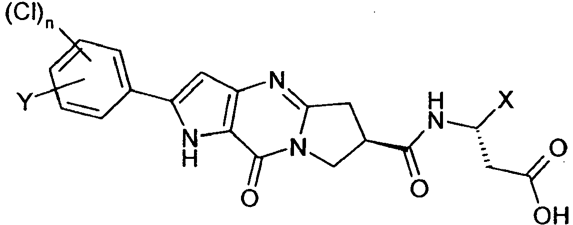 |
| | wherein n is 0 or 1; |
| 9. | 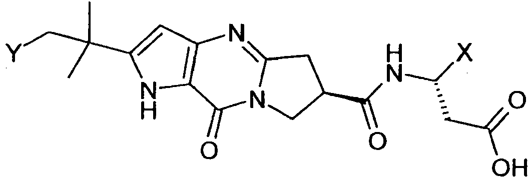 |
| 10. | 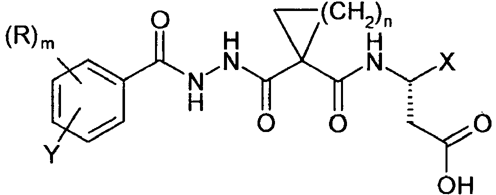 |
| | wherein n is 1-4 |
| | m is 1 or 2, and |
| | R is methyl or methoxy; |
| 11. | 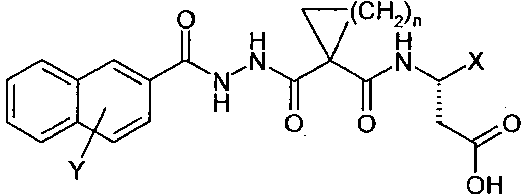 |
| | wherein n is 1-4; |
| 12. | 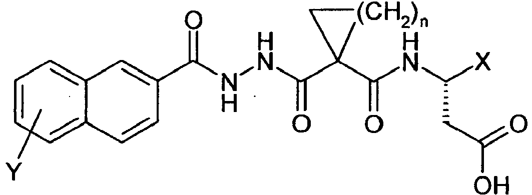 |
| | wherein n is 1-4; |
| 13. | 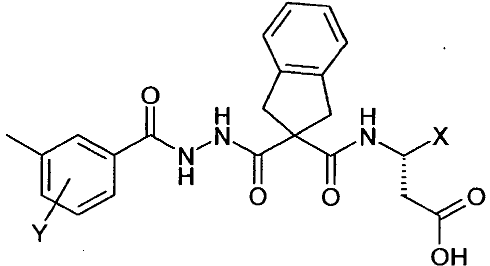 |
| 14. | 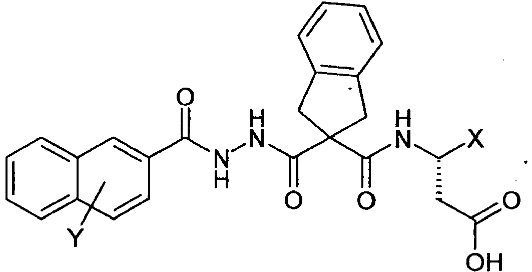 |
| 15. | 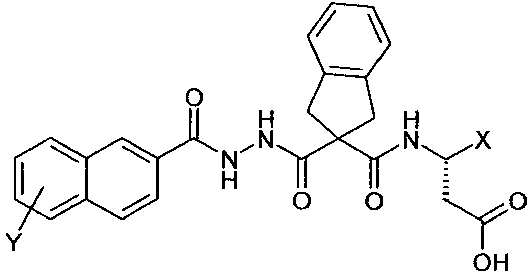 |
| 16. | 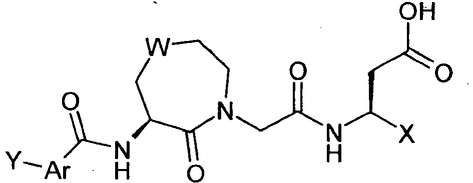 |
| | wherein W is S or S(O)₂, and |
| | Ar is aryl or heteroaryl, preferably phenyl, naphthyl, benzothiophene or isoquinolyl; |
| 17. | 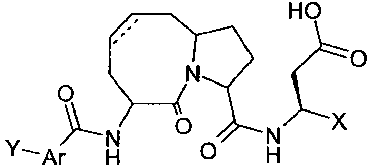 |
| | wherein Ar is an aryl or heteroaryl group selected from phenyl, benzothiophene, isoquinolyl, cinnamyl, naphthyl, which is optionally once or independently twice substituted by methoxy, chloro, methyl, CF₃, and wherein |
| | 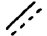 |
| | means either a single or a double bond; |
| 18. | 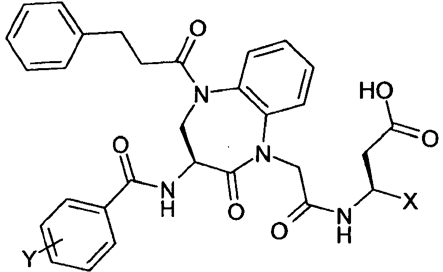 |
| 19. | 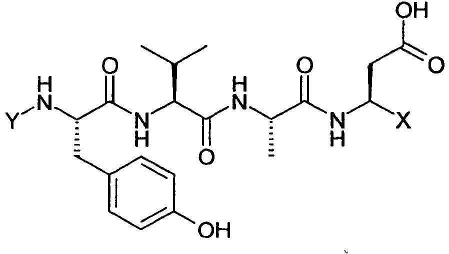 |
| 20. | 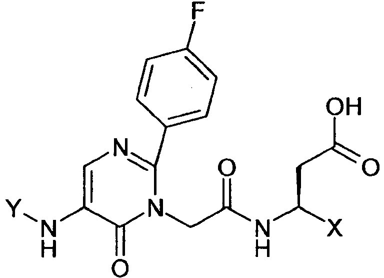 |
| 21. | 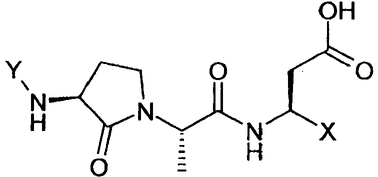 |
| 22. | 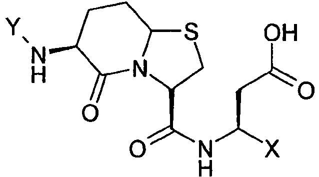 |
| 23. | 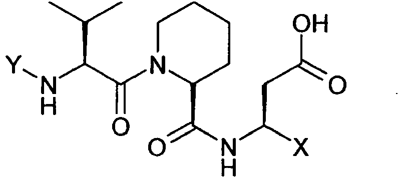 |
| 24. | 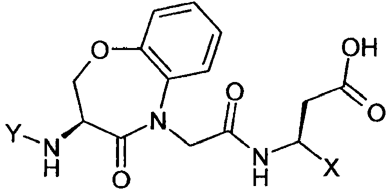 |
| 25. | 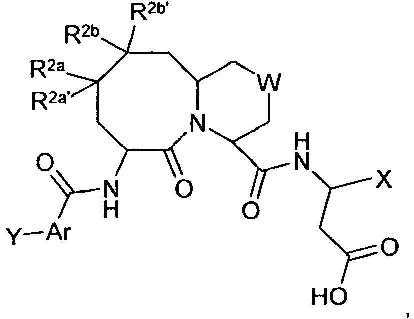 |
| | preferably in the all-(S) configuration, |
| | wherein Ar is aryl or heteroaryl; |
| | W is CH₂, O or NR9 wherein R9 is hydrogen or (C₁-C₆)alkyl, aryl, heteroaryl, heterocyclyl; |
| | R^{2a}, R^{2a'}, R^{2b} and R^{2b'} are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond; |
| 26. | 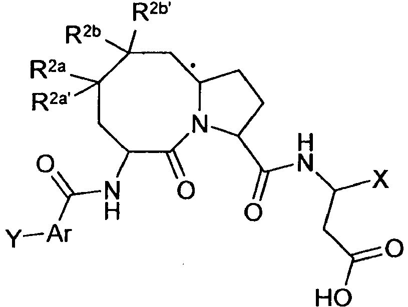 |
| | preferably in the all-(S) configuration, |
| | wherein Ar is aryl or heteroaryl; |
| | R^{2a}, R^{2a'}, R^{2b} and R^{2b'} are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond; |
| 27. | 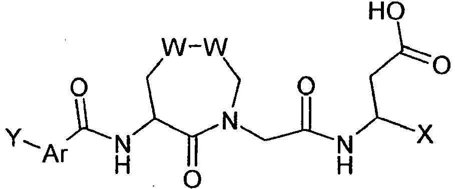 |
| | preferred in the all-(S) configuration |
| | wherein Ar is aryl or heteroaryl; and |
| | W is independently selected from: C(R¹)₂; C(O); NR²; S; S(O); S(O)₂; wherein R¹ and R² are independently hydrogen, [C(R³)₂]ₚ(CH=CH)_{q}R³, C(=Z)R³, C(=Z)[C(R³)₂]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂, SO₂R³, P(O)(OR³)R³, P(O)(OR³)₂; wherein p is 0 to 12; wherein q is 0 to 12; wherein Z is O, S, NR³; wherein R³ is independently hydrogen, alkyl, cycloalkyl, aryl, heteroaryl, heterocylyl. |
| 28. | 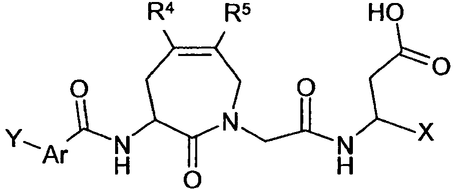 |
| | preferred in the all-(S) configuration |
| | wherein Ar is aryl or heteroaryl; and R⁴ and R⁵ are independently selected from: C(R¹)₂; C(O); NR²; S; S(O); S(O)₂; wherein R¹ and R² are independently hydrogen, [C(R³)₂]ₚ(CH=CH)_{q}R³, C(=Z)R³, C(=Z)[C(R³)₂]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂, SO₂R³, P(O)(OR³)R³, P(O)(OR³)₂; wherein p is 0 to 12; wherein q is 0 to 12; wherein Z is O, S, NR³; wherein R³ is independently hydrogen, alkyl, cycloalkyl, aryl, heteroaryl, heterocylyl; |
or A is recognisable by caspase-3 and is selected from a group 29. - 42. of the table below :
| | |
|---|---|
| 29. | 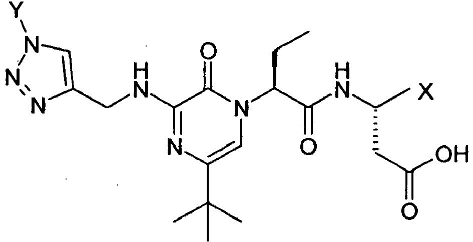 |
| 30. | 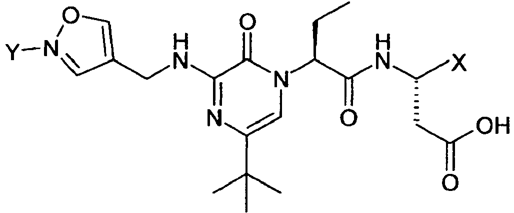 |
| 31. | 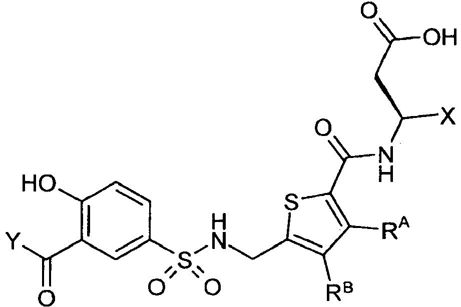 |
| | wherein R^{A} and R^{B} are independently hydrogen, (C₁-C₆)alkyl, hydroxyl, (C₁-C₆)alkoxy or halogen. |
| 32. | 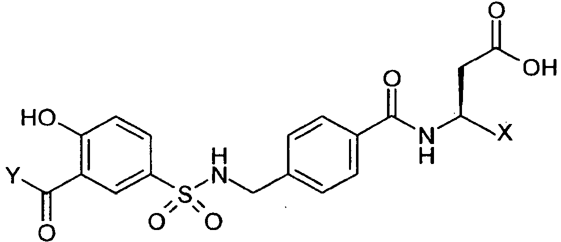 |
| 33. | 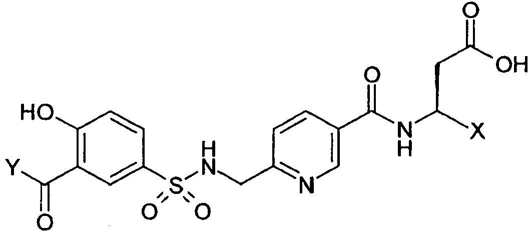 |
| 34. | 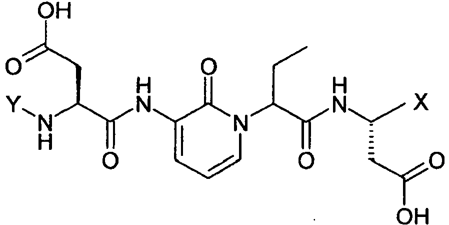 |
| 35. | 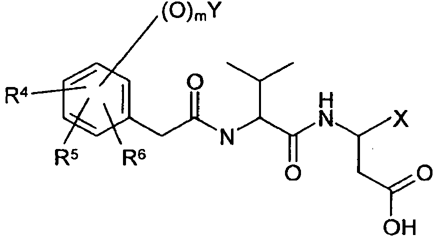 |
| | preferably in the all-(S) configuration, |
| | wherein m is 0 or 1; and |
| | R⁴, R⁵ and R⁶ are independently selected from the group consisting of: |
| | 1) H, |
| | 2) halogen, |
| | 3) (C₁-C₄)alkoxy optionally substituted with 1-3 halogen atoms, |
| | 4) N02, |
| | 5) OH, |
| | 6) benzyloxy, the benzyl portion of which is optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 7) NH(C₁-C₄)acyl, |
| | 8) (C₁-C₄)acyl, |
| | 9) O-(C₁-C₄)alkyl-CO₂H, optionally esterified with a (C₁-C₆)alkyl or a (C₅-C₇)cycloalkyl group, |
| | 10) CH=CH-CO₂H, |
| | 11) CO₂H, |
| | 12) (C₁-C₅)alkyl-CO₂H, |
| | 13) C(O)NH₂, optionally substituted on the nitrogen atom by 1-2 (C₁-C₄)alkyl groups; |
| | 14) (C₁-C₅)alkyl-C(O)NH₂, optionally substituted on the nitrogen atom by 1-2 (C₁-C₄)alkyl groups; |
| | 15) S(O)₀₋₂-(C₁-C₄)alkyl; |
| | 16) (C₁-C₂)alkyl-S(O)₀₋₂-(C₁-C₄)alkyl; |
| | 17) S(O)₀₋₂-(C₁-C₆)alkyl or S(O)₀₋₂-phenyl, said alkyl and phenyl portions thereof being optionally substituted with 1-3 members selected from the group consisting of: halogen, CN, (C₁-C₄)alkyl and (C₁-C₄)alkoxy, said alkyl and alkoxy being optionally substituted by 1-3 halogen groups, |
| | 18) benzoyl optionally substituted by 1-2 members selected from the group consisting of: halogen, CN, (C₁-C₄)alkyl and (C₁-C₄)alkoxy, said alkyl and alkoxy groups being optionally substituted by 1-3 halogen groups, |
| | 19) phenyl or naphthyl, optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 20) CN, |
| | 21) (C₁-C₄)alkylene-HET2, wherein HET2 represents a 5-7 membered aromatic or non-aromatic ring containing 1-4 heteroatoms selected from O, S, NH and N(C₁-C₄) and optionally containing 1-2 oxo groups, and optionally substituted with 1-3 (C₁-C₄)alkyl, OH, halogen or (C₁-C₄)acyl groups; |
| | 22) O-(C₁-C₄)alkyl-HET3, wherein HET3 is a 5 or 6 membered aromatic or non-aromatic ring containing from 1 to 3 heteroatoms selected from O, S and N, and optionally substituted with one or two groups selected from halogen and (C₁-C₄)alkyl, and optionally containing 1-2 oxo groups, and |
| | 23) HET4, wherein HET4 is a 5 or 6 membered aromatic or non-aromatic ring, and the benzofused analogs thereof, containing from 1 to 4 heteroatoms selected from O, S and N, and is optionally substituted by one or two groups selected from halogen, (C₁-C₄)alkyl and (C₁-C₄)acyl; and wherein halogen includes F, Cl, Br and I. |
| 36. | 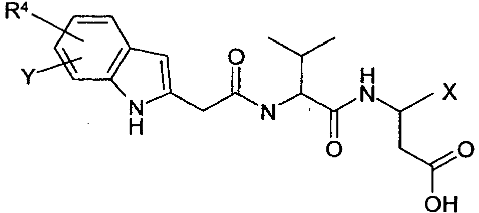 |
| | preferably in the all-(S) configuration, |
| | wherein R⁴ is selected from the group consisting of: |
| | 1) H, |
| | 2) halogen, |
| | 3) (C₁-C₄)alkoxy optionally substituted with 1-3 halogen atoms, |
| | 4) NO₂, |
| | 5) OH, |
| | 6) benzyloxy, the benzyl portion of which is optionally substituted with 1-2 members selected from the group consisting of: halogen, CN, (C₁-C₄)alkyl and (C₁-C₄)alkoxy, said alkyl and alkoxy being optionally substituted with 1-3 halogen groups, |
| | 7) NH(C₁-C₄)acyl, |
| | 8) (C₁-C₄)acyl, |
| | 9) O-(C₁-C₄)alkyl-CO₂H, optionally esterified with a (C₁-C₆)alkyl or a (C₅-C₇)cycloalkyl group, |
| | 10) CH=CH-CO₂H, |
| | 11) CO₂H, |
| | 12) (C₁-C₅)alkyl-CO₂H, |
| | 13) C(O)NH₂, optionally substituted on the nitrogen atom by 1-2 (C₁-C₄)alkyl groups; |
| | 14) (C₁-C₅)alkyl-C(O)NH₂, optionally substituted on the nitrogen atom by 1-2 (C₁-C₄)alkyl groups; |
| | 15) S(O)₀₋₂-(C₁-C₄)alkyl; |
| | 16) (C₁-C₂)alkyl-S(O)₀₋₂-(C₁-C₄)alkyl; |
| | 17) S(O)₀₋₂-(C₁-C₆)alkyl or S(O)₀₋₂-phenyl, said alkyl and phenyl portions thereof being optionally substituted with 1-3 members selected from the group consisting of: halogen, CN, (C₁-C₄)alkyl and (C₁-C₄)alkoxy, said alkyl and alkoxy being optionally substituted by 1-3 halogen groups, |
| | 18) benzoyl optionally substituted by 1-2 members selected from the group consisting of: halogen, CN, (C₁-C₄)alkyl and (C₁-C₄)alkoxy, said alkyl and alkoxy groups being optionally substituted by 1-3 halogen groups, |
| | 19) phenyl or naphthyl, optionally substituted with 1-2 members selected from the group consisting of: halogen, CN, (C₁-c₄)alkyl and (C₁-C₄)alkoxy, said alkyl and alkoxy being optionally substituted with 1-3 halogen groups, |
| | 20) CN, |
| | 21) (C₁-C₄)alkylene-HET2, wherein HET2 represents a 5-7 membered aromatic or non-aromatic ring containing 1-4 heteroatoms selected from O, S, NH and N(C₁-C₄) and optionally containing 1-2 oxo groups, and optionally substituted with 1-3 (C₁-C₄)alkyl, OH, halogen or (C₁-C₄)acyl groups; |
| | 22) O-(C₁-C₄)alkyl-HET3, wherein HET3 is a 5 or 6 membered aromatic or non-aromatic ring containing from 1 to 3 heteroatoms selected from O, S and N, and optionally substituted with one or two groups selected from halogen and (C₁-C₄)alkyl, and optionally containing 1-2 oxo groups, and |
| | 23) HET4, wherein HET4 is a 5 or 6 membered aromatic or non-aromatic ring, and the benzofused analogs thereof, containing from 1 to 4 heteroatoms selected from O, S and N, and is optionally substituted by one or two groups selected from halogen, (C₁-C₄)alkyl and (C₁-C₄)acyl; and wherein halogen includes F, Cl, Br and I. |
| 37. | 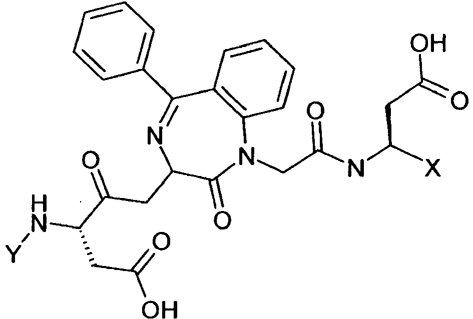 |
| 38. | 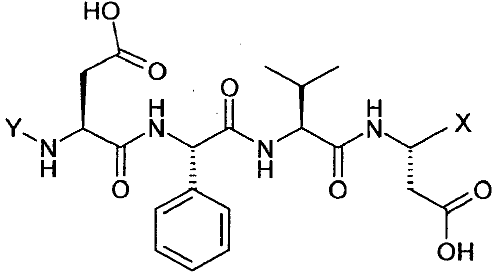 |
| 39. | 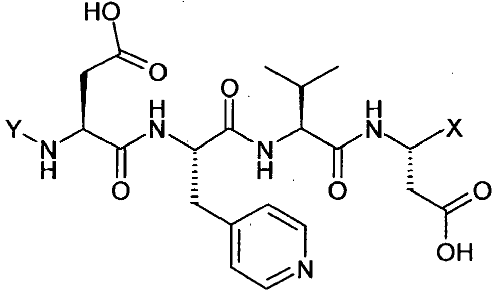 |
| 40. | 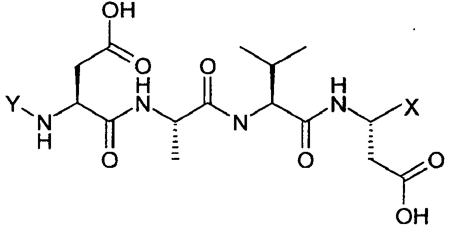 |
| 41. | 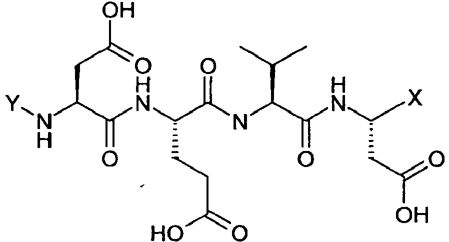 |
| 42. | 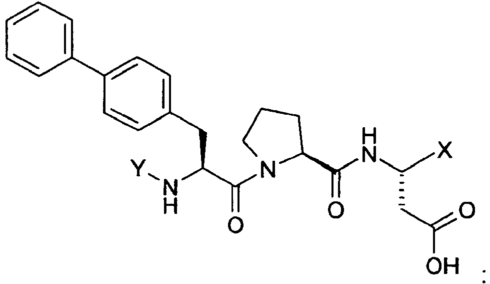 |
or A is recognizable by caspase-8 and is selected from a group 43. - 44. of the table below:
| | |
|---|---|
| 43. | 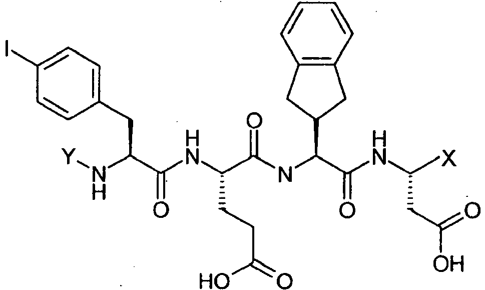 |
| 44. | 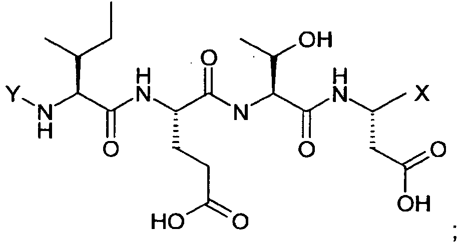 |
X is -CONH-R2-L2;
Y is -L-R1-L1;
R1 is a linker;
R2 is a bond or a linker
wherein the linker is a straight or branched chain alkylene group with 1 to 300 carbon atoms, wherein optionally
(a) one or more carbon atoms are replaced by oxygen, in particular wherein every third carbon atom is replaced by oxygen, e.g. a poylethyleneoxy group with 1 to 100 ethyleneoxy units; and/or
(b) one or more carbon atoms are replaced by nitrogen carrying a hydrogen atom, and the adjacent carbon atoms are substituted by oxo, representing an amide function -NH-CO-; and/or
(c) one or more carbon atoms are replaced by an ester function -O-CO-;
(d) the bond between two adjacent carbon atoms is a double or a triple bond; and/or
(e) two adjacent carbon atoms are replaced by a disulfide linkage;
L is a bond or a group selected from 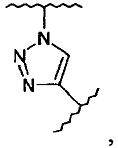 -(NRx)-, -O-, -C=N-, -C(=O)-, -C(=O)-NH-, -NH-C(=O)-, -C(=O)H, -CRx=CRy-, -C≡C- and phenyl, wherein Rx and Ry are independently H or (C₁-C₆)alkyl;
L1 and L2 are, independent of each other, at least one label optionally bound to a solid support; and
n is 1,
or
R2 is a bond;
L2 is a substrate, suitable for a coupled bioluminescent assay;
Y is H; and
n is 0.

2. Probe according to claim 1, wherein label L1 and L2 are independently of each other a spectroscopic probe such as a fluorophore; a quencher or a chromophore; a magnetic probe; a contrast reagent; a molecule which is one part of a specific binding pair which is capable of specifically binding to a partner; a molecule covalently attached to a solid support, where the support may be a glass slide, a microtiter plate or any polymer; a biomolecule with desirable enzymatic, chemical or physical properties; or a molecule possessing a combination of any of the properties listed above; or a positively charged linear or branched polymer.

3. Probe according to claim 2, wherein label L1 and L2 are independently of each other bound to a positively charged linear or branched polymer.

4. Probe according to claim 3, wherein one label L1 and L2 is a linear poly(arginine) of D- and/or L-arginine with 6 - 15 arginine residues.

5. Probe according to any one of claims 2 to 4, wherein L1 is one member and L2 is the other member of two interacting spectroscopic probes L1 / L2.

6. Probe according to claim 5, wherein L1 / L2 is a FRET pair.

7. Probe according to claim 6, wherein one L1 / L2 is a fluorophor selected from Alexa 350, dimethylaminocoumarin, 5/6-carboxyfluorescein, Alexa 488, ATTO 488, DY-505, 5/6-carboxyfluorescein, Alexa 488, Alexa 532, Alexa 546, Alexa 555, ATTO 488, ATTO 532, tetramethylrhodamine, Cy 3, DY-505, DY-547, Alexa 635, Alexa 647, ATTO 600, ATTO 655, DY-632, Cy 5, DY-647 Cy 5.5, and the other label L1 / L2 is a quencher selected from Dabsyl, Dabcyl, BHQ 1, QSY 35, BHQ 2, QSY 9, ATTO 540Q, BHQ 3, ATTO 612Q, QSY 21.

8. Probe according to claim 1, wherein n is 0, R2 is a bond and L2 is a substrate, suitable for a coupled bioluminescent assay **characterized in** a modified aminoluciferin or a carboxy-terminal protected derivative thereof as a reporter group, which upon cleavage from the central scaffold A can generate a luminescent signal through its conversion by a luciferase.

9. A caspase probe selected from 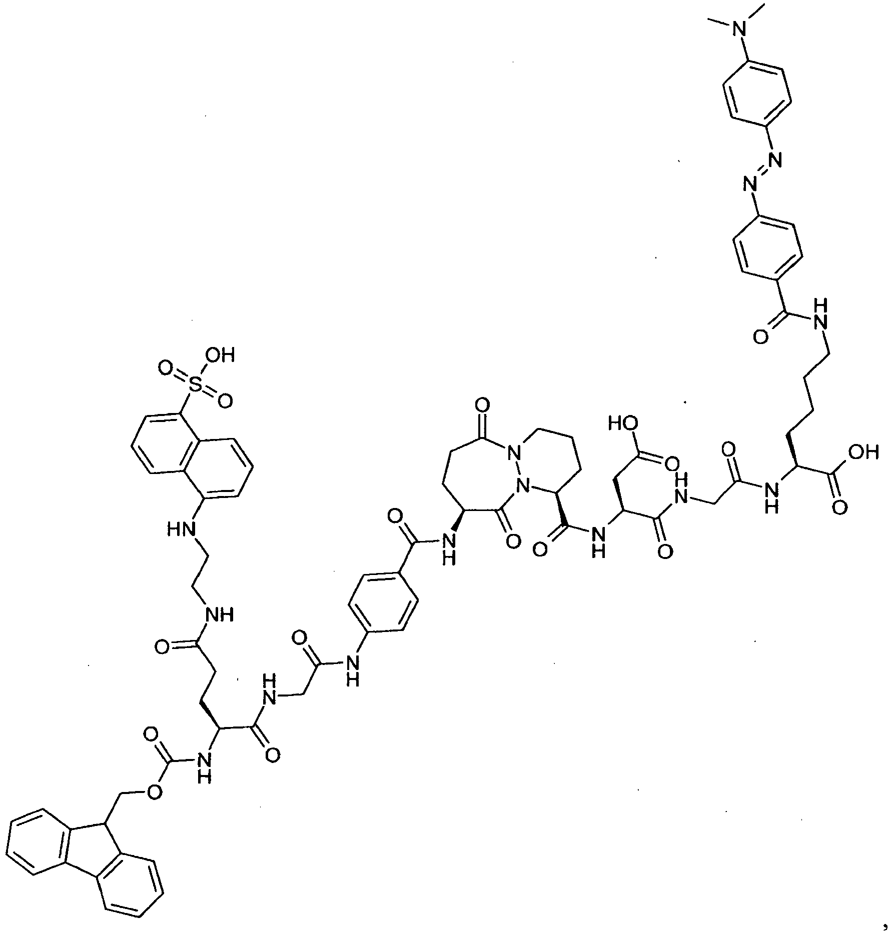 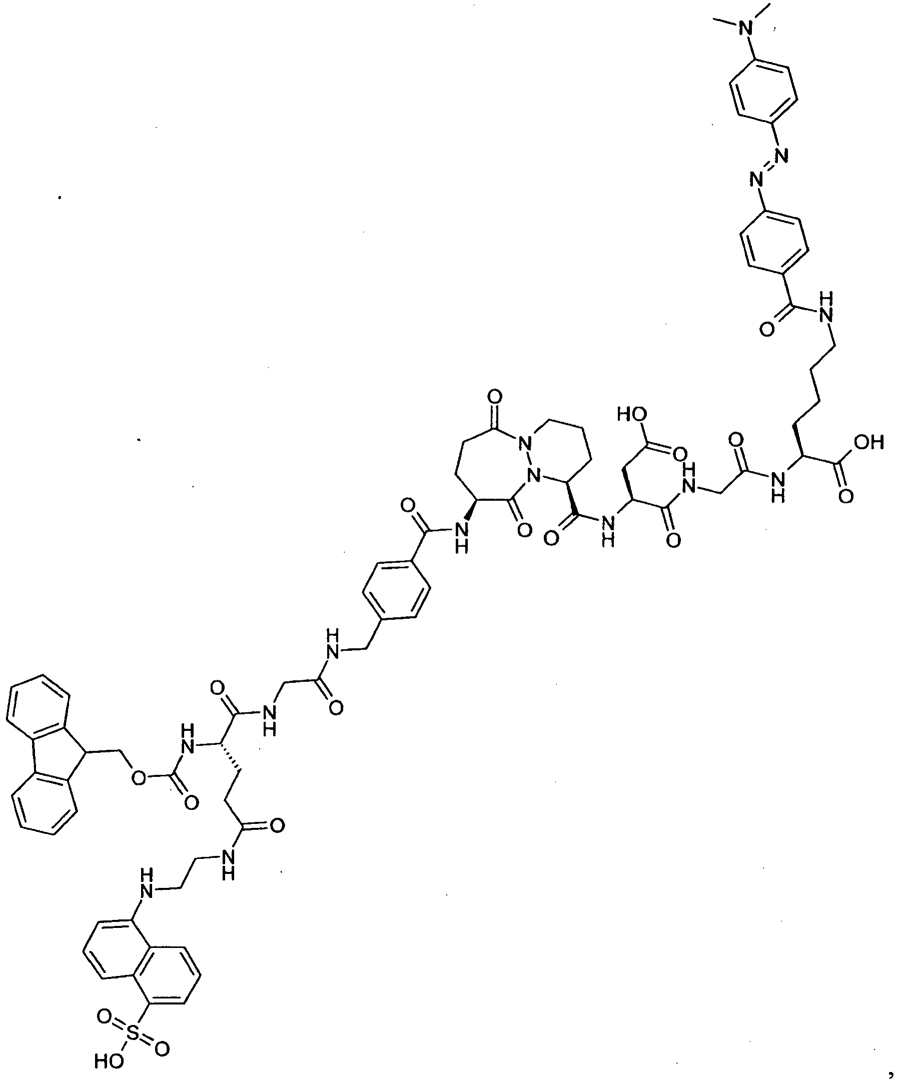 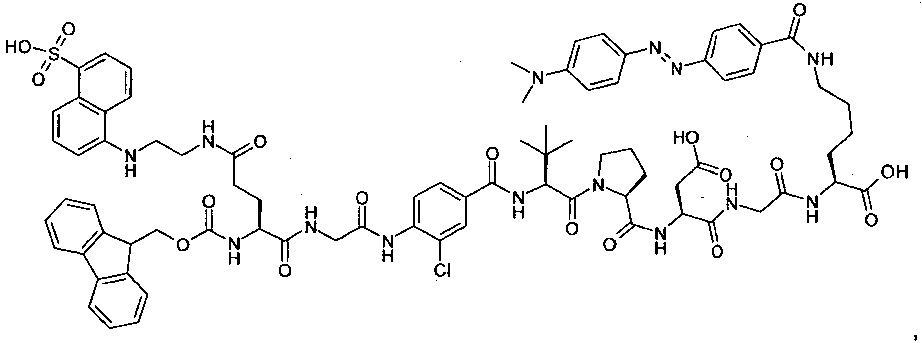 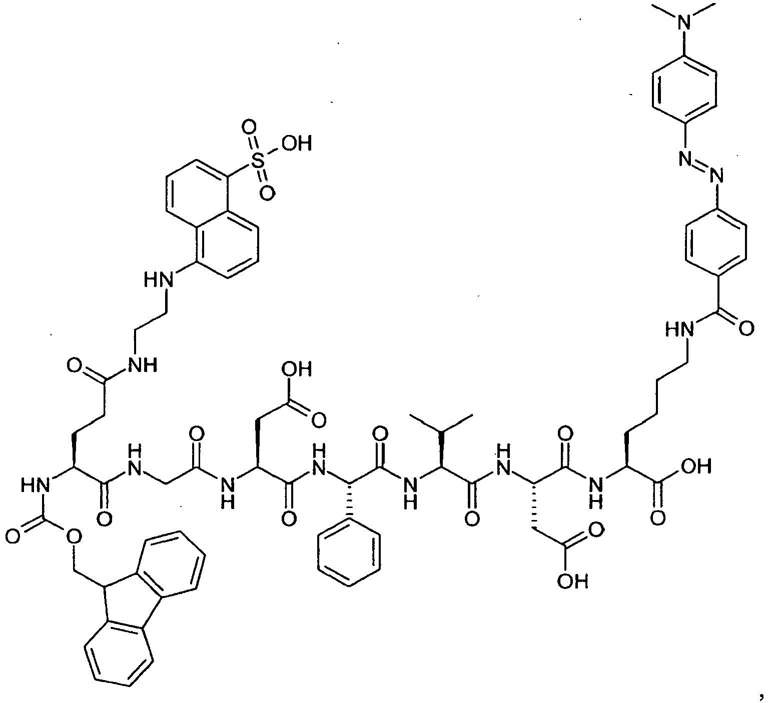 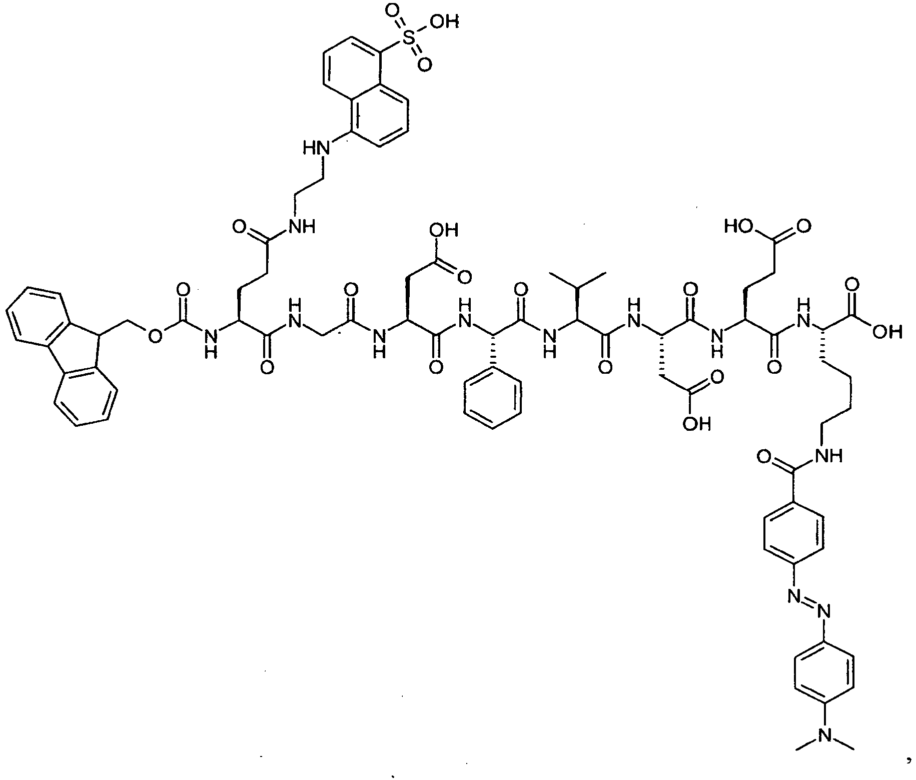 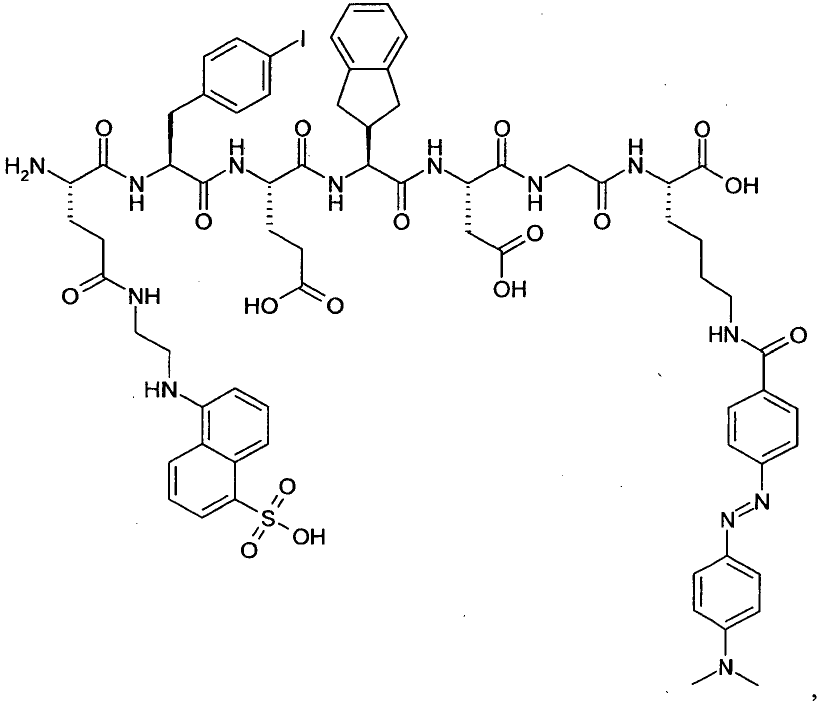 or

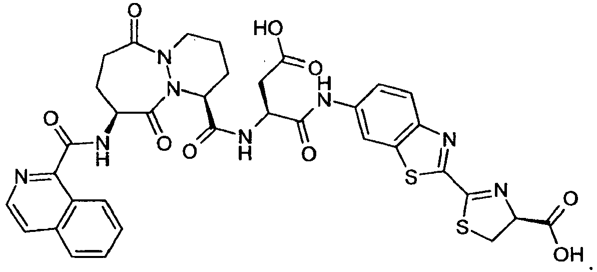

10. Preparation of a probe of the formula (I) according to claim 1 to 9 **characterized in**
if n is 1:
(a) reacting a compound of the formula (II)
L'-A-CO-OH (II)
with a compound of the formula L1-R1-H to a compound of the formula (III)
L1-R1-L-A-CO-OH (III)
(b) reacting the compound (III) with a compound H₂N-R2-L2 to the probe of the formula (I),
wherein L' is fluoro, chloro, bromo, cyano, nitro, amino, azido, alkylcarbonylamino, carboxy, carbamoyl, alkoxycarbonyl, aryloxycarbonyl, carbaldehyde, hydroxy, alkoxy, aryloxy, alkylcarbonyloxy, arylcarbonyloxy, a carbon-carbon double bond, a carbon-carbon triple bond, preferably amino, azido, hydroxy, cyano, carboxy, carbamoyl, carbaldehyde, or a carbon-carbon double or a carbon-carbon triple bond, more preferred amino, and
R1 and/or R2 may be protected by suitable orthogonally protecting groups and sequentially cleaved in the course of the preparation of the compound (I); and
if n is 0:
reacting a compound of the formula A-CO-OH (IV) with a compound H₂N-R2-L2 to the probe of the formula (I).

11. Use of a probe of the formula (I) according to claims 1 to 9 for molecular imaging in vitro, in cell-culture experiments, or in ex-vivo experiments..

12. Probe of the formula (I) according to claims 1 to 9 for use in a method for molecular imaging in a living organism.

## Patentansprüche

1. Molekülsonde für Cysteinproteasen der Formel (I)
{L1 -R1-L}ₙ-A-CO-NH-R2-L2 (I)
wobei
A für eine Gruppe steht, die von einer Caspase erkannt werden kann;
wobei A von Caspase-1 erkannt werden kann und aus einer Gruppe 1. - 28. der nachfolgenden Tabelle ausgewählt ist:
| | |
|---|---|
| 1. | 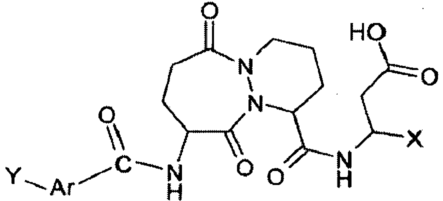 |
| | wobei Ar für Phenyl steht, wenn n gleich 1 ist, und Ar für Isochinolin steht, wenn n gleich 0 ist, vorzugsweise in der Konfiguration: |
| | 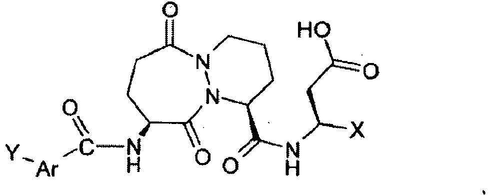 |
| 2. | 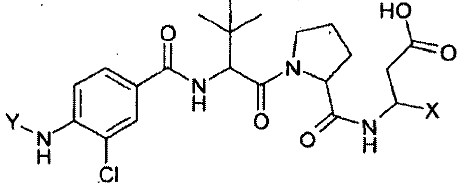 |
| | vorzugsweise in der Konfiguration: |
| | 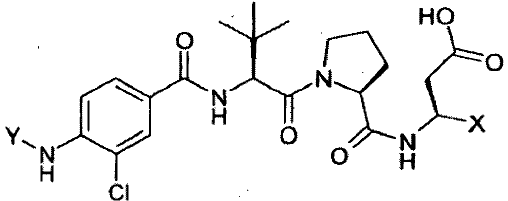 |
| 3. | 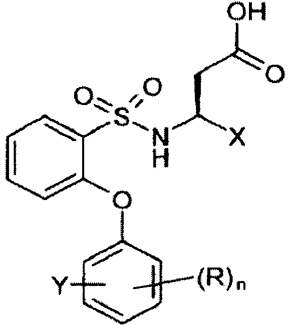 |
| | wobei R für (C₁-C₅)Alkyl, Phenyl, (C₅-C₆)Cycloalkyl steht; und n gleich 1-3 ist; |
| 4. | 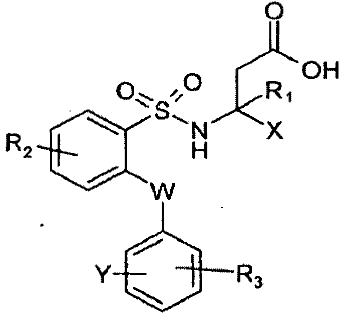 |
| | vorzugsweise in der Konfiguration: |
| | 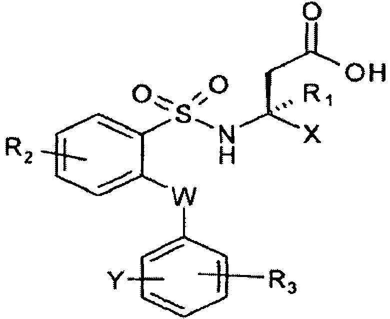 |
| | wobei R₁ für Wasserstoff, (C₁-C₆)Alkyl, Aryl oder - CH₂-Aryl steht, |
| | R₂ und R₃ unabhängig voneinander für Wasserstoff oder eine Aryl-, substituierte Aryl-, Heteroaryl-, substituierte Heteroaryl-, Cycloalkyl-, substituierte Cycloalkyl-, Heterocyclus- oder substituierte Heterocyclus-Gruppe, die an die Phenylgruppe, die eine Gruppe R₂ als Substituenten enthält, kondensiert ist, stehen; |
| | W für eine Bindung, NR₅, CO, S, O, SO₂, O(CHR₅)ₙ-, CHR₅, NR₅CO, CONR₅, OCHR₅, CHR₅O, SCHR₅, CHR₅S, SO₂NR₅, (C₁-C₆)Alkyl, NR₅SO₂, CH₂CHR₅, CHR₅CH₂, COCH₂ oder CH₂CO steht; wobei R₅ unabhängig für Wasserstoff, (C₁-C₆)Alkyl, Aryl, (CH₂)ₙ-Aryl oder (CH₂)ₙ-Cycloalkyl steht; und n jeweils unabhängig gleich 0 bis 5 ist; |
| 6. | 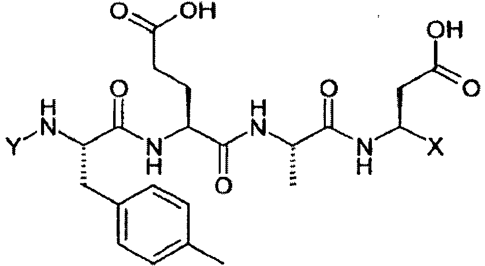 |
| 7. | 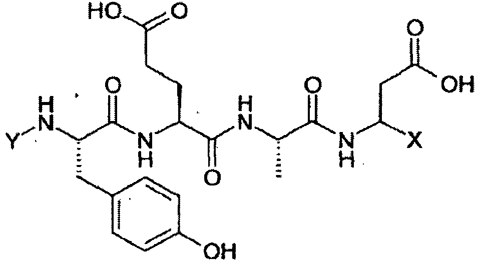 |
| 8. | 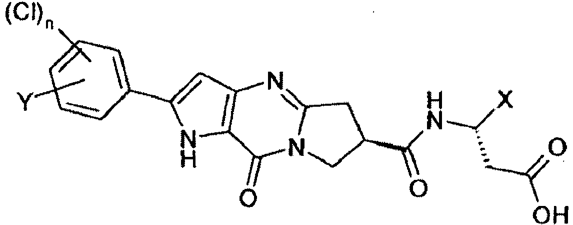 |
| | wobei n gleich 0 oder 1 ist; |
| 9. | 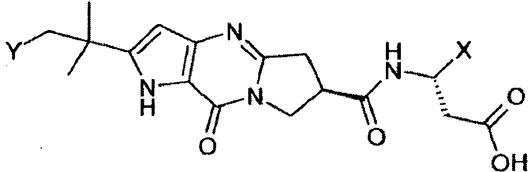 |
| 10. | 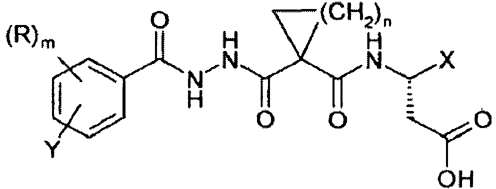 |
| | wobei n gleich 1-4 ist, |
| | m gleich 1 oder 2 ist und |
| | R für Methyl oder Methoxy steht; |
| 11. | 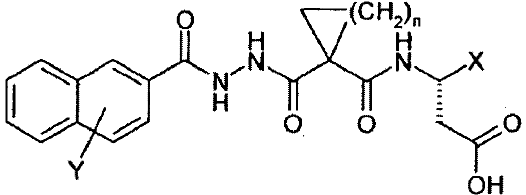 |
| | wobei n gleich 1-4 ist; |
| 12. | 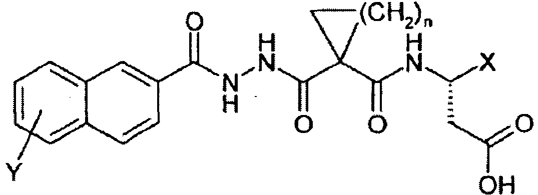 |
| | wobei n gleich 1-4 ist; |
| 13. | 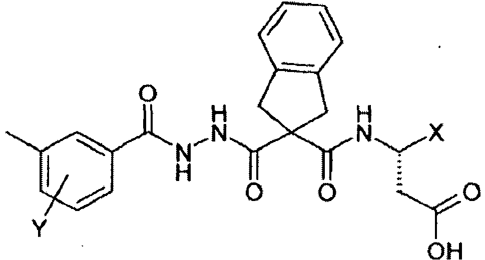 |
| 14. | 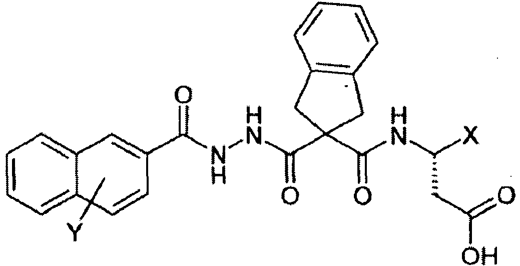 |
| 15. | 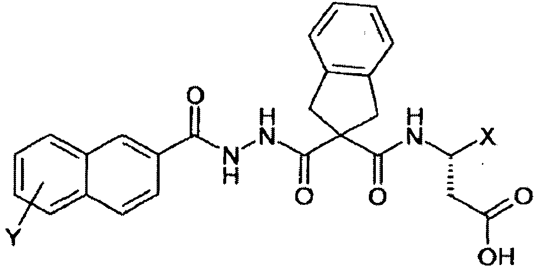 |
| 16. | 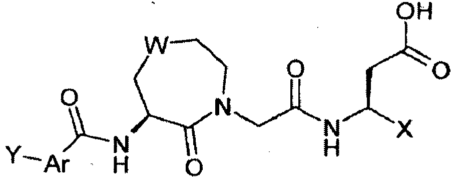 |
| | wobei W für S oder S(O)₂ steht und Ar für Aryl oder Heteroaryl, vorzugsweise Phenyl, Naphthyl, Benzothiophen oder Isochinolyl, steht; |
| 17. | 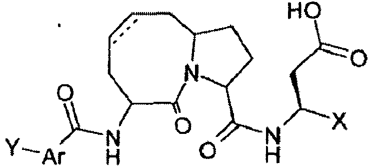 |
| | wobei Ar für eine aus Phenyl, Benzothiophen, Isochinolyl, Cinnamyl, Naphthyl ausgewählte Aryl- oder Heteroarylgruppe steht, die gegebenenfalls einfach oder unabhängig zweifach mit Methoxy, Chlor, Methyl, CF₃ substituiert ist, und wobei |
| | 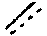 |
| | entweder eine Einfach- oder eine Doppelbindung bedeutet; |
| 18. | 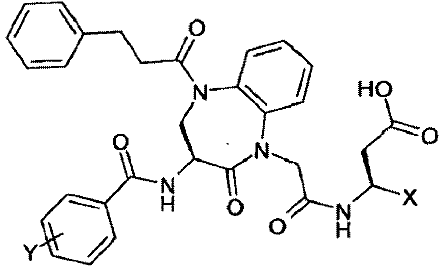 |
| 19. | 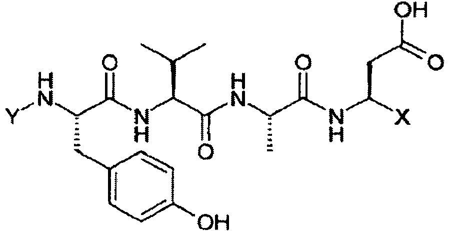 |
| 20. | 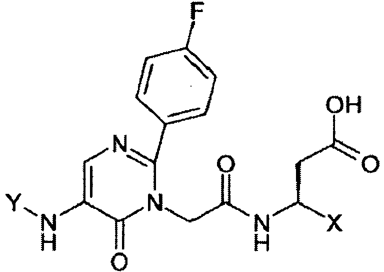 |
| 21. | 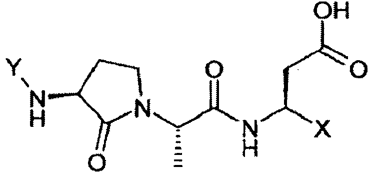 |
| 22. | 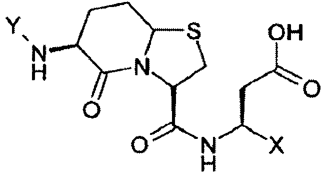 |
| 23. | 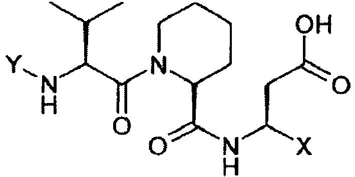 |
| 24. | 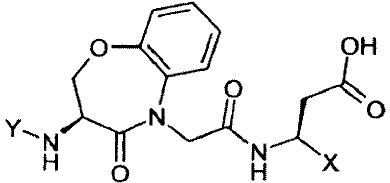 |
| 25. | 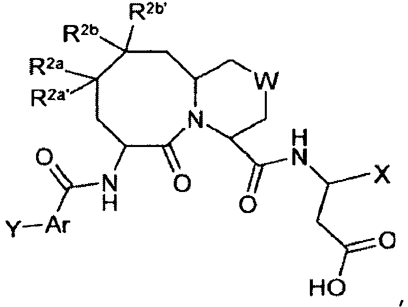 |
| | vorzugsweise in der all-(S)-Konfiguration, |
| | wobei Ar für Aryl oder Heteroaryl steht; |
| | W für CH₂, O oder NR9 steht, wobei R9 für Wasserstoff oder (C₁-C₆)Alkyl, Aryl, Heteroaryl, Heterocyclyl steht; |
| | R^{2a}, R^{2a'}, R^{2b} und R^{2b'} jeweils unabhängig für Wasserstoff, Hydroxyl, N(R⁶)₂, Halogen, (C₁-C₄)Alkyl, (C₁-C₄)Alkoxy und Gemische davon stehen, wobei R⁶ für Wasserstoff, (C₁-C₆)Alkyl, Cycloalkyl, (C₆-C₁₀)Aryl steht; oder R^{2a} und R^{2b} zusammengenommen eine Doppelbindung bilden können; |
| 26. | 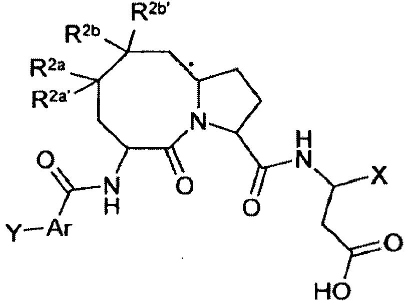 |
| | vorzugsweise in der all-(S)-Konfiguration, |
| | wobei Ar für Aryl oder Heteroaryl steht; |
| | R^{2a}, R^{2a'}, R^{2b} und R^{2b'} jeweils unabhängig für Wasserstoff, Hydroxyl, N(R⁶)₂, Halogen, (C₁-C₄)Alkyl, (C₁-C₄)Alkoxy und Gemische davon stehen, wobei R⁶ für Wasserstoff, (C₁-C₆)Alkyl, Cycloalkyl, (C₆-C₁₀)Aryl steht; oder R^{2a} und R^{2b} zusammengenommen eine Doppelbindung bilden können; |
| 27. | 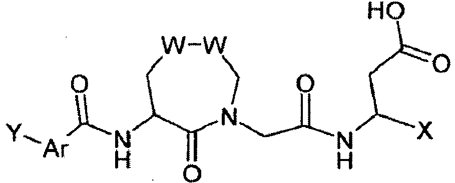 |
| | bevorzugt in der all-(S)-Konfiguration, |
| | wobei Ar für Aryl oder Heteroaryl steht; und |
| | W unabhängig ausgewählt ist aus: C(R¹)₂; C(O); NR²; S; S(O); S(O)₂; wobei R¹ und R² unabhängig für Wasserstoff, [C(R³)₂]ₚ(CH=CH)_{q}R³, C(=Z)R³, C(=Z) [C(R³)₂]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂, SO₂R³, P(O)(OR³)R³, P(O)(OR³)₂ stehen; wobei p gleich 0 bis 12 ist; wobei q gleich 0 bis 12 ist; wobei Z für O, S, NR³ steht; wobei R³ unabhängig für Wasserstoff, Alkyl, Cycloalkyl, Aryl, Heteroaryl, Heterocyclyl steht; |
| 28. | 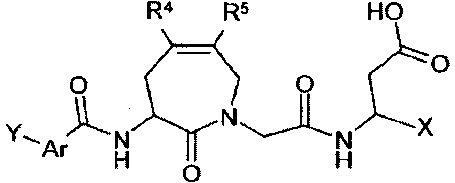 |
| | bevorzugt in der all-(S)-Konfiguration, |
| | wobei Ar für Aryl oder Heteroaryl steht; und R⁴ und R⁵unabhängig ausgewählt sind aus: C(R¹)₂; C(O); NR²; S; S(O); S(O)₂; wobei R¹ und R² unabhängig für Wasserstoff, [C(R³)₂]ₚ(CH=CH)_{q}R³, C(=Z)R³, C(=Z)[C(R³)₂]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂, SO₂R³, P(O) (OR³)R³, P(O) (OR³)₂ stechen; wobei p gleich 0 bis 12 ist; wobei q gleich 0 bis 12 ist; wobei Z für O, S, NR³ steht; wobei R³ unabhängig für Wasserstoff, Alkyl, Cycloalkyl, Aryl, Hetero-aryl, Heterocyclyl steht; |
oder A von Caspase-3 erkannt werden kann und aus einer Gruppe 29. - 42. der nachfolgenden Tabelle ausgewählt ist:
| | |
|---|---|
| 29. | 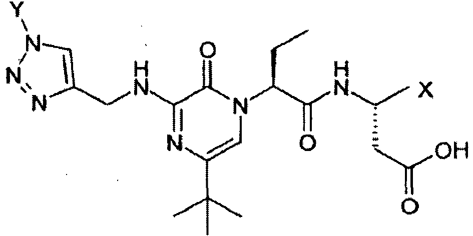 |
| 30. | 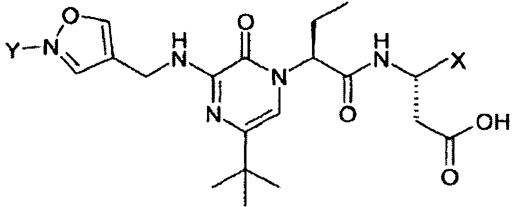 |
| 31. | 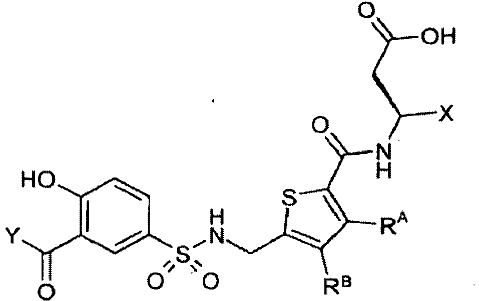 |
| | wobei R^{A} und R^{B} unabhängig für Wasserstoff, (C₁-C₆)-Alkyl, Hydroxyl, (C₁-C₆)Alkoxy oder Halogen stehen. |
| 32. | 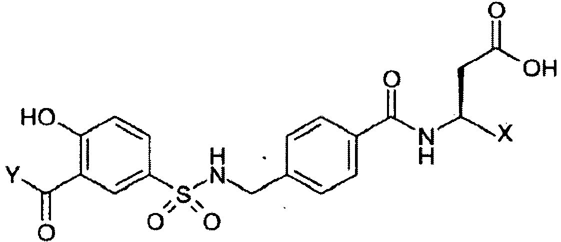 |
| 33. | 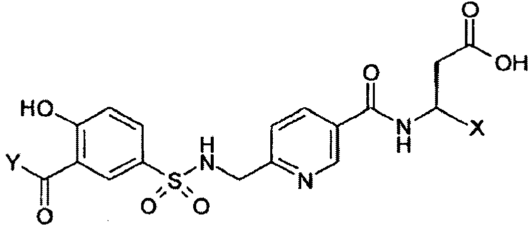 |
| 34. | 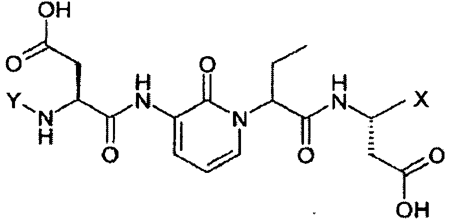 |
| 35. | 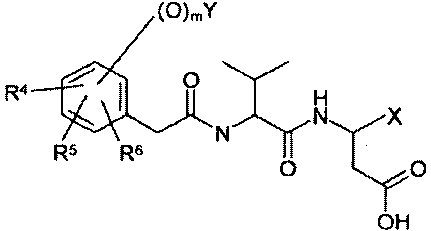 |
| | vorzugsweise in der all-(S)-Konfiguration, |
| | wobei m gleich 0 oder 1 ist; und |
| | R⁴, R⁵ und R⁶ unabhängig aus folgender Gruppe ausgewählt sind: |
| | 1) H, |
| | 2) Halogen, |
| | 3) (C₁-C₄)Alkoxy, gegebenenfalls substituiert mit 1-3 Halogenatomen, |
| | 4) NO₂, |
| | 5) OH, |
| | 6) Benzyloxy, wobei der Benzylanteil gegebenenfalls mit 1-2 aus der aus: Halogen, CN, (C₁-C₄)Alkyl und (C₁-C₄)Alkoxy, wobei das Alkyl und Alkoxy gegebenenfalls mit 1-3 Halogengruppen substituiert ist, bestehenden Gruppe ausgewählten Gliedern substituiert ist, |
| | 7) NH(C₁-C₄)Acyl, |
| | 8) (C₁-C₄)Acyl |
| | 9) O-(C₁-C₄)Alkyl-CO₂H, gegebenenfalls verestert mit einer (C₁-C₆)Alkyl- oder einer (C₅-C₇)Cycloalkylgruppe, |
| | 10) CH=CH-CO₂H, |
| | 11) CO₂H, |
| | 12) (C₁-C₅)Alkyl-CO₂H, |
| | 13) C(O)NH₂, gegebenenfalls am Stickstoffatom substituiert mit 1-2 (C₁-C₄)Alkylgruppen; |
| | 14) (C₁-C₅)Alkyl-C(O)NH₂, gegebenenfalls am Stickstoffatom substituiert mit 1-2 (C₁-C₄)Alkylgruppen; |
| | 15) S(O)₀₋₂₋(C₁-C₄)Alkyl; |
| | 16) (C₁-C₂) Alkyl-S(O)₀₋₂-(C₁-C₄) alkyl; |
| | 17) S(O)₀₋₂-(C₁-C₆)Alkyl oder S(O)₀₋₂-Phenyl, wobei der Alkyl- bzw. Phenylanteil davon gegebenenfalls mit 1-3 aus der aus: Halogen, CN, (C₁-C₄)Alkyl und (C₁-C₄)-Alkoxy, wobei das Alkyl und Alkoxy gegebenenfalls mit 1-3 Halogengruppen substituiert ist, bestehenden Gruppe ausgewählten Gliedern substituiert ist, |
| | 18) Benzoyl, gegebenenfalls substituiert mit 1-2 aus der aus: Halogen, CN, (C₁-C₄)Alkyl und (C₁-C₄)Alkoxy, wobei die Alkyl- und Alkoxygruppen gegebenenfalls mit 1-3 Halogengruppen substituiert sind, bestehenden Gruppe ausgewählten Gliedern, |
| | 19) Phenyl oder Naphthyl, gegebenenfalls substituiert mit 1-2 aus der aus: Halogen, CN, (C₁-C₄)Alkyl und (C₁-C₄)Alkoxy, wobei das Alkyl und Alkoxy gegebenenfalls mit 1-3 Halogengruppen substituiert ist, bestehenden Gruppe ausgewählten Gliedern, |
| | 20) CN, |
| | 21) (C₁-C₄)Alkylen-HET2, wobei HET2 einen 5-7-gliedrigen aromatischen oder nichtaromatischen Ring mit 1-4 aus O, S, NH und N(Cᵢ-C₄) ausgewählten Heteroatomen und gegebenenfalls mit 1-2 Oxogruppen repräsentiert, der gegebenenfalls mit 1-3 (C₁-C₄)-Alkyl-, OH-, Halogen- oder (C₁-C₄)Acyl-Gruppen substituiert ist; |
| | 22) O-(C₁-C₄)Alkyl-HET3, wobei HET3 für einen 5- oder 6-gliedrigen aromatischen oder nichtaromatischen Ring mit 1 bis 3 aus O, S und N ausgewählten Heteroatomen steht, der gegebenenfalls mit einer oder zwei aus Halogen und (C₁-C₄)Alkyl ausgewählten Gruppen substituiert ist und gegebenenfalls 1-2 Oxogruppen enthält, und |
| | 23) HET4, wobei HET4 für einen 5- oder 6-gliedrigen aromatischen oder nichtaromatischen Ring, und die benzokondensierten Analoge davon, mit 1 bis 4 aus O, S und N ausgewählten Heteroatomen steht und gegebenenfalls mit einer oder zwei aus Halogen, (C₁-C₄)Alkyl und (C₁-C₄)Acyl ausgewählten Gruppen substituiert ist; und wobei Halogen F, Cl, Br und I umfasst. |
| 36. | 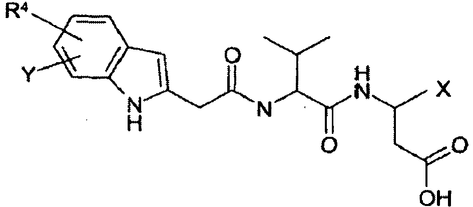 |
| | vorzugsweise in der all-(S)-Konfiguration, |
| | wobei R⁴ aus folgender Gruppe ausgewählt ist: |
| | 1) H, |
| | 2) Halogen, |
| | 3) (C₁-C₄)Alkoxy, gegebenenfalls substituiert mit 1-3 Halogenatomen, |
| | 4) NO₂, |
| | 5) OH, |
| | 6) Benzyloxy, wobei der Benzylanteil gegebenenfalls mit 1-2 aus der aus: Halogen, CN, (C₁-C₄)Alkyl und (C₁-C₄)Alkoxy, wobei das Alkyl und Alkoxy gegebenenfalls mit 1-3 Halogengruppen substituiert ist, bestehenden Gruppe ausgewählten Gliedern substituiert ist, |
| | 7) NH(C₁-C₄)Acyl, |
| | 8) (C₁-C₄)Acyl |
| | 9) O-(C₁-C₄)Alkyl-CO₂H, gegebenenfalls verestert mit einer (C₁-C₆)Alkyl- oder einer (C₅-C₇)Cycloalkylgruppe, |
| | 10) CH=CH-CO₂H, |
| | 11) CO₂H, |
| | 12) (C₁-C₅)Alkyl-CO₂H, |
| | 13) C(O)NH₂, gegebenenfalls am Stickstoffatom substituiert mit 1-2 (C₁-C₄)Alkylgruppen; |
| | 14) (C₁-C₅)Alkyl-C(O)NH₂, gegebenenfalls am Stickstoffatom substituiert mit 1-2 (C₁-C₄)Alkylgruppen; |
| | 15) S(O)₀₋₂-(C₁-C₄)Alkyl; |
| | 16) (C₁-C₂)Alkyl-S(O)₀₋₂-(C₁-C₄)alkyl; |
| | 17) S(O)₀₋₂-(C₁-C₆)Alkyl oder S(O)₀₋₂-Phenyl, wobei der Alkyl- bzw. Phenylanteil davon gegebenenfalls mit 1-3 aus der aus: Halogen, CN, (C₁-C₄)Alkyl und (C₁-C₄)-Alkoxy, wobei das Alkyl und Alkoxy gegebenenfalls mit 1-3 Halogengruppen substituiert ist, bestehenden Gruppe ausgewählten Gliedern substituiert ist, |
| | 18) Benzoyl, gegebenenfalls substituiert mit 1-2 aus der aus: Halogen, CN, (C₁-C₄)Alkyl und (C₁-C₄)Alkoxy, wobei die Alkyl- und Alkoxygruppen gegebenenfalls mit 1-3 Halogengruppen substituiert sind, bestehenden Gruppe ausgewählten Gliedern, |
| | 19) Phenyl oder Naphthyl, gegebenenfalls substituiert mit 1-2 aus der aus: Halogen, CN, (C₁-C₄)Alkyl und (C₁-C₄)Alkoxy, wobei das Alkyl und Alkoxy gegebenenfalls mit 1-3 Halogengruppen substituiert ist, bestehenden Gruppe ausgewählten Gliedern, |
| | 20) CN, |
| | 21) (C₁-C₄)Alkylen-HET2, wobei HET2 einen 5-7-gliedrigen aromatischen oder nichtaromatischen Ring mit 1-4 aus O, S, NH und N(C₁-C₄) ausgewählten Heteroatomen und gegebenenfalls mit 1-2 Oxogruppen repräsentiert, der gegebenenfalls mit 1-3 (C₁-C₄)-Alkyl-, OH-, Halogen- oder (C₁-C₄)Acyl-Gruppen substituiert ist; |
| | 22) O-(C₁-C₄)Alkyl-HET3, wobei HET3 für einen 5- oder 6-gliedrigen aromatischen oder nichtaromatischen Ring mit 1 bis 3 aus O, S und N ausgewählten Heteroatomen steht, der gegebenenfalls mit einer oder zwei aus Halogen und (C₁-C₄)Alkyl ausgewählten Gruppen substituiert ist und gegebenenfalls 1-2 Oxo-gruppen enthält, und |
| | 23) HET4, wobei HET4 für einen 5- oder 6-gliedrigen aromatischen oder nichtaromatischen Ring, und die benzokondensierten Analoge davon, mit 1 bis 4 aus O, S und N ausgewählten Heteroatomen steht und gegebenenfalls mit einer oder zwei aus Halogen, (C₁-C₄)Alkyl und (C₁-C₄)Acyl ausgewählten Gruppen substituiert ist; und wobei Halogen F, Cl, Br und I umfasst. |
| 37. | 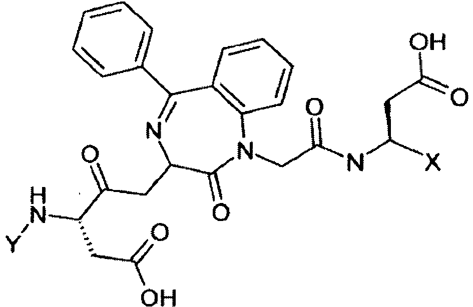 |
| 38. | 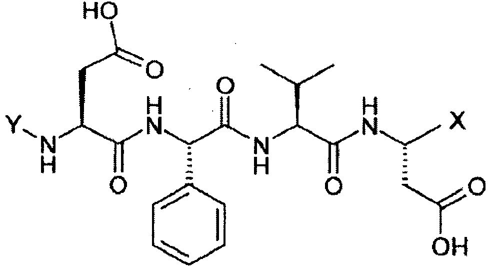 |
| 39. | 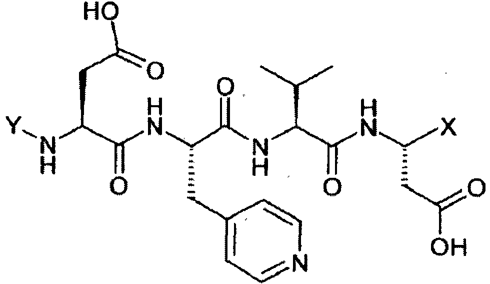 |
| 40. | 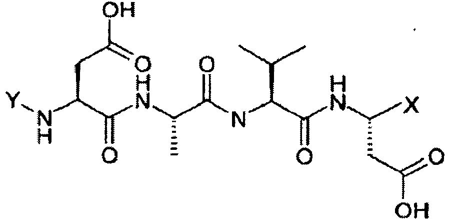 |
| 41. | 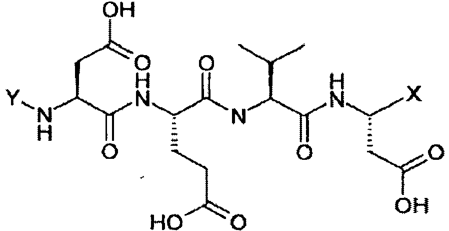 |
| 42. | 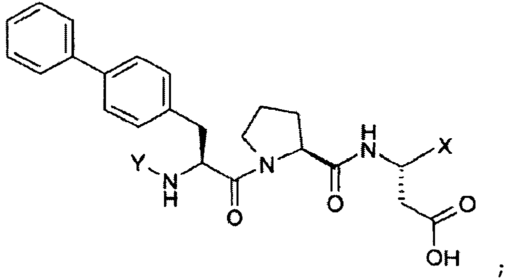 |
oder A von Caspase-8 erkannt werden kann und aus einer Gruppe 43. - 44. der nachfolgenden Tabelle ausgewählt ist:
| | |
|---|---|
| 43. | 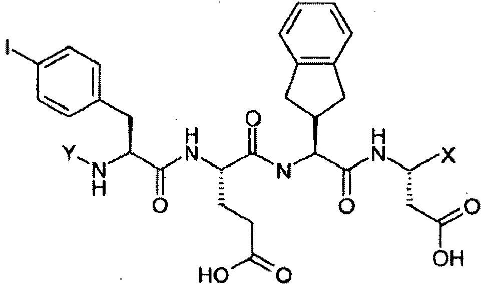 |
| 44. | 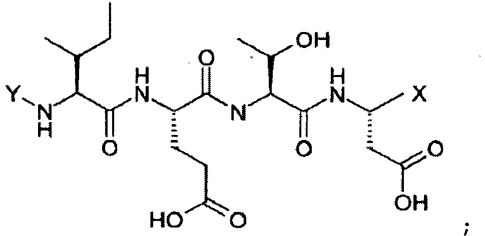 |
X für -CONH-R2-L2 steht;
Y für -L-R1-L1 steht;
R1 für einen Linker steht;
R2 für eine Bindung oder einen Linker steht,
wobei es sich bei dem Linker um eine gerad- oder verzweigtkettige Alkylengruppe mit 1 bis 300 Kohlenstoffatomen handelt, wobei gegebenenfalls
(a) ein oder mehrere Kohlenstoffatome durch Sauerstoff ersetzt sind, insbesondere wobei jedes dritte Kohlenstoffatom durch Sauerstoff ersetzt ist, z.B. eine Polyethylenoxygruppe mit 1 bis 100 Ethylenoxy-Einheiten; und/oder
(b) ein oder mehrere Kohlenstoffatome durch ein Wasserstoffatom tragenden Stickstoff ersetzt sind und die benachbarten Kohlenstoffatome mit Oxo substituiert sind, was eine Amidfunktion -NH-COrepräsentiert; und/oder
(c) ein oder mehrere Kohlenstoffatome durch eine Esterfunktion -O-CO- ersetzt sind;
(d) es sich bei der Bindung zwischen zwei benachbarten Kohlenstoffatomen um eine Doppel- oder eine Dreifachbindung handelt; und/oder
(e) zwei benachbarte Kohlenstoffatome durch eine Disulfid-Verknüpfung ersetzt sind;
L für eine Bindung oder eine aus 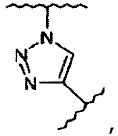 -(NRx)-, -O-, -C=N-, -C(=O)-, -C(=O)-NH-, -NH-C(=0)-, -C(=O)H, -CRx=CRy-, -C=C- und Phenyl, wobei Rx und Ry unabhängig für H oder (C₁-C₆)Alkyl stehen, ausgewählte Gruppe steht;
L1 und L2 unabhängig voneinander für wenigstens eine gegebenenfalls an einen festen Träger gebundene Markierung stehen; und
n gleich 1 ist,
oder
R2 für eine Bindung steht;
L2 für ein Substrat steht, das für einen gekoppelten Biolumineszenz-Test geeignet ist;
Y für H steht; und
n gleich 0 ist.

2. Sonde nach Anspruch 1, wobei es sich bei der Markierung L1 und L2 unabhängig voneinander um eine spektroskopische Sonde, wie etwa einen Fluorophor; einen Quencher oder einen Chromophor; eine magnetische Sonde; ein Kontrastreagens; ein Molekül, das einen Teil eines spezifischen Bindungspaars darstellt, das spezifisch an einen Partner binden kann; ein kovalent an einen festen Träger, bei dem es sich um einen Glasobjektträger, eine Mikrotiterplatte oder ein beliebiges Polymer handeln kann, gebundenes Molekül; ein Biomolekül mit wünschenswerten enzymatischen, chemischen oder physikalischen Eigenschaften; oder ein Molekül, das eine Kombination beliebiger der oben aufgeführten Eigenschaften besitzt; oder ein positiv geladenes lineares oder verzweigtes Polymer handelt.

3. Sonde nach Anspruch 2, wobei Markierung L1 und L2 unabhängig voneinander an ein positiv geladenes lineares oder verzweigtes Polymer gebunden sind.

4. Sonde nach Anspruch 3, wobei es sich bei einer Markierung L1 und L2 um ein lineares Polyarginin von D- und/oder L-Arginin mit 6 - 15 Argininresten handelt.

5. Sonde nach einem der Ansprüche 2 bis 4, wobei es sich bei L1 um ein Mitglied und bei L2 um das andere Mitglied zweier wechselwirkender spektroskopischer Sonden L1 / L2 handelt.

6. Sonde nach Anspruch 5, wobei es sich bei L1 / L2 um ein FRET-Paar handelt.

7. Sonde nach Anspruch 6, wobei es sich bei einem L1 bzw. L2 um einen aus Alexa 350, Dimethylaminocumarin, 5/6-Carboxyfluorescein, Alexa 488, ATTO 488, DY-505, 5/6-Carboxyfluorescein, Alexa 488, Alexa 532, Alexa 546, Alexa 555, ATTO 488, ATTO 532, Tetramethylrhodamin, Cy 3, DY-505, DY-547, Alexa 635, Alexa 647, ATTO 600, ATTO 655, DY-632, Cy 5, DY-647, Cy 5.5 ausgewählten Fluorophor und bei der anderen Markierung L1 bzw. L2 um einen aus Dabsyl, Dabcyl, BHQ 1 , QSY 35, BHQ 2, QSY 9, ATTO 540Q, BHQ 3, ATTO 612Q, QSY 21 ausgewählten Quencher handelt.

8. Sonde nach Anspruch 1, wobei n gleich 0 ist, R2 für eine Bindung und L2 für ein Substrat steht, das für einen gekoppelten Biolumineszenz-Test geeignet ist, der durch ein modifiziertes Aminoluciferin oder ein carboxyterminal geschütztes Derivat davon als Reportergruppe gekennzeichnet ist, die bei Abspaltung von dem zentralen Gerüst A ein Lumineszenzsignal über ihre Umwandlung durch eine Luciferase erzeugen kann.

9. Caspase-Sonde, ausgewählt aus 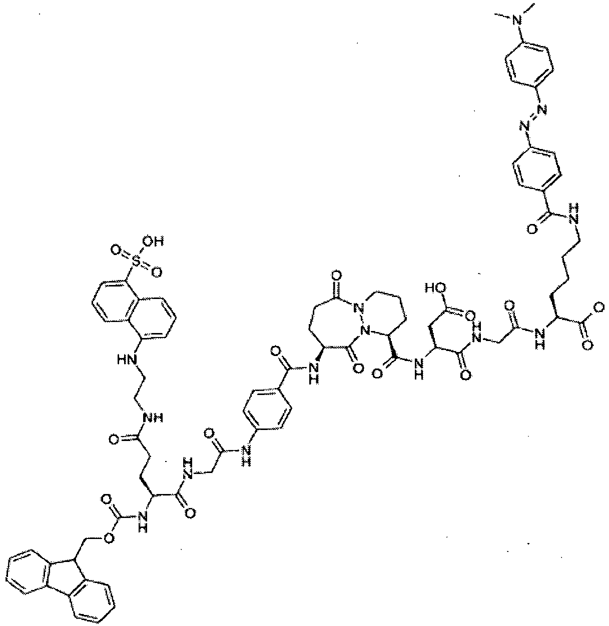 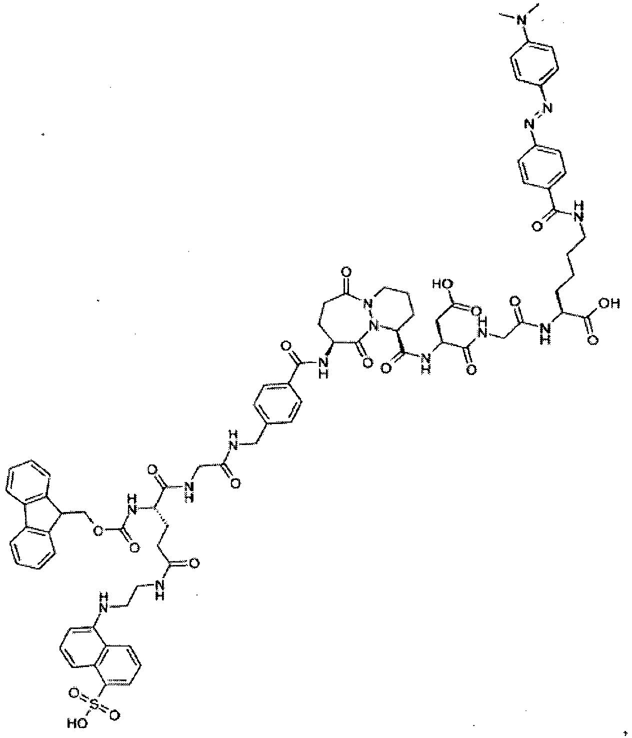 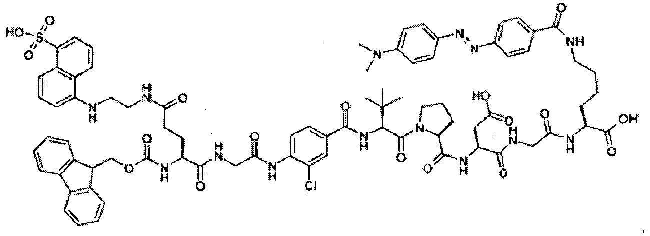 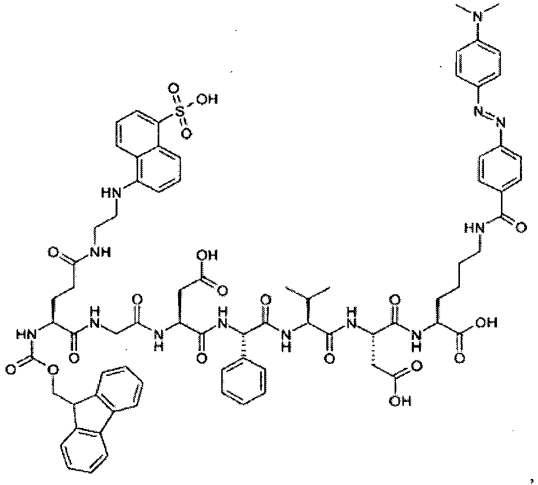 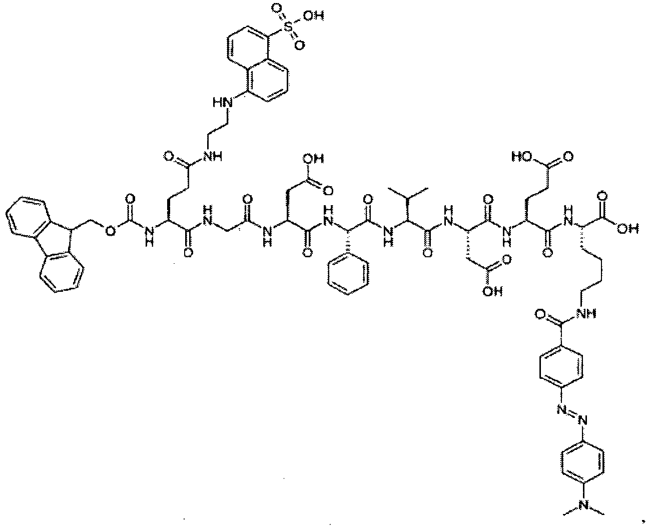 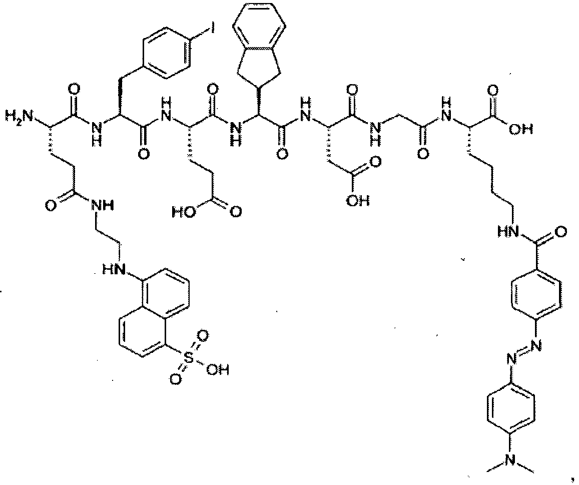 oder

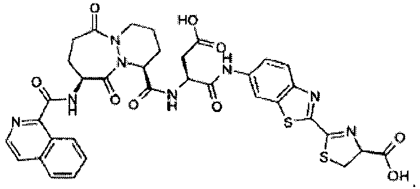

10. Herstellung einer Sonde der Formel (I) nach Anspruch 1 bis 9, **gekennzeichnet durch**,
wenn n gleich 1 ist:
(a) Umsetzen einer Verbindung der Formel (II)
L'-A-CO-OH (II)
mit einer Verbindung der Formel L1-R1-H zu einer Verbindung der Formel (III)
L1-R1-L-A-CO-OH (III)
(b) Umsetzen der Verbindung (III) mit einer Verbindung H₂N-R2-L2 zur Sonde der Formel (I),
wobei L' für Fluor, Chlor, Brom, Cyano, Nitro, Amino, Azido, Alkylcarbonylamino, Carboxy, Carbamoyl, Alkoxycarbonyl, Aryloxycarbonyl, Carbaldehyd, Hydroxy, Alkoxy, Aryloxy, Alkylcarbonyloxy, Arylcarbonyloxy, eine Kohlenstoff-Kohlenstoff-Doppelbindung, eine Kohlenstoff-Kohlenstoff-Dreifachbindung, vorzugsweise Amino, Azido, Hydroxy, Cyano, Carboxy, Carbamoyl, Carbaldehyd oder eine Kohlenstoff-Kohlenstoff-Doppel- oder eine Kohlenstoff-Kohlenstoff-Dreifachbindung, stärker bevorzugt Amino steht und
R1 und/oder R2 **durch** geeignete Orthogonalschutz-gruppen geschützt sein und nacheinander bei der Herstellung der Verbindung (I) gespalten werden können; und
wenn n gleich 0 ist:
Umsetzen einer Verbindung der Formel A-CO-OH (IV) mit einer Verbindung H₂N-R2-L2 zur Sonde der Formel (I).

11. Verwendung einer Sonde der Formel (I) nach Anspruch 1 bis 9 zur molekularen Bildgebung in vitro, bei Zellkulturexperimenten oder bei Ex-vivo-Experimenten.

12. Sonde der Formel (I) nach Anspruch 1 bis 9 zur Verwendung bei einem Verfahren zur molekularen Bildgebung bei einem lebenden Organismus.

## Revendications

1. Sonde moléculaire pour des cystéine protéases de formule (I)
{L1-R1-L}ₙ-A-CO-NH-R2-L2 (I)
dans laquelle
A est un groupe reconnaissable par une caspase ;
A étant reconnaissable par la caspase-1 et étant choisi parmi un groupe 1 à 28 du tableau ci-dessous :
| | |
|---|---|
| 1. | 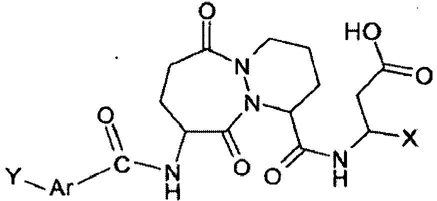 |
| | où Ar est phényle si n est 1 et Ar est isoquinoléine si n est 0, de préférence dans la configuration : |
| | 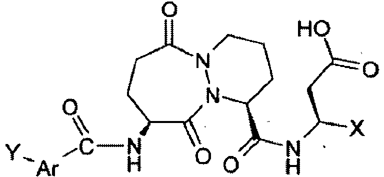 |
| 2. | 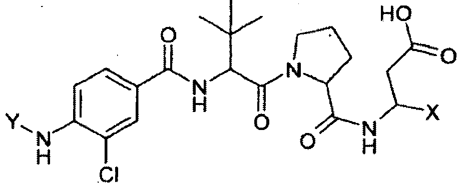 |
| | de préférence dans la configuration : |
| | 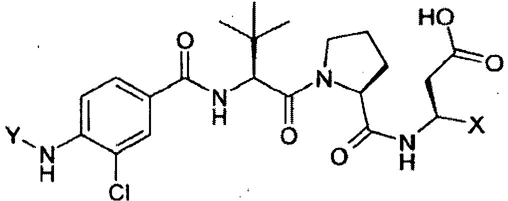 |
| 3. | 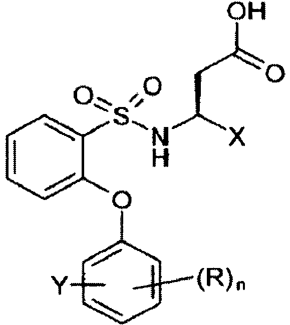 |
| | où R est alkyle en C₁-C₅, phényle, cycloalkyle en C₅-C₆ ; et n est 1 à 3 ; |
| 4. | 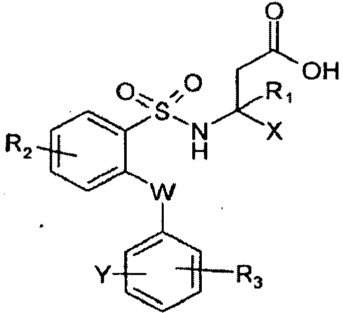 |
| | de préférence dans la configuration : |
| | 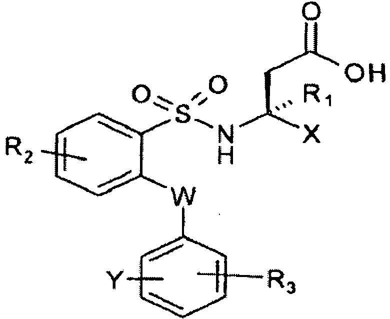 |
| | où R₁ est hydrogène, alkyle en C₁-C₆, aryle ou -CH₂-aryle, |
| | R₂ et R₃ sont indépendamment l'un de l'autre hydrogène, ou un groupe aryle, aryle substitué, hétéroaryle, hétéroaryle substitué, cycloalkyle, cycloalkyle substitué, hétérocycle, ou hétérocycle substitué qui est condensé avec le groupe phényle qui contient un groupe R₂ en tant que substituant ; W est une liaison, NR₅, CO, S, O, SO₂, O(CHR₅)ₙ-, CHR₅, NR₅CO, CONR₅, OCHR₅, CHR₅O, SCHR₅, CHR₅S, SO₂NR₅, alkyle en C₁-C₆, NR₅SO₂, CH₂CHR₅, CHR₅CH₂, COCH₂ ou CH₂CO ; |
| | où R₅ est indépendamment hydrogène, alkyle en C₁-C₆, aryle, (CH₂)ₙ-cycloaalkyle ; et chaque n est indépendamment 0 à 5 ; |
| 6. | 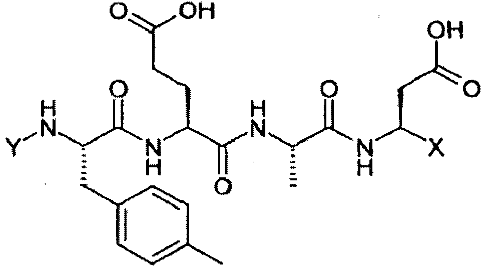 |
| 7. | 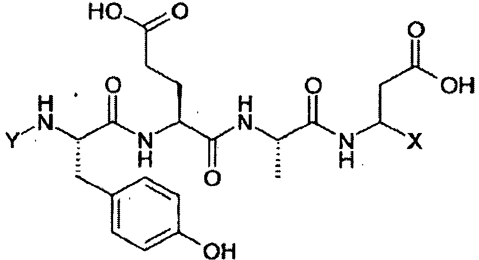 |
| 8. | 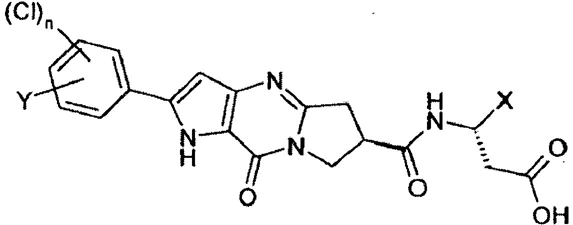 |
| | où n est 0 ou 1 ; |
| 9. | 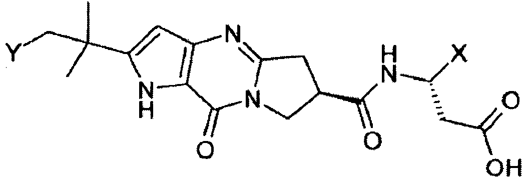 |
| 10. | 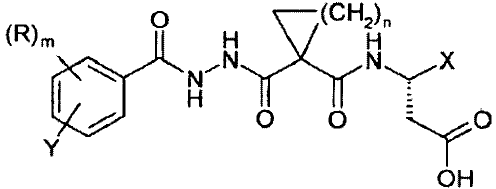 |
| | où n est 1 à 4 |
| | m est 1 ou 2, et |
| | R est méthyle ou méthoxy ; |
| 11. | 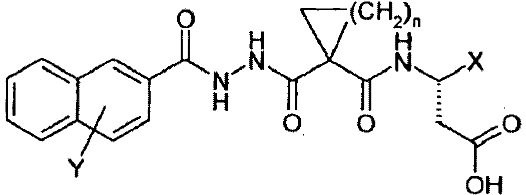 |
| | où n est 1 à 4 ; |
| 12. | 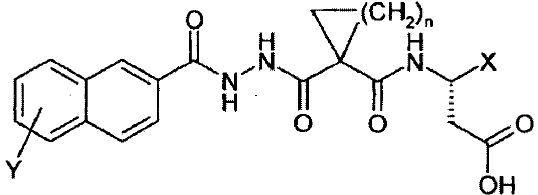 |
| | où n est 1 à 4 ; |
| 13. | 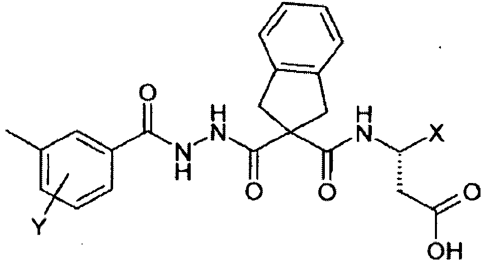 |
| 14. | 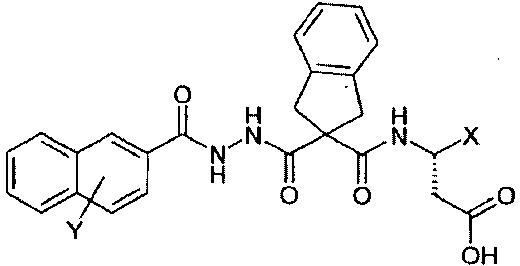 |
| 15. | 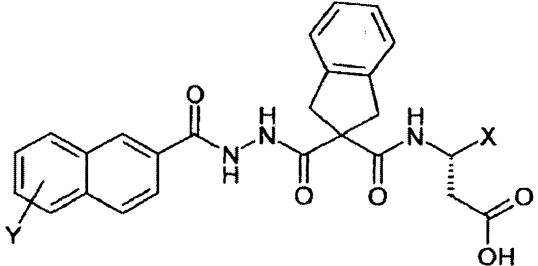 |
| 16. | 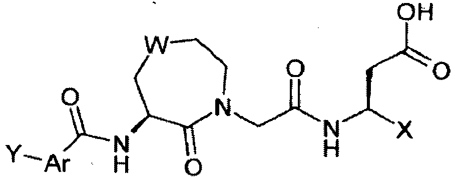 |
| | où W est S ou S(O)₂, et |
| | Ar est aryle ou hétéroaryle, de préférence phényle, naphtyle, benzothiophène ou isoquinolyle ; |
| 17. | 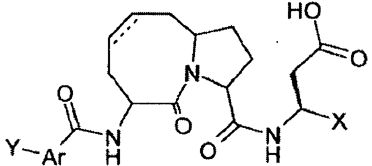 |
| | où Ar est un groupe aryle ou hétéroaryle choisi parmi phényle, benzothiophène, isoquinolyle, cinnamyle, naphtyyle, qui est facultativement substitué une fois ou indépendamment deux fois par méthoxy, chloro, méthyle, CF₃ et où |
| | 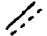 |
| | désigne une simple ou double liaison ; |
| 18. | 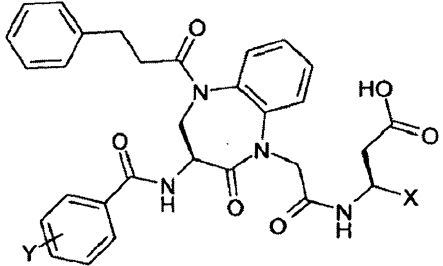 |
| 19. | 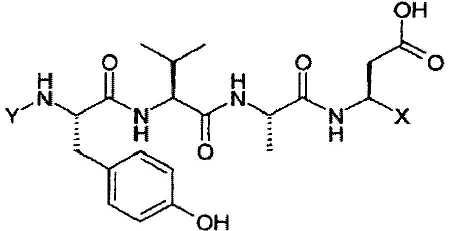 |
| 20. | 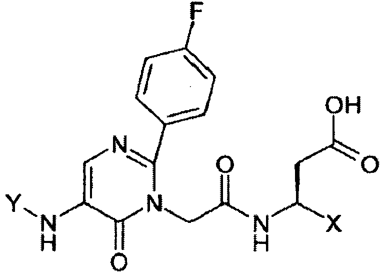 |
| 21. | 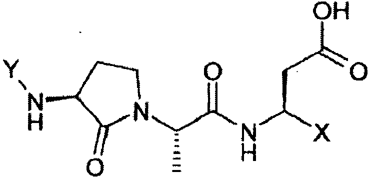 |
| 22. | 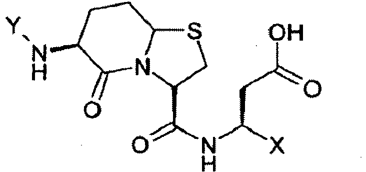 |
| 23. | 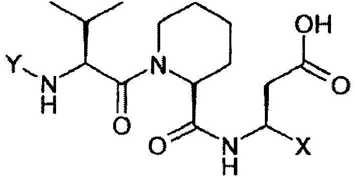 |
| 24. | 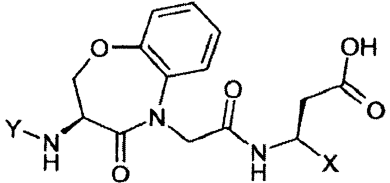 |
| 25. | 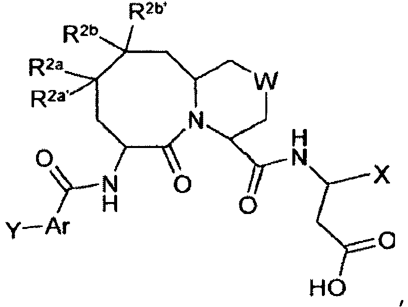 |
| | de préférence dans la configuration tout-(S), où Ar est aryle ou hétéroaryle ; |
| | W est CH₂, O ou NR9 où R9 est hydrogène ou alkyle en C₁-C₆, aryle, hétéroaryle, hétérocyclyle ; |
| | R^{2a}, R^{2a}', R^{2b} et R^{2b}' sont chacun indépendamment hydrogène, hydroxyle, N(R⁶)₂, halogène, alkyle en C₁-C₄, alcoxy en C₁-C₄, et des mélanges de ceux-ci dans lesquels R⁶ est hydrogène, alkyle en C₁-C₆, cycloalkyle, aryle en C₆-C₁₀ ; ou R^{2a} et R^{2b} peuvent former conjointement une double liaison ; |
| 26. | 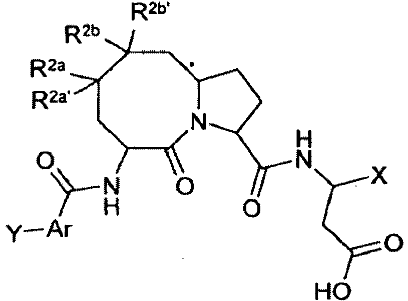 |
| | de préférence dans la configuration tout-(S), où Ar est aryle ou hétéroaryle ; |
| | R^{2a}, R^{2a'}, R^{2b} et R^{2b'} sont chacun indépendamment hydrogène, hydroxyle, N(R⁶)₂, halogène, alkyle en C₁-C₄, alcoxy en C₁-C₄, et des mélanges de ceux-ci dans lesquels R⁶ est hydrogène, alkyle en C₁-C₆, cycloalkyle, aryle en C₆-C₁₀ ; ou R^{2a} et R^{2b} peuvent former conjointement une double liaison ; |
| 27. | 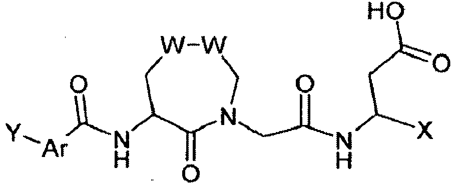 |
| | de préférence dans la configuration tout-(S), où Ar est aryle ou hétéroaryle ; et W est indépendamment choisi parmi : C(R¹)₂ ; C(O) ; NR² ; S ; S(O) ; S(O)₂ ; où R¹ et R² sont indépendamment hydrogène , [C(R³)₂]ₚ(CH=CH)_{q}R³, C(=Z)R³, C(=Z)[C(R³)₂]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, Cl, Br, I, SO₃H, OSO₃H, SO₂N(R³)₂, SO₂R³, P(O) (OR³)R³, P(O)(OR³)₂ ; où p est 0 à 12 ; où q est 0 à 12 ; où Z est 0, S, NR³ ; où R³ est indépendamment hydrogène, alkyle, cycloalkyle, aryle, hétéroaryle, hétérocyclyle. |
| 28. | 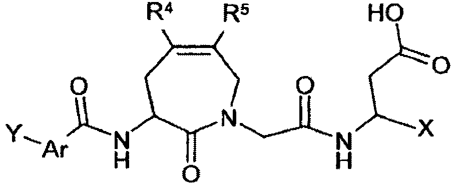 |
| | de préférence dans la configuration tout-(S) où Ar est aryle ou hétéroaryle ; et R⁴ et R⁵ sont indépendamment choisis parmi : C(R¹)₂ ; C(O) ; NR² ; S ; S(O) ; S(O)₂; où R¹ et R² sont indépendamment hydrogène , [C(R³)₂]ₚ(CH=CH)_{q}R³, C(=Z)R³, C(=Z)[C(R³)₂]ₚ(CH=CH)_{q}R³, C(=Z)N(R³)₂, C(=Z)NR³N(R³)₂, CN, CF₃, N(R³)₂, NR³CN, NR³C(=Z)R³, NRC(=Z)N(R³)₂, NHN(R³)₂, NHOR³, NO₂, OR³, OCF₃, F, C1, Br, I, SO₃H, OSO₃H, SO₂N(R³)₂, SO₂R³, P(O)(OR³)R³, P(O)(OR³)₂ ; où p est 0 à 12 ; où q est 0 à 12 ; où Z est O, S, NR³ ; où R³ est indépendamment hydrogène, alkyle, cycloalkyle, aryle, hétéroaryle, hétérocyclyle ; |
ou A est reconnaissable par la caspase 3 et est choisi dans le groupe de 29 à 42 dans le tableau ci-dessous :
| | |
|---|---|
| 29. | 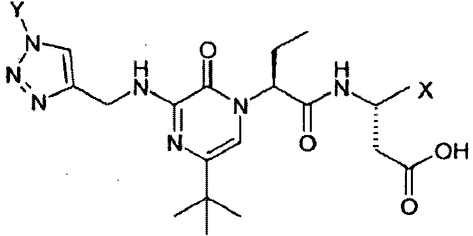 |
| 30. | 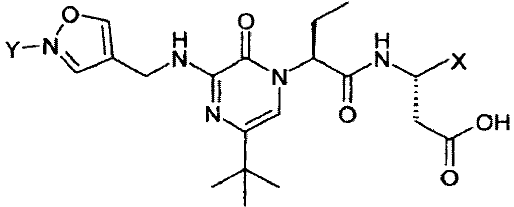 |
| 31. | 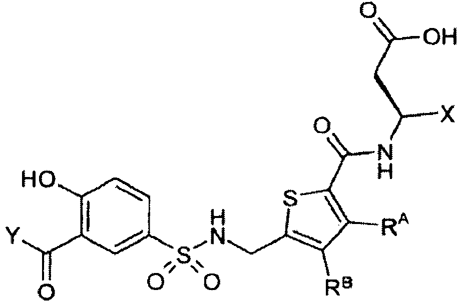 |
| | où R^{A} et R^{B} sont indépendamment hydrogène, alkyle en C₁-C₆, hydroxyle, alcoxy en C₁-C₆ ou halogène. |
| 32. | 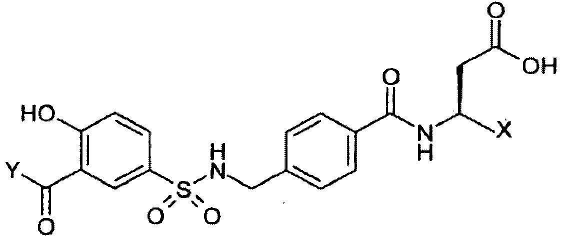 |
| 33. | 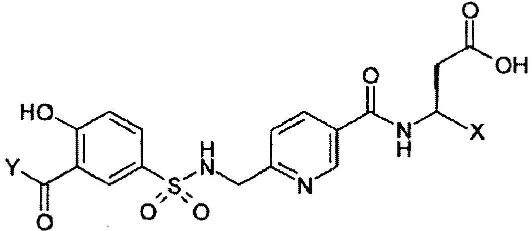 |
| 34. | 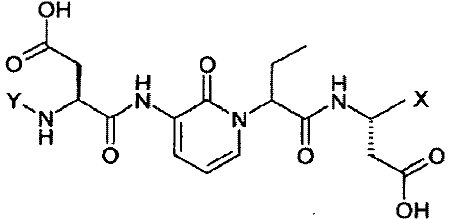 |
| 35. | 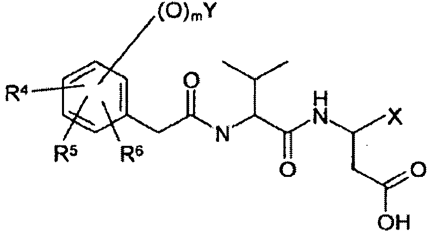 |
| | de préférence dans la configuration tout-(S), où m est 0 ou 1 ; et |
| | R⁴ R⁵ et R⁶ sont indépendamment choisis dans le groupe constitué de : |
| | 1) H, |
| | 2) halogène, |
| | 3) alcoxy en C₁-C₄ facultativement substitué par 1 à 3 atomes d'halogène, |
| | 4) NO₂, |
| | 5) OH, |
| | 6) benzyloxy, dont le fragment benzyle est facultativement substitué par 1 à 2 membres choisis dans le groupe constitué de : halogène, CN, alkyle en C₁-C₄ et alcoxy en C₁-C₄, lesdits alkyle et alcoxy étant facultativement substitués par 1 à 3 groupes halogène, |
| | 7) NH(acyle en C₁-C₄), |
| | 8) acyle en C₁-C₄, |
| | 9) O-(alkyle en C₁-C₄)-CO₂H, facultativement estérifié par un groupe alkyle en C₁-C₆ ou cycloalkyle en C₅-C₇, |
| | 10) CH=CH-CO₂H, |
| | 11) CO₂H, |
| | 12) (alkyle en C₁-C₅)-CO₂H, |
| | 13) C(O)NH₂, facultativement substitué sur l'atome d'azote par 1 à 2 groupes alkyle en C₁-C₄ ; |
| | 14) (alkyle en C₁-C₅)-C(O)NH₂, facultativement substitué sur l'atome d'azote par 1 à 2 groupes alkyle en C₁-C₄ ; |
| | 15) S (O) ₀₋₂-(alkyle en C₁-C₄) ; |
| | 16) (alkyle en C₁-C₂)-S(O)₀₋₂-(alkyle en C₁-C₄) ; |
| | 17) S(O)₀₋₂-(alkyle en C1-6) ou S(O)₀₋₂-phényle, lesdits fragments alkyle et phényle de ceux-ci étant facultativement substitués par 1 à 3 membres choisis dans le groupe constitué de : halogène, CN, alkyle en C₁-C₄ et alcoxy en C₁-C₄, lesdits alkyle et alcoxy étant facultativement substitués par 1 à 3 groupes halogène, |
| | 18) benzoyle facultativement substitué par 1 à 2 membres choisis dans le groupe constitué de : halogène, CN, alkyle en C₁-C₄ et alcoxy en C₁-C₄, lesdits groupes alkyle et alcoxy étant facultativement substitués par 1 à 3 groupes halogène, |
| | 19) phényle ou naphtyle, facultativement substitué par 1 à 2 membres choisis dans le groupe constitué de : halogène, CN, alkyle en C₁-C₄ et alcoxy en C₁-C₄, lesdits alkyle et alcoxy étant facultativement substitués par 1 à 3 groupes halogène, |
| | 20) CN, |
| | 21) (alkylène en C₁-C₄)-HET2, où HET2 représente un cycle aromatique ou non aromatique de 5 à 7 chaînons contenant de 1 à 4 hétéroatomes choisis parmi O, S, NH et N(C₁-C₄) et contenant facultativement 1 à 2 groupes oxo, et facultativement substitué par 1 à 3 groupes alkyle en C₁-C₄, OH, halogène ou acyle en C₁-C₄ ; |
| | 22) O-(alkyle en C₁-C₄)-HET3, où HET3 est un cycle aromatique ou non aromatique de 5 ou 6 chaînons contenant de 1 à 3 hétéroatomes choisis parmi O, S et N, et facultativement substitués par un ou deux groupes choisis parmi halogène et alkyle en C₁-C₄, et contenant facultativement 1 à 2 groupes oxo, et |
| | 23) HET4, où HET4 est un cycle aromatique ou non aromatique de 5 ou 6 chaînons, et les analogues benzocondensés de ceux-ci, contenant de 1 à 4 hétéroatomes choisis parmi O, S et N, et est facultativement substitué par un ou deux groupes choisis parmi halogène, alkyle en C₁-C₄ et acyle en C₁-C₄ ; et où l'halogène comprend F, C1, Br et I. |
| 36. | 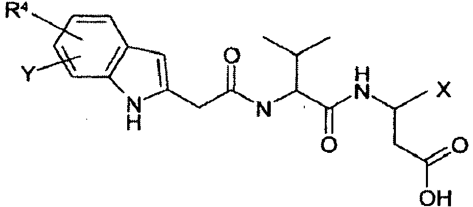 |
| | de préférence dans la configuration tout-(S), où R⁴ est choisi dans le groupe constitué de : |
| | 1) H, |
| | 2) halogène, |
| | 3) alcoxy en C₁-C₄ facultativement substitué par 1 à 3 atomes d'halogène, |
| | 4) NO₂, |
| | 5) OH, |
| | 6) benzyloxy, dont le fragment benzyle est facultativement substitué par 1 à 2 membres choisis dans le groupe constitué de : halogène, CN, alkyle en C₁-C₄ et alcoxy en C₁-C₄, lesdits alkyle et alcoxy étant facultativement substitués par 1 à 3 groupes halogène, |
| | 7) NH(acyle en C₁-C₄), |
| | 8) acyle en C₁-C₄, |
| | 9) O-(alkyle en C₁-C₄)-CO₂H, facultativement estérifié par un groupe alkyle en C₁-C₆ ou cycloalkyle en C₅-C₇, |
| | 10) CH=CH-CO₂H, |
| | 11) CO₂H, |
| | 12) (alkyle en C₁-C₅)-CO₂H, |
| | 13) C(O)NH₂, facultativement substitué sur l'atome d'azote par 1 à 2 groupes alkyle en C₁-C₄ ; |
| | 14) (alkyle en C₁-C₅)-C(O)NH₂, facultativement substitué sur l'atome d'azote par 1 à 2 groupes alkyle en C₁-C₄ ; |
| | 15) S (O)₀₋₂-(alkyle en C₁-C₄) ; |
| | 16) (alkyle en C₁-C₂)-S(O)₀₋₂-(alkyle en C₁-C₄) ; |
| | 17) S (O)₀₋₂-(alkyle en C1-6) ou S(O)₀₋₂-phényle, lesdits fragments alkyle et phényle de ceux-ci étant facultativement substitués par 1 à 3 membres choisis dans le groupe constitué de : halogène, CN, alkyle en C₁-C₄ et alcoxy en C₁-C₄, lesdits alkyle et alcoxy étant facultativement substitués par 1 à 3 groupes halogène, |
| | 18) benzoyle facultativement substitué par 1 à 2 membres choisis dans le groupe constitué de : halogène, CN, alkyle en C₁-C₄ et alcoxy en C₁-C₄, lesdits groupes alkyle et alcoxy étant facultativement substitués par 1 à 3 groupes halogène, |
| | 19) phényle ou naphtyle, facultativement substitué par 1 à 2 membres choisis dans le groupe constitué de : halogène, CN, alkyle en C₁-C₄ et alcoxy en C₁-C₄, lesdits alkyle et alcoxy étant facultativement substitués par 1 à 3 groupes halogène, |
| | 20) CN, |
| | 21) (alkylène en C₁-C₄)-HET2, où HET2 représente un cycle aromatique ou non aromatique de 5 à 7 chaînons contenant de 1 à 4 hétéroatomes choisis parmi O, S, NH et N(C₁-C₄) et contenant facultativement 1 à 2 groupes oxo, et facultativement substitué par 1 à 3 groupes alkyle en C₁-C₄, OH, halogène ou acyle en C₁-C₄ ; |
| | 22) O-(alkyle en C₁-C₄)-HET3, où HET3 est un cycle aromatique ou non aromatique de 5 ou 6 chaînons contenant de 1 à 3 hétéroatomes choisis parmi O, S et N, et facultativement substitués par un ou deux groupes choisis parmi halogène et alkyle en C₁-C₄, et contenant facultativement 1 à 2 groupes oxo, et |
| | 23) HET4, où HET4 est un cycle aromatique ou non aromatique de 5 ou 6 chaînons, et les analogues benzocondensés de ceux-ci, contenant de 1 à 4 hétéroatomes choisis parmi O, S et N, et est facultativement substitué par un ou deux groupes choisis parmi halogène, alkyle en C₁-C₄ et acyle en C₁-C₄ ; et où l'halogène comprend F, C1, Br et I. |
| 37. | 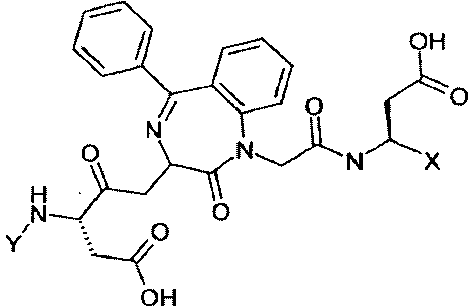 |
| 38. | 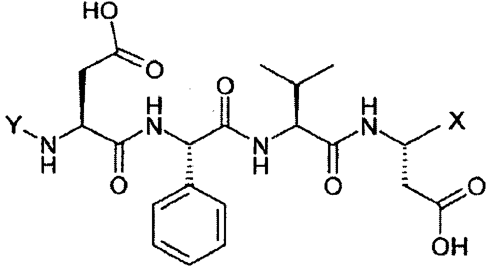 |
| 39. | 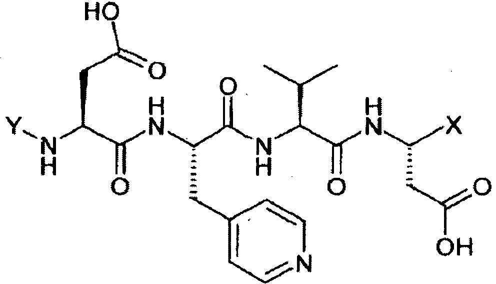 |
| 40. | 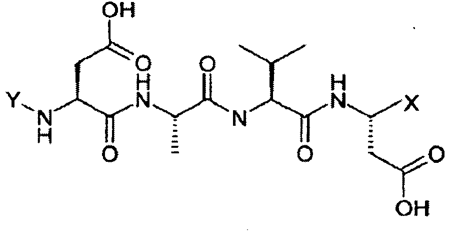 |
| 41. | 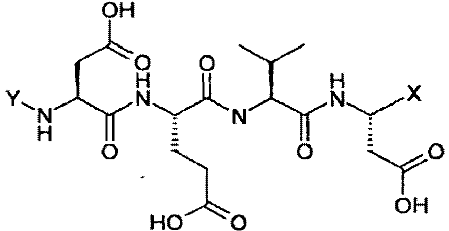 |
| 42. | 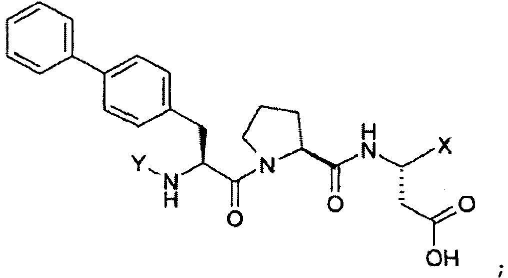 |
ou A est reconnaissable par la caspase 8 et est choisi dans le groupe de 43 à 44 dans le tableau ci-dessous :
| | |
|---|---|
| 43. | 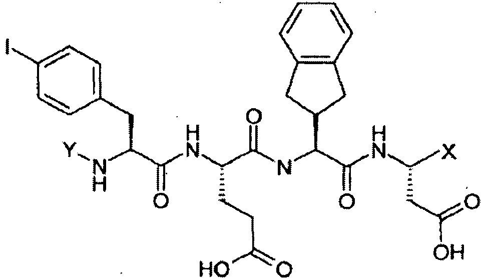 |
| 44. | 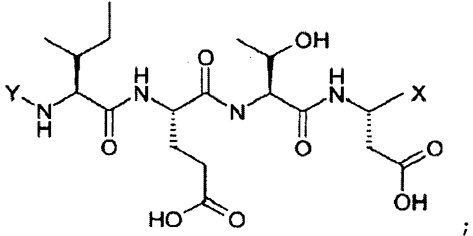 |
X est -CONH-R2-L2 ;
Y est -L-R1-L1 ;
R1 est un lieur ;
R2 est une liaison ou un lieur
où le lieur est un groupe alkylène à chaîne linéaire ou ramifiée avec 1 à 300 atomes de carbone, où facultativement
(a) un ou plusieurs atomes de carbone sont remplacés par l'oxygène, en particulier où un atome de carbone sur trois est remplacé par l'oxygène, par exemple un groupe polyéthylénoxy avec 1 à 100 motifs éthylénoxy ; et/ou
(b) un ou plusieurs atomes de carbone sont remplacés par un azote portant un atome d'hydrogène et les atomes de carbone adjacents sont substitués par oxo, représentant une fonction amide -NH-CO- ; et/ou
(c) un ou plusieurs atomes de carbone sont remplacés par une fonction ester -O-CO- ;
(d) la liaison entre deux atomes de carbone adjacents est une double ou triple liaison ; et/ou
(e) deux atomes de carbone adjacents sont remplacés par une liaison disulfure ;
L est une liaison ou un groupe choisi parmi 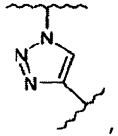 -(NRx)-, -0-, -C=N-, -C(=O)-, -C(=O)-NH-, -NH-C(=O)-, -C(=O)H, -CRx=CRy-, -C=C- et phényle, où Rx et Ry sont indépendamment H ou alkyle en C₁-C₆ ;
L1 et L2 sont, indépendamment l'un de l'autre, au moins un marqueur facultativement lié à un support solide, et n est 1,
ou
R2 est une liaison ;
L2 est un substrat, adapté pour un dosage bioluminescent couplé ;
Y est H ; et
n est 0.

2. Sonde selon la revendication 1, dans laquelle les marqueurs L1 et L2 sont indépendamment l'un de l'autre une sonde spectroscopique telle qu'un fluorophore ; un désactivateur ou un chromophore ; une sonde magnétique ; un réactif de contraste ; une molécule qui est une partie d'une paire de liaison spécifique qui est capable de se lier spécifiquement à un partenaire ; une molécule liée de façon covalente à un support solide, où le support peut être une lame de verre, une plaque de microtitrage ou un polymère quelconque ; une biomolécule ayant des propriétés enzymatiques, chimiques ou physiques souhaitables ; ou une molécule possédant une combinaison quelconque des propriétés énumérées ci-dessus ; ou un polymère linéaire ou ramifié positivement chargé.

3. Sonde selon la revendication 2, dans laquelle les marqueurs L1 et L2 sont indépendamment l'un de l'autre liés à un polymère linéaire ou ramifié positivement chargé.

4. Sonde selon la revendication 3, dans laquelle un marqueur L1 et L2 est une poly(arginine) linéaire de D- et/ou L-arginine avec 6 à 15 résidus arginine.

5. Sonde selon l'une quelconque des revendications 2 à 4, dans laquelle L1 est un membre et L2 est l'autre membre de deux sondes spectroscopiques interactives L1 / L2.

6. Sonde selon la revendication 5, dans laquelle L1 / L2 est une paire FRET.

7. Sonde selon la revendication 6, dans laquelle un L1 / L2 est un fluorophore choisi parmi Alexa 350, diméthylaminocoumarine, 5/6-carboxyfluorescéine, Alexa 488, ATTO 488, DY-505, 5/6-carboxyfluorescéine, Alexa 488, Alexa 532, Alexa 546, Alexa 555, ATTO 488, ATTO 532, tétraméthyl-rhodamine, Cy 3, DY-505, DY-547, Alexa 635, Alexa 647, ATTO 600, ATTO 655, DY-632, Cy 5, DY-647 Cy 5.5, et l'autre marqueur L1 / L2 est un désactivateur choisi parmi Dabsyl, Dabcyl, BHQ 1, QSY 35, BHQ 2, QSY 9, ATTO 540Q, BHQ 3, ATTO 612Q, QSY 21.

8. Sonde selon la revendication 1, dans laquelle n est 0, R2 est une liaison et L2 est un substrat, adapté pour un dosage bioluminescent couplé, **caractérisé par** une aminoluciférine modifiée ou un dérivé protégé carboxy-terminal de celle-ci en tant que groupe rapporteur, qui, après clivage de l'échafaudage central A, peut générer un signal luminescent par conversion par une luciférase.

9. Sonde de caspase choisie parmi 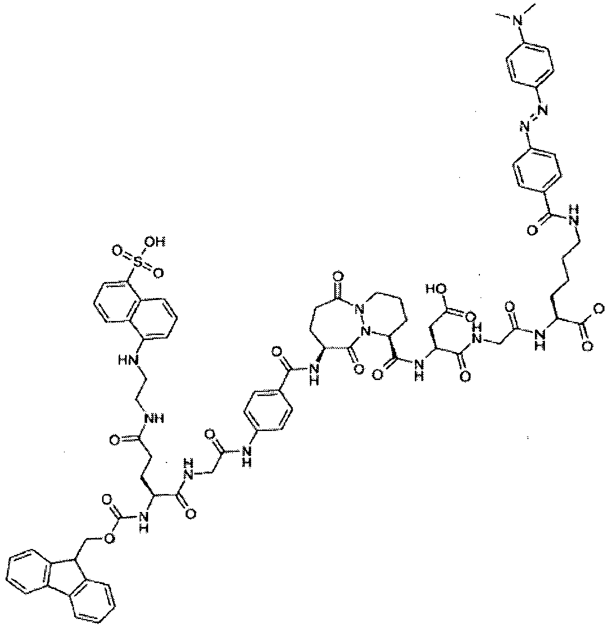 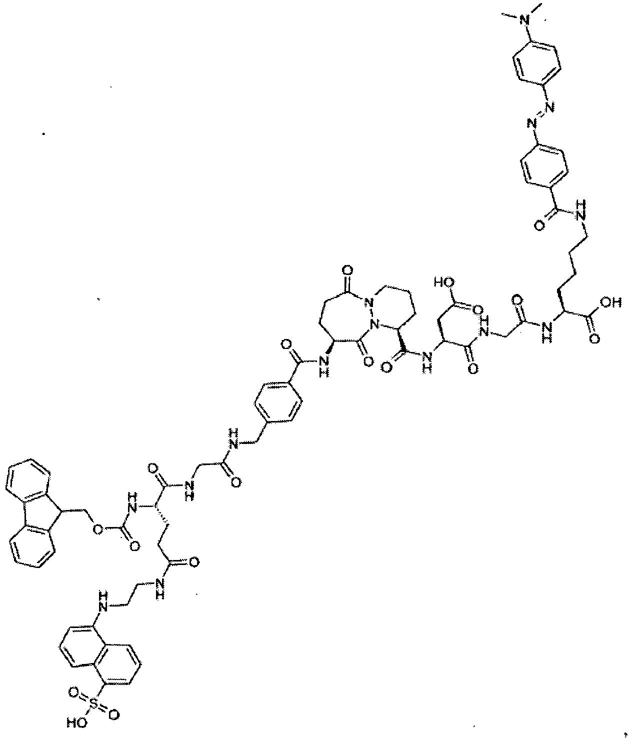 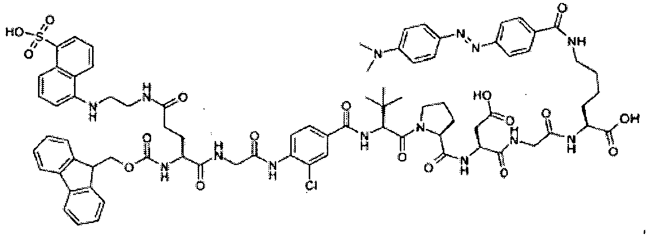 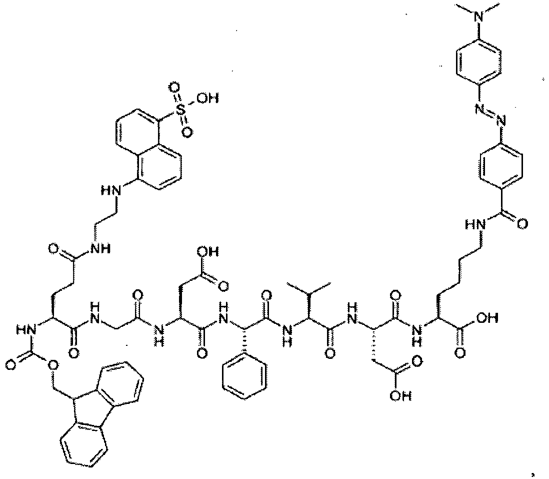 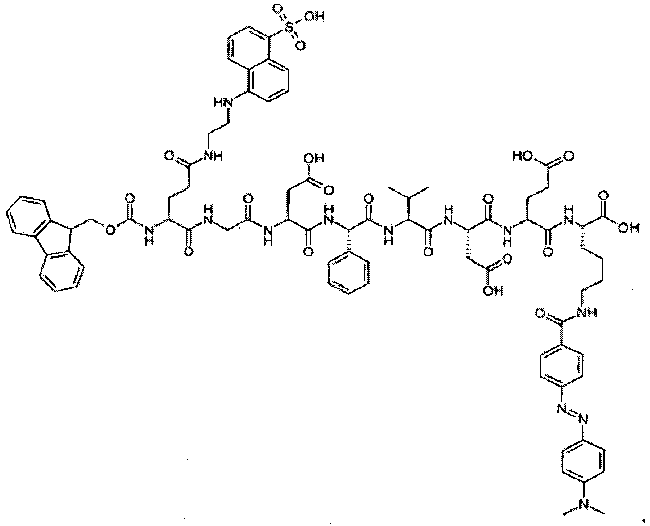 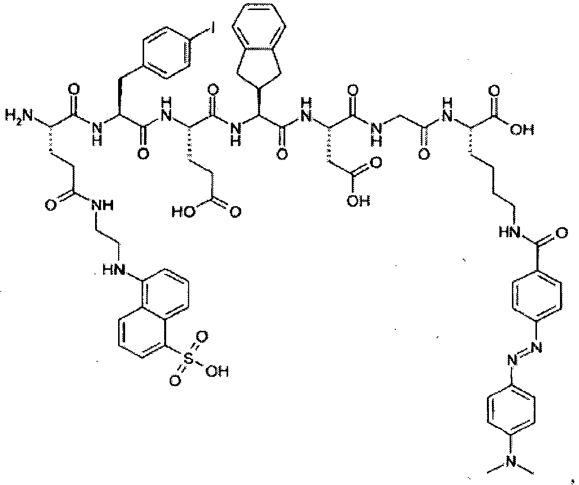 ou

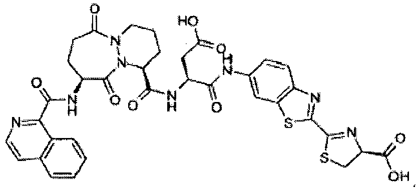

10. Préparation d'une sonde de formule (I) selon la revendication 1 à 9 **caractérisée par**
si n est 1 :
(a) réaction d'un composé de formule (II)
L'-A-CO-OH (II)
avec un composé de formule L1-R1-H pour obtenir un composé de formule (III)
L1-R1-L-A-CO-OH (III)
(b) réaction du composé (III) avec un composé H₂N-R2-L2 pour obtenir la sonde de formule (I),
où L' est fluoro, chloro, bromo, cyano, nitro, amino, azido, alkylcarbonylamino, carboxy, carbamoyle,
alkoxycarbonyle, aryloxycarbonyle, carbaldéhyde, hydroxy, alcoxy, aryloxy, alkylcarbonyloxy, arylcarbonyloxy, une double liaison carbone-carbone, une triple liaison carbone-carbone, de préférence amino, azido, hydroxy, cyano, carboxy, carbamoyle, carbaldéhyde, ou une double liaison carbone-carbone ou une triple liaison carbone-carbone, plus préférablement amino, et
R1 et/ou R2 peuvent être protégés par des groupes protecteurs orthogonalement adaptés et séquentiellement clivés au cours de la préparation du composé (I) ; et si n est 0 :
réaction d'un composé de formule A-CO-OH (IV) avec un composé H₂N-R2-L2 pour obtenir la sonde de formule (I).

11. Utilisation d'une sonde de formule (I) selon les revendications 1 à 9 pour l'imagerie moléculaire in vitro, dans des essais de culture de cellules, ou dans des essais *ex vivo*.

12. Sonde de formule (I) selon les revendications 1 à 9 pour utilisation dans un procédé pour l'imagerie moléculaire dans un organisme vivant.
